# EUROPEAN PATENT SPECIFICATION

(11) **EP 1 613 304 B1**
(45) Date of publication and mention of the grant of the patent: **12.09.2007**
(21) Application number: 04803694.1
(22) Date of filing: 09.12.2004
(51) Int. Cl.: A61K 31/397, A61K 31/4015, A61K 31/4025, A61K 31/435, C07D 205/04, C07D 207/08, C07D 207/09, C07D 211/22, C07D 211/26, C07D 401/12, C07D 403/12, C07D 413/12, A61P 3/10

(54) **DPP-IV INHIBITORS**
DPP-IV-HEMMER
INHIBITEURS DE DPP-IV

(30) Priority: 09.12.2003 EP 03028211
(43) Date of publication of application: 11.01.2006
(73) Proprietor: Santhera Pharmaceuticals (Schweiz) AG, 4410 Liestal (CH)
(72) Inventor: EDWARDS, Paul, John, 69115 Heidelberg (DE); LOPEZ-CANET, Meritxell, 69123 Heidelberg (DE); FEURER, Achim, 69259 Wilhelmsfeld (DE); CEREZO-GALVEZ, Silvia, 42287 Wuppertal (DE); MATASSA, Victor, Giulio, Barcelona 08198 (ES); NORDHOFF, Sonja, 69198 Schriesheim (DE); THIEMANN, Meinolf, 69121 Heidelberg (DE); HILL, Oliver, 69239 Neckarsteinach (DE)
(74) Representative: Grünecker, Kinkeldey, Stockmair & Schwanhäusser Anwaltssozietät
(86) International application number: PCT/EP2004/014040
(87) International publication number: WO 2005/056003

(56) References cited:
- WO-A-02/083128
- WO-A-03/000180
- WO-A-03/000181

## Description

The present invention relates to a novel class of dipeptidyl peptidase inhibitors, including pharmaceutically acceptable salts and prodrugs thereof, which are useful as therapeutic compounds, particularly in the treatment of Type 2 diabetes mellitus, often referred to as non-insulin dependent diabetes mellitus (NIDDM), and of conditions that are often associated with this disease, such as obesity and lipid disorders. The invention also relates to a process for the preparation of such inhibitors.

Diabetes refers to a disease process derived from multiple causative factors and characterized by elevated levels of plasma glucose or hyperglycemia in the fasting state or after administration of glucose during an oral glucose tolerance test Persistent or uncontrolled hyperglycemia is associated with increased and premature morbidity and mortality. Often abnormal glucose homeostasis is associated both directly and indirectly with alterations of the lipid, lipoprotein and apolipoprotein metabolism and other metabolic and hemodynamic disease. Therefore patients with Type 2 diabetes mellitus are at an increased risk of macrovascular and microvascular complications, including coronary heart disease, stroke, peripheral vascular disease, hypertension, nephropathy, neuropathy, and retinopathy. Therefore, therapeutical control of glucose homeostasis, lipid metabolism and hypertension are critically important in the clinical management and treatment of diabetes mellitus.

There are two generally recognized forms of diabetes. In Type 1, or insulin-dependent, diabetes mellitus (IDDM), patients produce little or no insulin, which is the hormone regulating glucose utilization. In Type 2, or noninsulin dependent, diabetes mellitus (NIDDM), patients often have plasma insulin levels that are the same or elevated compared to nondiabetic subjects. These patients develop a resistance to the insulin stimulating effect on glucose and lipid metabolism in the main insulin-sensitive tissues, namely the muscle, liver and adipose tissues. Further, the plasma insulin levels, while elevated, are insufficient to overcome the pronounced insulin resistance.

Insulin resistance is not primarily due to a diminished number of insulin receptors but to a post-insulin receptor binding defect that is not yet understood. This resistance to insulin responsiveness results in insufficient insulin activation of glucose uptake, oxidation and storage in muscle, and inadequate insulin repression of lipolysis in adipose tissue and of glucose production and secretion in the liver.

The available treatments for Type 2 diabetes, which have not changed substantially in many years, have recognized limitations. While physical exercise and reductions in dietary intake of calories will dramatically improve the diabetic condition, compliance with this treatment is very poor because of well-entrenched sedentary lifestyles and excess food consumption, especially of foods containing high amounts of saturated fat. Increasing the plasma level of insulin by administration of sulfonylureas (e.g., tolbutamide and glipizide) or meglitinide, which stimulate the pancreatic β-cells to secrete more insulin, and/or by injection of insulin when sulfonylureas or meglitinide become ineffective, can result in insulin concentrations high enough to stimulate the very insulin-resistant tissues. However, dangerously low levels of plasma glucose can result from administration of insulin or insulin secretagogues (sulfonylureas or meglitinide), and an increased level of insulin resistance, due to the even higher plasma insulin levels, can occur. The biguanides increase insulin sensitivity resulting in some correction of hyperglycemia. However, the two biguanides, phenformin and metformin, can induce lactic acidosis and nausea/diarrhoea. Metformin has fewer side effects than phenformin and is often prescribed for the treatment of Type 2 diabetes.

The glitazones (*i.e*., 5-benzylthiazolidine-2,4-diones) are a recently described class of compounds with potential for ameliorating many symptoms of Type 2 diabetes. These agents substantially increase insulin sensitivity in muscle, liver and adipose tissue in several animal models of Type 2 diabetes, resulting in partial or complete correction of the elevated plasma levels of glucose without occurrence of hypoglycemia. The glitazones that are currently marketed are agonists of the peroxisome proliferator activated receptor (PPAR), primarily the PPAR-gamma subtype. PPAR-gamma agonism is generally believed to be responsible for the improved insulin sensitization that is observed with the glitazones. Newer PPAR agonists that are being tested for treatment of Type 2 diabetes are agonists of the alpha, gamma or delta subtype, or a combination of these, and in many cases are chemically different from the glitazones (*i.e*., they are not thiazolidinediones). Serious side effects (*e*.*g*., liver toxicity) have occurred with some of the glitazones, such as troglitazone.

Additional methods of treating the disease are still under investigation. New biochemical approaches that have been recently introduced or are still under development include treatment with alpha-glucosidase inhibitors (e.g., acarbose) and protein tyrosine phosphatase-IB (PTP-1B) inhibitors.

Compounds that are inhibitors of the dipeptidyl peptidase-IV (DPP-IV) enzyme are also under investigation as drugs that may be useful in the treatment of diabetes, and particularly Type 2 diabetes. See for example WO-A-97/40832, WO-A-98/19998, WO-A-03/180 and WO-A-03/181. The usefulness of DPP-IV inhibitors in the treatment of Type 2 diabetes is based on the fact that DPP-IV *in vivo* readily inactivates glucagon like peptide-1 (GLP-1) and gastric inhibitory peptide (GIP). GLP-1 and GIP are incretins and are produced when food is consumed. The incretins stimulate production of insulin. Inhibition of DPP-IV leads to decreased inactivation of the incretins, and this in turn results in increased effectiveness of the incretins in stimulating production of insulin by the pancreas. DPP-IV inhibition therefore results in an increased level of serum insulin. Advantageously, since the incretins are produced by the body only when food is consumed, DPP-IV inhibition is not expected to increase the level of insulin at inappropriate times, such as between meals, which can lead to excessively low blood sugar (hypoglycemia). Inhibition of DPP-IV is therefore expected to increase insulin without increasing the risk of hypoglycemia, which is a dangerous side effect associated with the use of insulin secretagogues.

DPP-IV inhibitors may also have other therapeutic utilities, as discussed elsewhere in this application. DPP-IV inhibitors have not been studied extensively to date, especially for utilities other than diabetes. New compounds are needed so that improved DPP-IV inhibitors can be found for the treatment of diabetes and potentially other disease and conditions.

Thus, the object of the present invention is to provide a new class of DPP-IV inhibitors which may be effective in the treatment of Type 2 diabetes and other DPP-IV modulated diseases.

Accordingly, the present invention provides novel compounds of formula (I) as defined in the claims.

Preferably, the present invention provides novel compounds of formula (I): 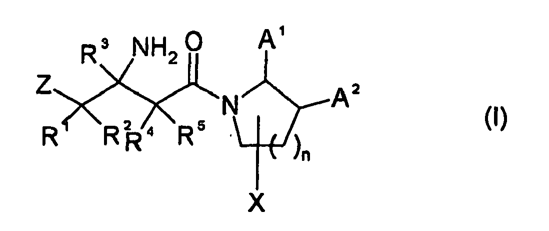 or a pharmaceutically acceptable salt thereof, wherein
Z is selected from the group consisting of
phenyl;
naphthyl;
C₃₋₇ cycloalkyl;
heterocycle; and
heterobicycle;
wherein Z is optionally substituted with one, or independently from each other, more of
halogen;
CN;
OH;
=O, where the ring is at least partially saturated;
C₁₋₆ alkyl, optionally substituted with one or more F; and
O-C₁₋₆ alkyl, optionally substituted with one or more F;
R¹, R², R⁴, R⁵ are independently from each other selected from the group consisting of
H;
F;
OH;
C₁₋₆ alkyl, optionally substituted with one or more F; and
O-C₁₋₆ alkyl, optionally substituted with one or more F;
and/or R¹ and R² optionally form together C₃₋₇ cycloalkyl, which is optionally substituted with one or more F;
and/or R² and R³ optionally form together C₃₋₇ cycloalkyl, which is optionally substituted with one or more F;
and/or R³ and R⁴ optionally form together C₃₋₇ cycloalkyl, which is optionally substituted with one or more F;
and/or R⁴ and R⁵ optionally form together C₃₋₇ cycloalkyl, which is optionally substituted with one or more F;
R³ is H or C₁₋₆ alkyl;
X is selected from the group consisting of
H;
F; and
C₁₋₆ alkyl, optionally substituted with one or more F;
n is 0 or 1;
A¹, A² are independently from each other selected from the group consisting of
H;
halogen;
C₁₋₆ alkyl, optionally substituted with one or more F; and
R⁶; provided that one of A¹ and A² is R⁶;
R⁶ is -C(R⁷R⁸)-Y-T;
R⁷, R⁸ are independently from each other selected from the group consisting of
H;
F; and
C₁₋₆ alkyl, optionally substituted with one or more F;
and/or R⁷ and R⁸ optionally form together C₃₋₇ cycloalkyl, which is optionally substituted with one or more F;
Y is selected from the group consisting of
-0-;
-C₁₋₆ alkyl-O-;
-N(R⁹)-;
-C₁₋₆ alkyl-N(R⁹)-
-S-;
-C₁₋₆ alkyl-S-;
-S(O)-;
-C₁₋₆ alkyl-S(O)-;
-S(O)₂-; and
-C₁₋₆ alkyl-S(O)₂-;
wherein each C₁₋₆ alkyl is optionally substituted with one or more F;
R⁹, T are independently from each other T¹- T² or T²;
T¹ is selected from the group consisting of
-C₁₋₆ alkyl-;
-C₁₋₆ alkyl-O-
-C₁₋₆ alkyl-N(R¹⁰)-
-C(O)-;
-C(O)-C₁₋₆ alkyl-;
-C(O)-C₁₋₆ alkyl-O-;
-C(O)-C₁₋₆ alkyl-N(R¹⁰)-;
-C(O)O-;
-C(O)O-C₁₋₆ alkyl-;
-C(O)O-C₁₋₆ alkyl-O-;
-C(O)O-C₁₋₆ alkyl-N(R¹⁰)-;
-C(O)N(R¹⁰)-;
-C(O)N(R¹⁰)-C₁₋₆ alkyl-;
-C(O)N(R¹⁰)-C₁₋₆ alkyl-O-;
-C(O)N(R¹⁰)-C₁₋₆ alkyl-N(R¹¹)-;
-S(O)₂-;
-S(O)₂-C₁₋₆ alkyl-;
-S(O)₂-C₁₋₆ alkyl-O-; and
-S(O)₂-C₁₋₆ alkyl-N(R¹⁰)-;
wherein each C₁₋₆ alkyl is optionally substituted with one or more F;
R'°, R¹¹ are independently from each other H or C₁₋₆ alkyl, optionally substituted with one or more F;
T² is selected from the group consisting of
H;
C∓₃;
phenyl;
naphthyl;
wherein phenyl and naphthyl are optionally substituted with one, or independently from each other, more of
halogen;
CN;
R¹²;
COOH;
OH;
C(O)NH₂;
S(O)₂NH₂;
COOT³;
OT³;
C(O)NHT³;
S(O)₂NHT³; or
T³;
C₃₋₇ cycloalkyl;
heterocycle; and
heterobicycle;
wherein C₃₋₇ cycloalkyl, heterocycle and heterobicycle are optionally substituted with one, or independently from each other, more of
halogen;
CN;
R¹³;
OH;
=0, where the ring Is at least partially saturated;
NH₂
COOH;
C(O)NH₂;
S(O)₂NH₂;
COOT³;
OT³;
C(O)NHT³;
S(O)₂NHT³;
NHT³; or
T³;
R¹² is selected from the group consisting of
C₁₋₆ alkyl;
O-C₁₋₆ alkyl;
COO-C₁₋₆ alkyl;
OC(O)- C₁₋₆ alkyl;
C(O)N(R¹⁵)- C₁₋₆ alkyl;
S(O)₂N(R¹⁷)-C₁₋₆ alkyl;
S(O)-C₁₋₆ alkyl;
S(O)₂-C₁₋₆ alkyl; and
N(R¹⁸)S(O)₂₋C₁₋₆ alkyl;
wherein each C₁₋₆ alkyl is optionally substituted with one, or independently from each other, more of F, COOR¹⁹, C(O)N(R²⁰R²¹), S(O)₂N(R²²R²³), OR²⁴, N(R²⁵R²⁶), T³, O-T³ or N(R²⁷)-T³;
R¹³ is selected from the group consisting of
C₁₋₆ alkyl;
O-C₁₋₆ alkyl;
N(R¹⁴)-C₁₋₆ alkyl;
COO-C₁₋₆ alkyl;
OC(O)- C₁₋₆ alkyl;
C(O)N(R¹⁵)- C₁₋₆ alkyl;
N(R¹⁶)-C(O)-C₁₋₆ alkyl;
S(O)₂N(R¹⁷)-C₁₋₆ alkyl;
S(O)-C₁₋₆ alkyl;
S(O)₂-C₁₋₆ alkyl; and
-N(R¹⁸)S(O)₂-C₁₋₆ alkyl;
wherein each C₁₋₆ alkyl is optionally substituted with one, or Independently from each other, more of F, COOR¹⁹, C(O)N(R²⁰R²¹), S(O)₂N(R²²R²³), OR²⁴, N(R²⁵R²⁶), T³, O-T³ or N(R²⁷)-T³;
R¹⁴, R¹⁵, R¹⁶, R¹⁷, R¹⁸, R¹⁹, R²⁰ , R²¹, R²², R²³, R²⁴, R²⁵, R²⁶, R²⁷ are independently from each other H or C₁₋₆ alkyl;
T³ is selected from the group consisting of
phenyl;
naphthyl;
wherein phenyl and naphthyl are optionally substituted with one, or independently from each other, more of
halogen;
CN;
COOH;
OH;
C(O)NH₂;
S(O)₂NH₂
C₁₋₆ alkyl;
O-C₁₋₆ alkyl;
COO-C₁₋₆ alkyl;
OC(O)- C₁₋₆ alkyl;
C(O)N(R²⁸)- C₁₋₆ alkyl;
S(O)₂N(R²⁹)-C₁₋₆ alkyl;
S(O)₂-C₁₋₆ alkyl; or
N(R³⁰)S(O)₂-C₁₋₆ alkyl;
heterocycle;
heterobicyGe; and
C₃₋₇ cycloalkyl;
wherein C₃₋₇ cycloalkyl, heterocycle and heterobicycle are optionally substituted with one, or independently from each other, more of
halogen;
CN;
OH;
=O, where the ring is at least partially saturated;
NH₂
COOH;
C(O)NH₂;
S(O)₂NH₂
C₁₋₆ alkyl;
O-C₁₋₆ alkyl;
N(R³¹)-C₁₋₆ alkyl;
COO-C₁₋₆ alkyl;
OC(O)- C₁₋₆ alkyl;
C(O)N(R³²)- C₁₋₆ alkyl;
N(R³³)-C(O)-C₁₋₆ alkyl;
S(O)₂N(R³⁴)-C₁₋₆ alkyl;
S(O)₂-C₁₋₆ alkyl; or
-N(R³⁵)S(O)₂-C₁₋₆ alkyl.

Within the meaning of the present invention the terms are used as follows:
"Alkyl" means a straight-chain or branched carbon chain that may contain double or triple bonds. It is generally preferred that alkyl doesn't contain double or triple bonds. "C₁₋₆ Alkyl" means an alkyl chain having 1 - 6 carbon atoms, e.g. methyl, ethyl, -CH=CH₂, -C≡CH. n-propyl, isopropyl, -CH=CH-CH₃, -CH₂-CH=CH₂, n-butyl, isobutyl, -CH=CH-CH₂-CH₃, -CH=CH-CH=CH₂, sec-butyl tert-butyl, n-pentane, n-hexane, or amidst, e.g. -CH₂-, -CH₂-CH₂-, -CH=CH-, -CH(CH₃)-, -C(CH₂)-, -CH₂-CH₂-CH₂-, -CH(C₂H₅)-, -CH(CH₃)₂-. Each hydrogen of a C₁₋₆ alkyl carbon may be replaced by a substituent.
"C₃₋₇ Cycloalkyl" means a cyclic alkyl chain having 3-7 carbon atoms, e.g. cyclopropyl, cyclobutyl, cyclopentyl, cyclohexyl, cyclohexenyl, cycloheptyl. Each hydrogen of a cycloalkyl carbon may be replaced by a substituent.
"Halogen" means fluoro, chloro, bromo or iodo. It is generally preferred that halogen is fluoro or chloro.
"Heterocycle" means a cyclopentane, cyclohexane or cycloheptane ring that may contain up to the maximum number of double bonds (aromatic or non-aromatic ring which is fully, partially or un-saturated) wherein at least one carbon atom up to 4 carbon atoms are replaced by a heteroatom selected from the group consisting of sulfur (including -S(O)-, -S(O)₂-), oxygen and nitrogen (including =N(O)-) and wherein the ring is linked to the rest of the molecule via a carbon or nitrogen atom. Examples for a heterocycle are furan, thiophene, pyrrole, pyrroline, imidazole, imidazoline, pyrazole, pyrazoline, oxazole, oxazoline, isoxazole, isoxazoline, thiazole, thiazoline, isothiazole, isothiazoline, thiadiazole, thiadiazoline, tetrahydrofuran, tetrahydrothiophene, pyrrolidine, imidazolidine, pyrazolidine, oxazolidine, isoxazolidine, thiazolidine, isothiazolidine, thiadiazolidine, sulfolane, pyran, dihydropyran, tetrahydropyran, imidazolidine, pyridine, pyridazine, pyrazine, pyrimidine, piperazine, piperidine, morpholine, tetrazole, triazole, triazolidine, tetrazolidine, azepine or homopiperazine.
"Heterobicycle" means a heterocycle which is condensed with phenyl or an additional heterocycle to form a bicyclic ring system. "Condensed" to form a bicyclic ring means that two rings are attached to each other by sharing two ring atoms. Examples for a heterobicycle are indole, indoline, benzofuran, benzothiophene, benzoxazole, benzisoxazole, benzothiazole, benzisothiazole, benzimidazole, benzimidazoline, quinoline, quinazoline, dihydroquinazoline, dihydroquinoline, isoquinoline, tetrahydroisoquinoline, dihydroisoquinoline, benzazepine, purine or pteridine.

A preferred stereochemistry of compounds according to the present invention is shown in formula (la)

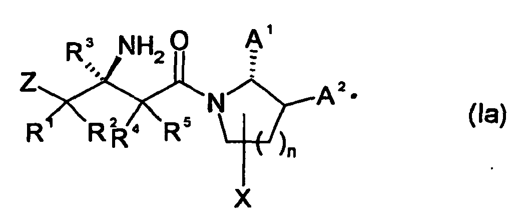

Preferred compounds of formula (I) or (Ia) are those compounds in which one or more of the residues contained therein have the meanings given below, with all combinations of preferred substituent definitions being a subject of the present invention. With respect to all preferred compounds of the formulas (I) or (Ia) the present invention also includes all tautomeric and stereoisomeric forms and mixtures thereof in all ratios, and their pharmaceutically acceptable salts.

In preferred embodiments of the present invention, the substituents R¹ - R⁵, Z, X, n, A¹ and A² of the formula (I) or (Ia) independently from each other have the following meaning. Hence, one or more of the substituents R¹ - R⁵, Z, X, n, A¹ and A² can have the preferred or more preferred meanings given below.

Z is preferably is phenyl or heterocycle and Z is optionally substituted independently from each other with 1, 2 or 3, more preferably up to 2 or 3, of Cl, F, CN, CH₃ or OCH₃. In one embodiment Z is substituted with up to 3 F.

It is preferred that R¹, R², R⁴, R⁵ are independently from each other selected from the group consisting of H, F, OH CH₃, OCH₃.

R³ is preferably H.

X is preferably H, F or CH₃.

Preferably, n is 1. In other embodiments, n is 0.

It is preferred that A¹ is R⁶ and A² is H, F or CH₃. In this case, n is preferably 1. In other embodiments, in particular, when n is 0, A² is preferably R⁶. In this case, A² is preferably H, F or CH₃.

R⁶ is preferably -CH₂-Y-T.

Y is preferably -O-, -N(R⁹)- or -S(O)₂-, more preferably -O- or -N(R⁹)-.

Preferably, R⁹ is selected from the group consisting of H, CH₃, COOH, COOCH₃, C(O)NH₂, C(O)N(CH₃)₂, and S(O)₂CH₃, more preferably H, CH₃, most preferably H.

It is preferred that T is T¹-T² or T², wherein T¹ is selected from the group consisting of
-CH₂-;
-C(O)-;
-C(O)-CH₂-;
-C(O)O-;
-C(O)O-CH₂-;
-C(O)NH-;
-C(O)NH-CH₂-;
-S(O)₂-; and
-S(O)₂-CH₂-.

More preferred is T¹ selected from the group consisting of -C(O)-; -CH₂-; -S(O)₂-; and -C(O)NH-.

It is preferred that T is T¹-T². In this case, T¹-T² is preferably a group as defined below.

In one embodiment, T¹-T² is preferably CH₂-phenyl, whereby phenyl may be substituted with 1-3, preferably 1 or 2, substituents selected from halogen, CN, O-C₁₋₄ alkyl, C₁₋₄ alkyl or S(O)₂CH₃, preferably F, CI, O-Me, Me or S(O)₂CH₃.
In one embodiment, T¹-T² is preferably CH₂-C₃₋₇ cycloalkyl, more preferably cyclopropyl or cyclobutyl, more preferably cyclopropyl, whereby cycloalkyl may be substituted with 1 or 2, preferably 1, of halogen; CN; OH; NH₂ COOH; C(O)NH₂; or S(O)₂NH₂, more preferably COOH or C(O)NH₂.
In one embodiment, T¹-T² is preferably C₁₋₄ alkyl, preferably methyl, ethyl or propyl, most preferably methyl.
In one embodiment, T¹- T² is preferably C(O)-phenyl, whereby phenyl may be substituted with 1-3, preferably 1 or 2, substituents selected from halogen, CN, O-C₁₋₄ alkyl, C₁₋₄ alkyl or S(O)₂CH₃, preferably F, Cl, O-Me, Me or S(O)₂CH₃.
In one embodiment, T¹-T² is preferably C(O)-C₃₋₇ cycloalkyl, more preferably cyclopropyl or cyclobutyl, more preferably cyclopropyl, whereby cycloalkyl may be substituted with 1-3, preferably 1 or 2, substituents selected from halogen, CN, O-C₁₋₄ alkyl, C₁₋₄ alkyl, whereby alkyl may be further substituted with 1 to 3 F; more preferably cycloalkyl may be substituted with 1 C₁₋₄ alkyl substituted with 1 to 3 F.
In one embodiment, T¹-T² is preferably C(O)-heterocycle, whereby heterocycle may be substituted with 1-3, preferably 1 or 2, substituents selected from halogen, CN, O-C₁₋₄ alkyl, C₁₋₄ alkyl or S(O)₂CH₃; preferably, the heterocycle is aromatic, more preferably containing 1 or 2 heteroatoms selected from N and O most preferably N.
In one embodiment, T¹-T² is preferably S(O)₂-phenyl, whereby phenyl may be substituted with 1-3, preferably 1 or 2, substituents selected from halogen, CN, O-C₁₋₄ alkyl, C₁₋₄ alkyl or S(O)₂CH₃, preferably F, Cl, O-Me, Me or S(O)₂CH₃.
In one embodiment, T¹-T² is preferably S(O)₂-C₃₋₇ cycloalkyl, more preferably cyclopropyl or cyclobutyl, more preferably cyclopropyl, whereby cycloalkyl may be substituted with 1-3, preferably 1 or 2, substituents selected from halogen, CN, O-C₁₋₄ alkyl, C₁₋₄ alkyl, whereby alkyl may be further substituted with 1 to 3 F; more preferably cycloalkyl may be substituted with 1 C₁₋₄ alkyl substituted with 1 to 3 F.
In one embodiment, T¹- T² is preferably S(O)₂-C₁₋₄ alkyl, preferably S(O)₂CH₃.

In one embodiment, T¹-T² is preferably C(O)-NH-phenyl, whereby phenyl may be substituted with 1-3, preferably 1 or 2, substituents selected from halogen, CN, O-C₁₋₄ alkyl, C₁₋₄ alkyl or S(O)₂CH₃.

When T is T², is preferably a group as defined below.

In one embodiment, T² is preferably H.
In one embodiment, T² is preferably phenyl, whereby phenyl may be substituted with 1-3, preferably 1 or 2, substituents selected from halogen, CN, O-C₁-₄ alkyl, C₁-₄ alkyl or S(O)₂CH₃, preferably F, Cl, O-Me, Me or S(O)₂CH₃.
In one embodiment, T² is preferably heterocycle, whereby heterocycle may be substituted with 1-3, preferably 1 or 2, substituents selected from halogen, CN, phenyl, heterocycle, O-C₁₋₄ alkyl, C₁₋₄ alkyl or S(O)₂CH₃; preferably, the heterocycle is aromatic,
more preferably containing 1, 2 or 3 heteroatoms selected from N and 0, most preferably N. When the heterocycle is substituted with phenyl or heterocycle, the heterocycle is preferably aromatic, more preferably containing 1, 2 or 3 heteroatoms selected from N and O, most preferably N, and the phenyl or heterocycle may be further substituted by 1 or 2 F or S(O)₂CH₃.

In one embodiment, T² is preferably CF₃.

T² is preferably phenyl or heterocycle.

Preferably, R⁶ is -CH₂-N(R³⁶)-T, wherein R³⁶ is H, S(O)₂CH₃ or S(O)₂-C₃₋₇ cycloalkyl, most preferably H.

In other embodiments, R⁶ is -CH₂-O-T.

In the case that Y contains the group R⁹, the following is preferred in embodiments:
When R⁹ is T¹-T² and represents -C₁₋₆ alkyl and T is T¹-T² and represents -C₁₋₆ alkyl then R⁹ and T may form together a 3 to 7 membered cyclic group containing 1 N, preferably a 5 or 6 membered cyclic group.

Compounds of the formula (I) or (Ia) in which some or all of the above-mentioned groups have the preferred or more preferred meanings are also an object of the present invention.

Preferred embodiments of the compounds according to present invention are shown in formula (IIa) to (IIi).

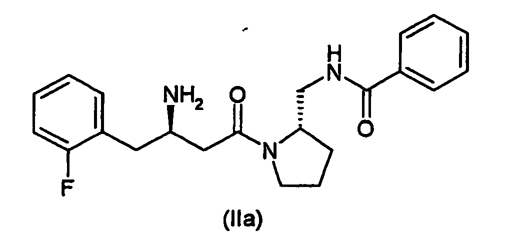

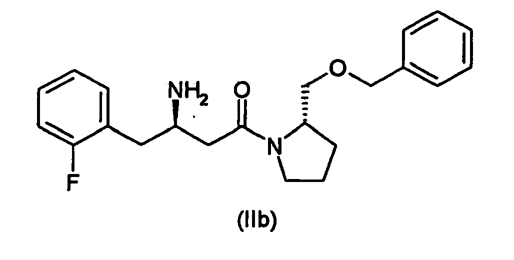

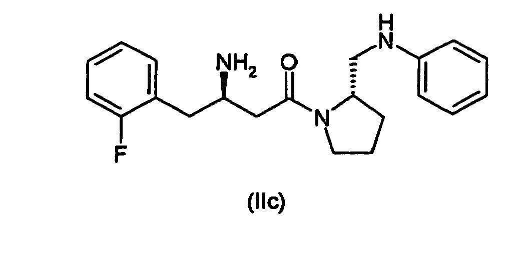

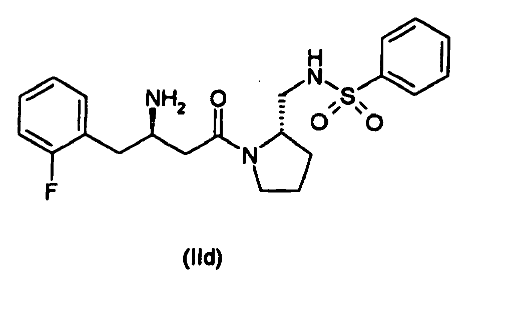

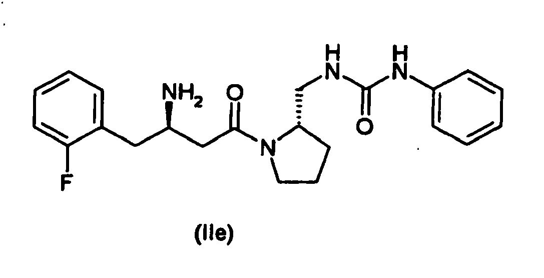

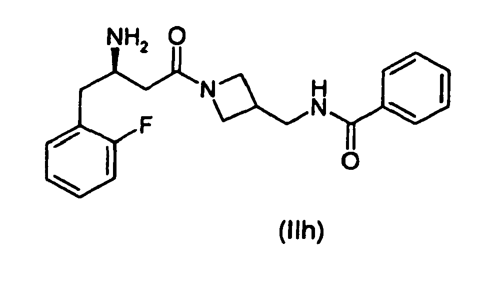

Also preferred are the following compounds:

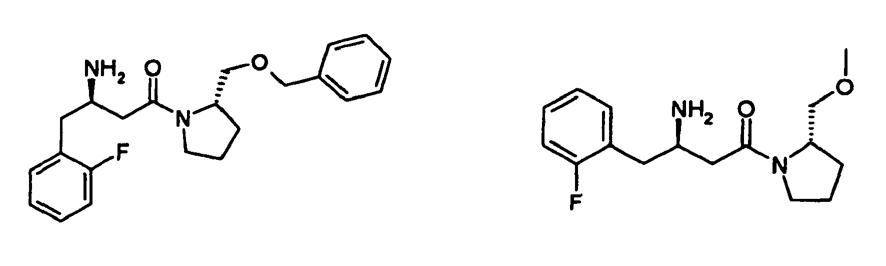

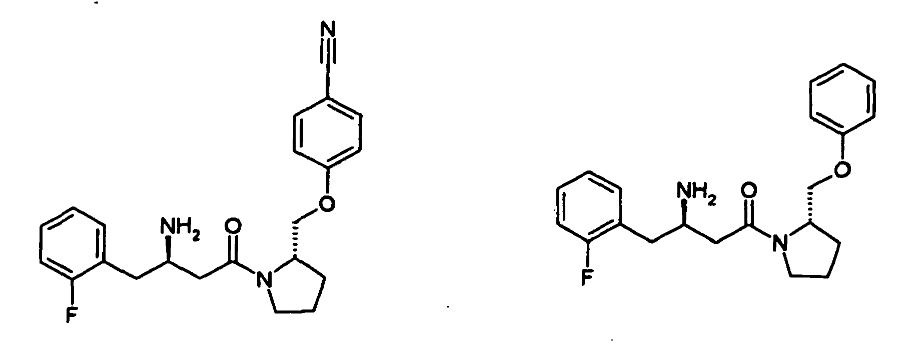

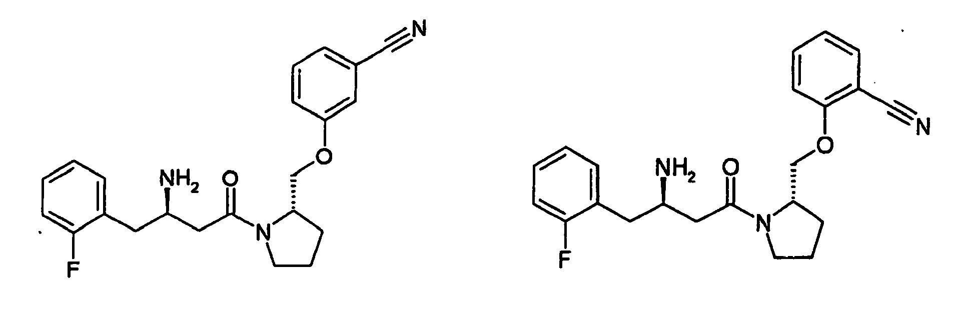

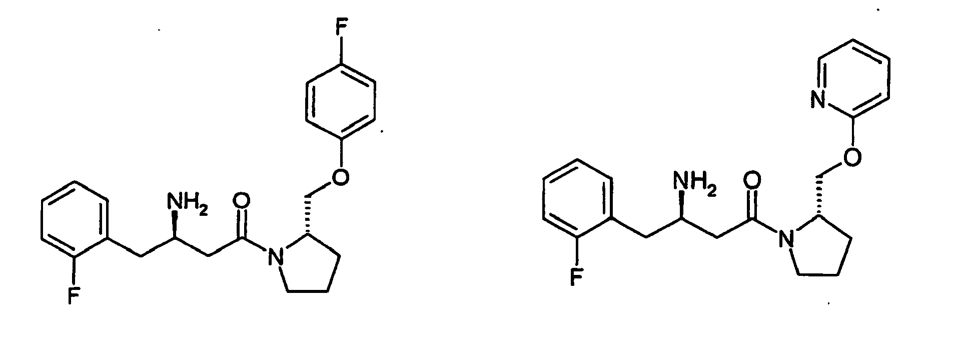

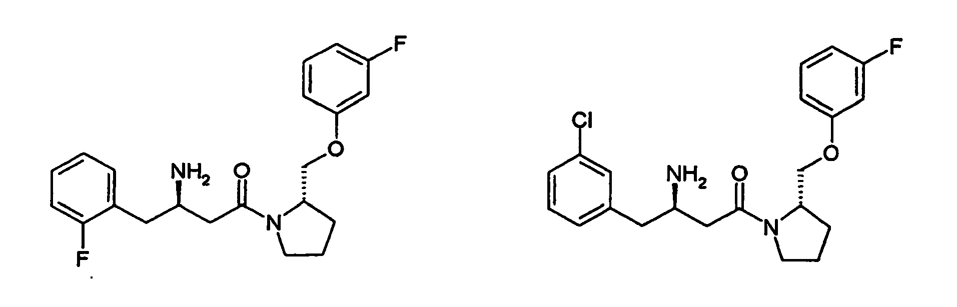

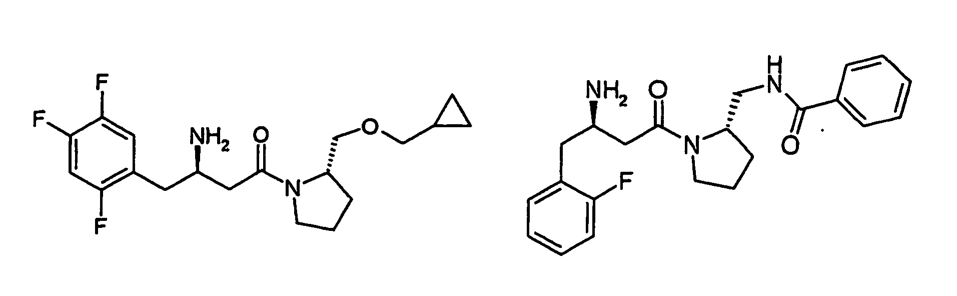

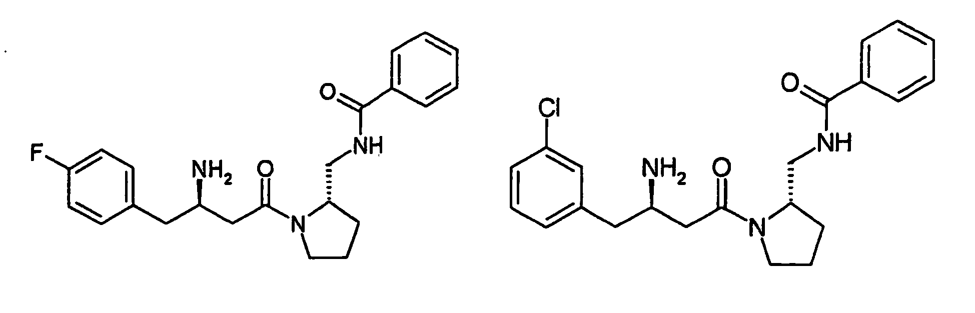

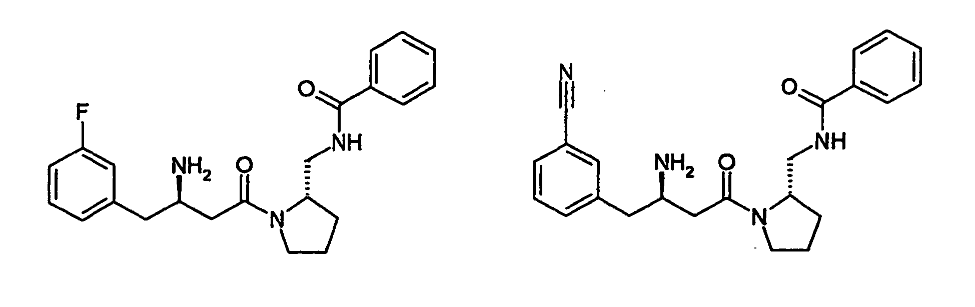

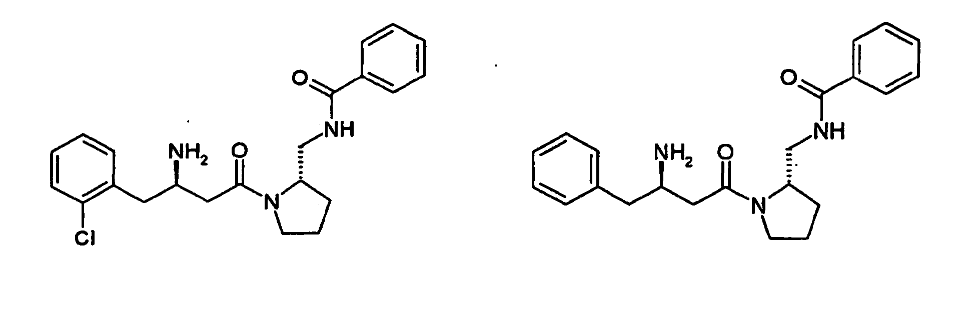

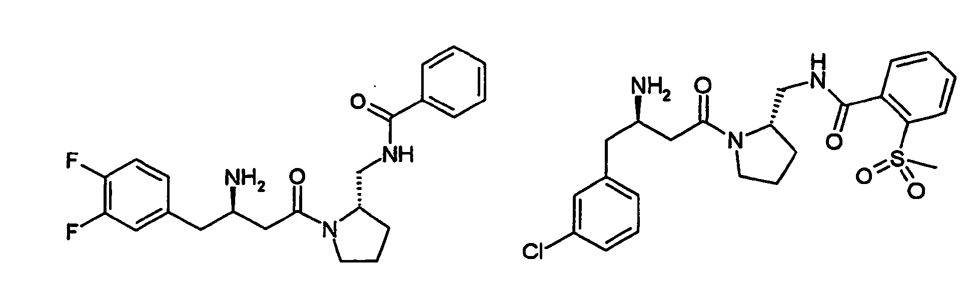

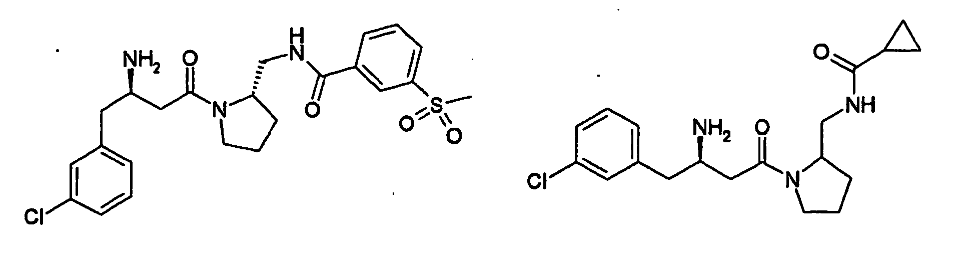

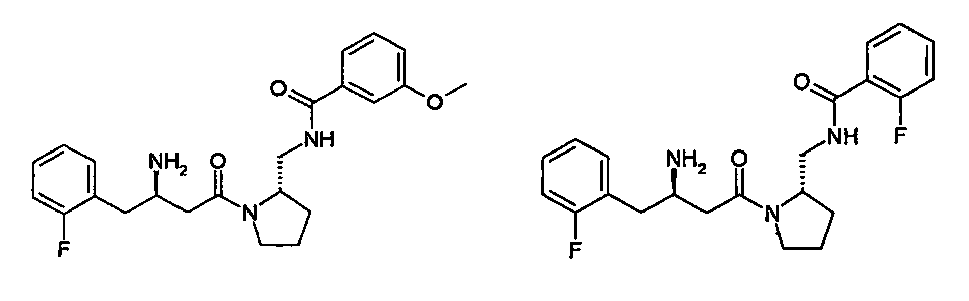

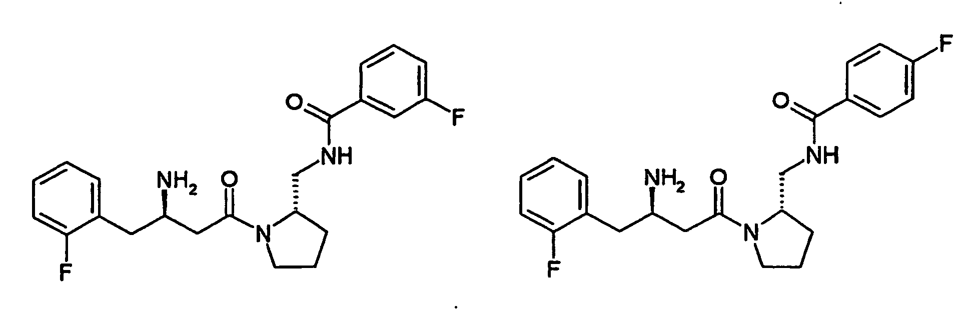

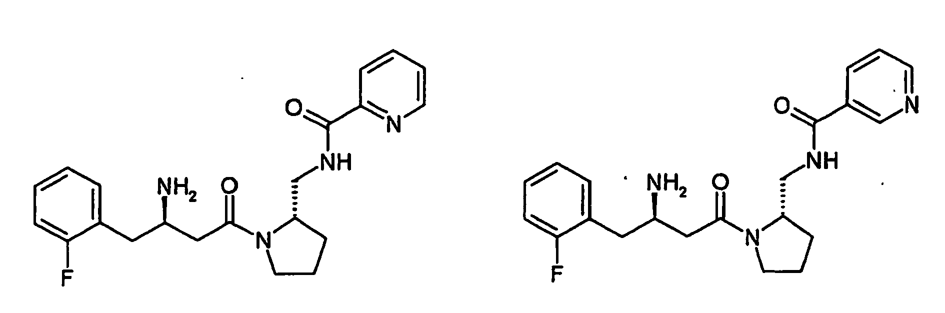

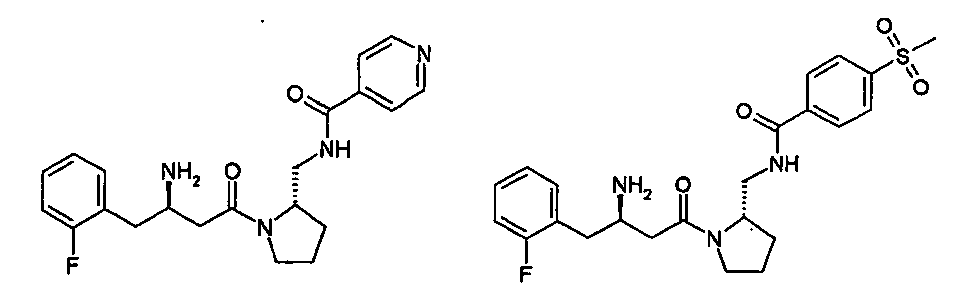

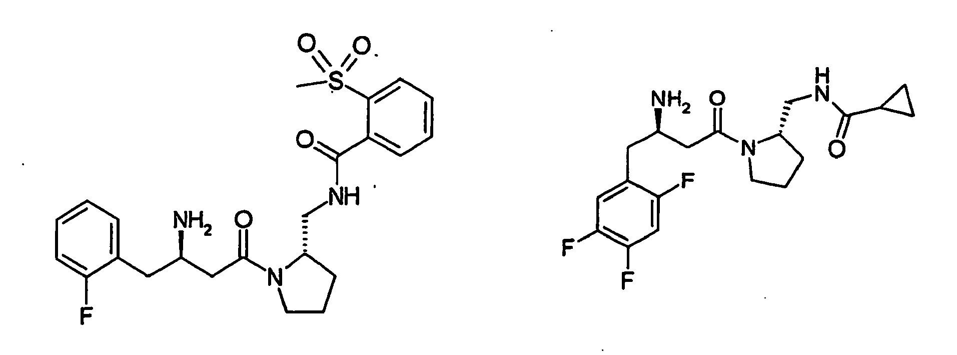

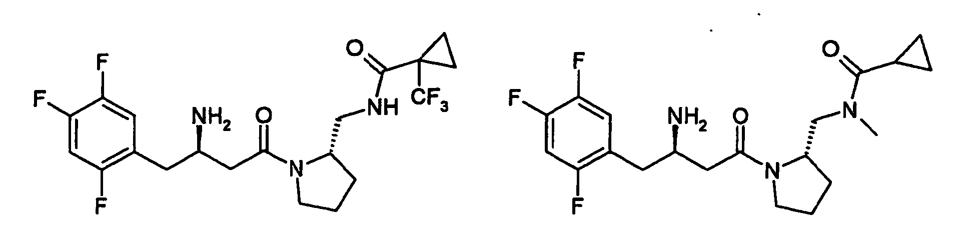

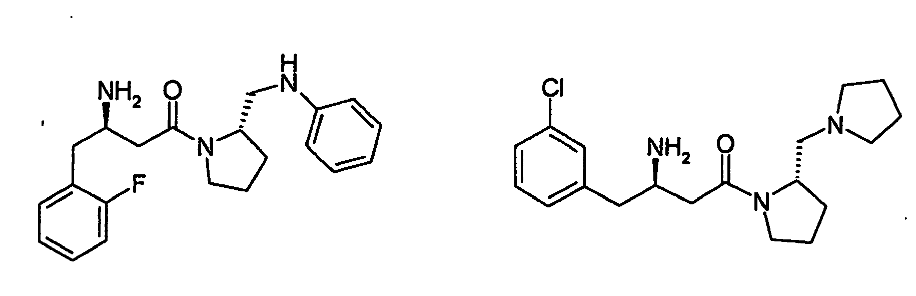

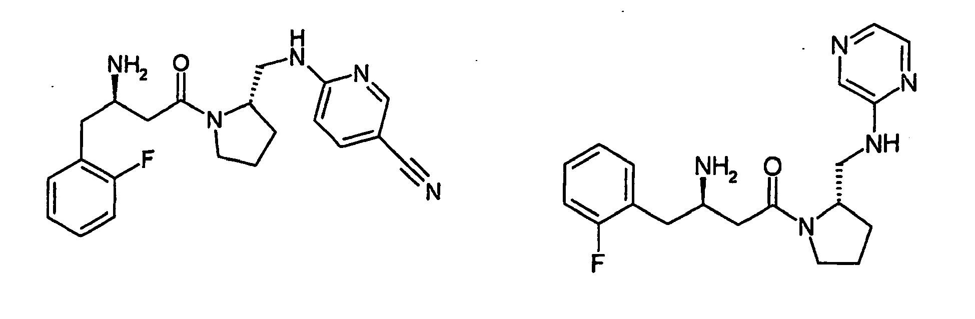

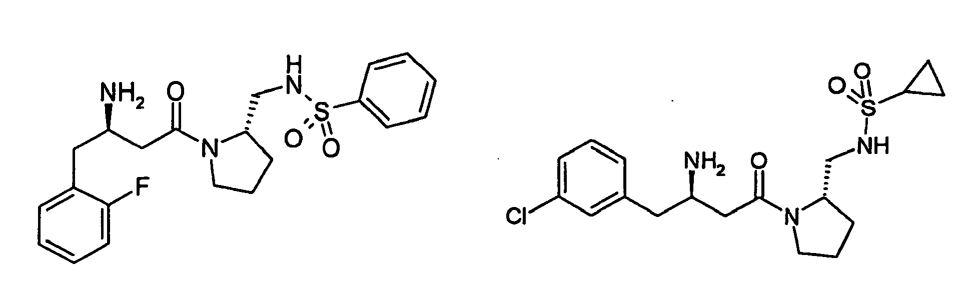

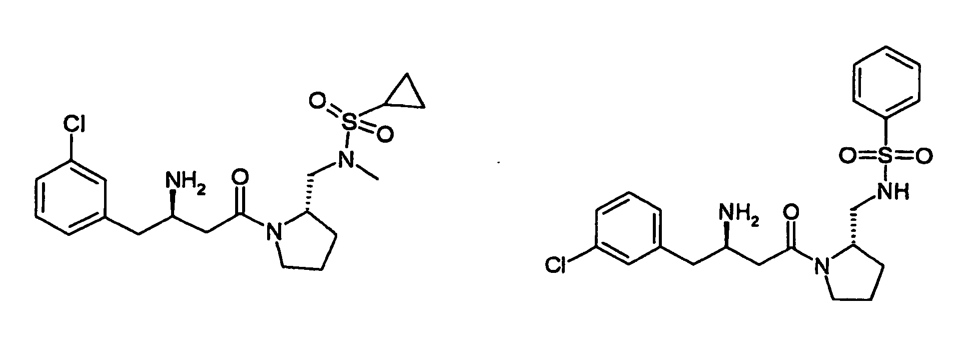

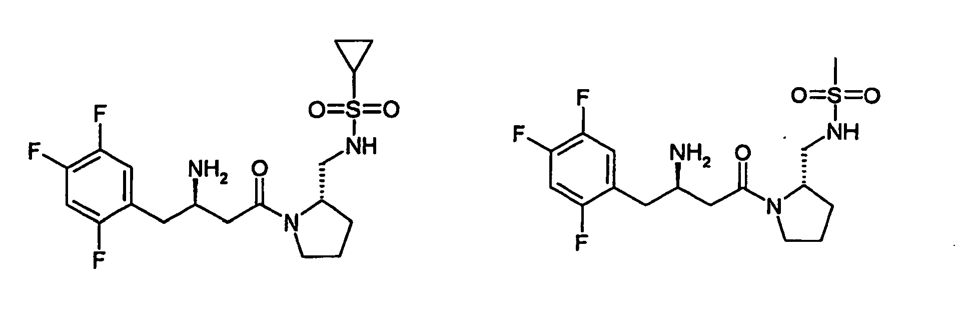

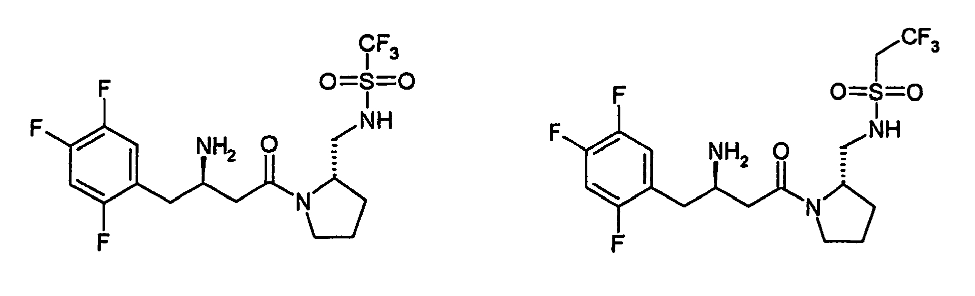

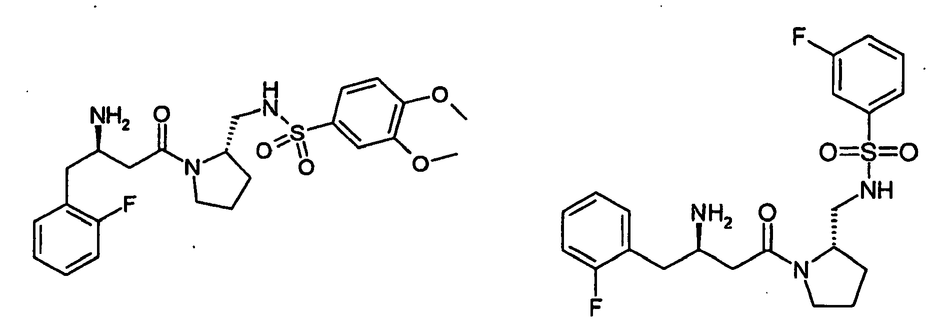

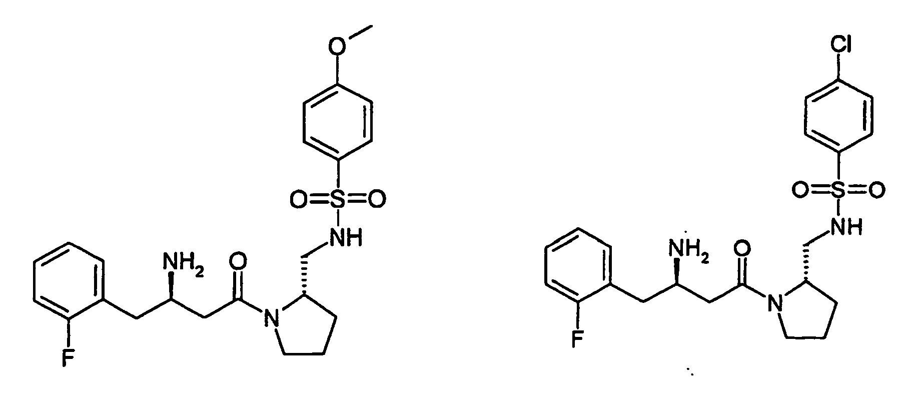

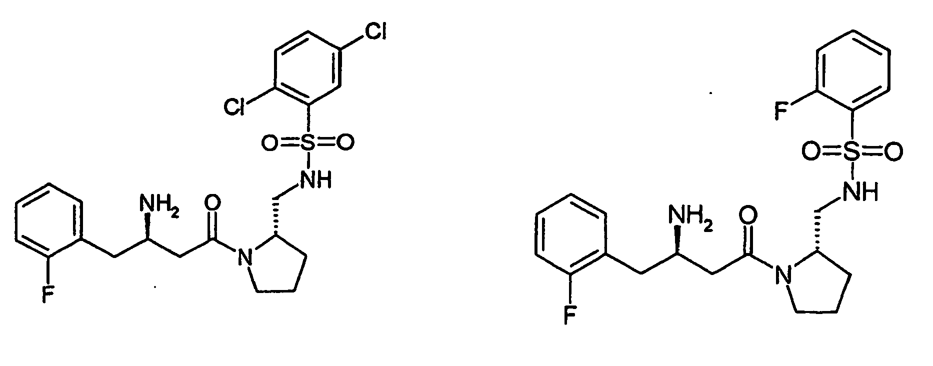

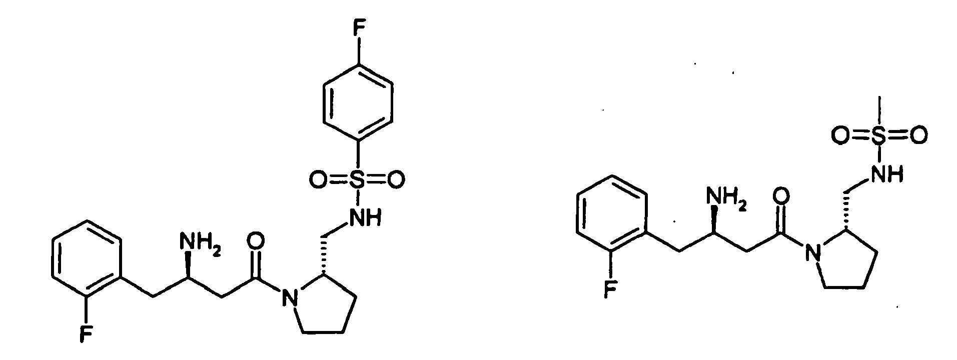

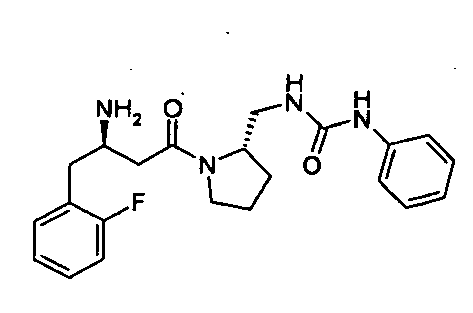

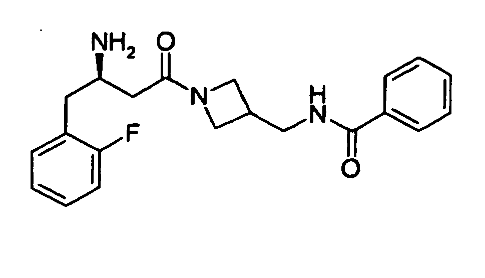

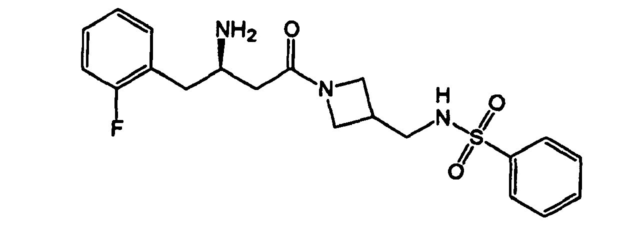

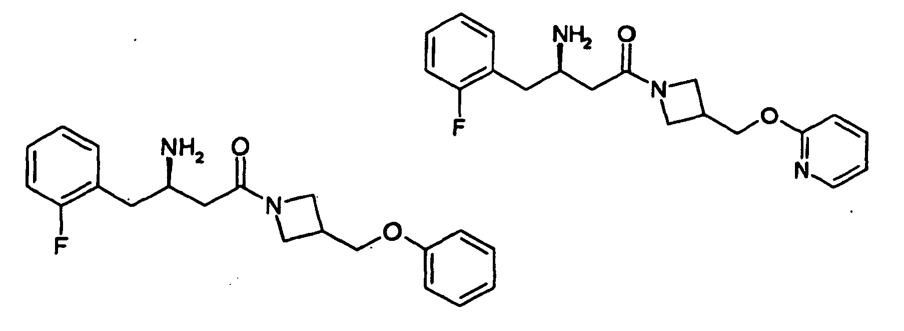

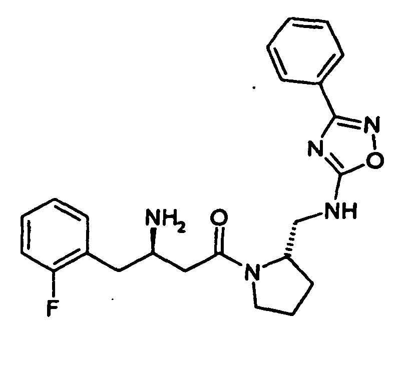

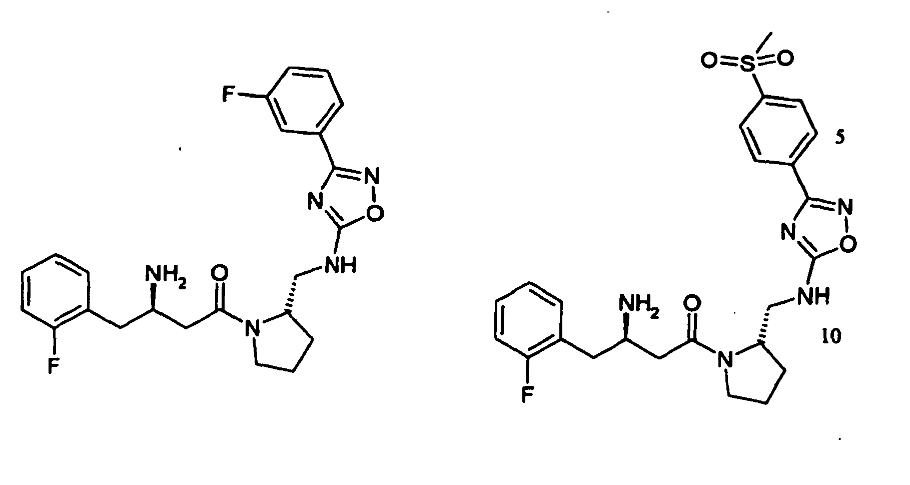

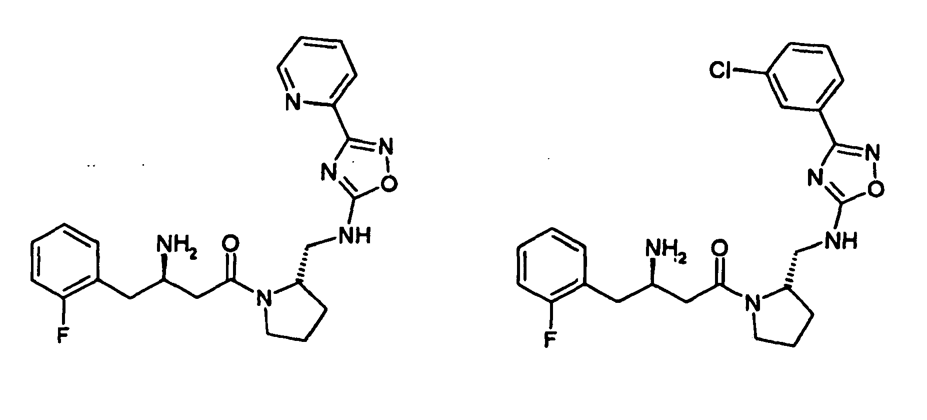

Furthermore, the present invention provides prodrug compounds of the compounds of the invention as described above.

"Prodrug compound" means a derivative that is converted into a compound according to the present invention by a reaction with an enzyme, gastric acid or the like under a physiological condition in the living body, e.g. by oxidation, reduction, hydrolysis or the like, each of which is carried out enzymatically. Examples of the prodrug are compounds, wherein the amino group in a compound of the present invention is acylated, alkylated or phosphorylated to form, e.g., eicosanoylamino, alanylamino, pivaloyloxymethylamino. These compounds can be produced from compounds of the present invention according to well-known methods.

Where tautomerism, like e.g. keto-enol tautomerism, of compounds of general formula (I) or (Ia) or their prodrugs may occur, the individual forms, like e.g. the keto and enol form, are claimed separately and together as mixtures in any ratio. Same applies for stereoisomers, like e.g. enantiomers, cis/trans isomers, conformers and the like.
If desired, isomers can be separated by methods well known in the art, e.g. by liquid chromatography. Same applies for enantiomers by using e.g. chiral stationary phases. Additionally, enantiomers may be isolated by converting them into diastereomers, i.e. coupling with an enantiomerically pure auxiliary compound, subsequent separation of the resulting diastereomers and cleavage of the auxiliary residue. Alternatively, any enantiomer of a compound of formula (I) or (Ia) may be obtained from stereoselective synthesis using optically pure starting materials.

In case the compounds according to formula (I) or (Ia) contain one or more acidic or basic groups, the invention also comprises their corresponding pharmaceutically or toxicologically acceptable salts, in particular their pharmaceutically utilizable salts.

Thus, the compounds of the formula (I) or (la) which contain acidic groups can be present on these groups and can be used according to the invention, for example, as alkali metal salts, alkaline earth metal salts or as ammonium salts. More precise examples of such salts include sodium salts, potassium salts, calcium salts, magnesium salts or salts with ammonia or organic amines such as, for example, ethylamine, ethanolamine, triethanolamine or amino acids. Compounds of the formula (I) or (la) which contain one or more basic groups, i.e. groups which can be protonated, can be present and can be used according to the invention in the form of their addition salts with inorganic or organic acids. Examples for suitable acids include hydrogen chloride, hydrogen bromide, phosphoric acid, sulfuric acid, nitric acid, methanesulfonic acid, p-toluenesulfonic acid, naphthalenedisulfonic, acids, oxalic acid, acetic acid, tartaric acid, lactic acid, salicylic acid, benzoic acid, formic acid, propionic acid, pivalic acid, diethylacetic acid, malonic acid, succinic acid, pimelic acid, fumaric acid, maleic acid, malic acid, sulfaminic acid, phenylpropionic acid, gluconic acid, ascorbic acid, isonicotinic acid, citric acid, adipic acid, and other acids known to the person skilled in the art. If the compounds of the formula (I) or (Ia) simultaneously contain acidic and basic groups in the molecule, the invention also includes, in addition to the salt forms mentioned, inner salts or betaines (zwitterions). The respective salts according to the formula (I) or (la) can be obtained by customary methods which are known to the person skilled in the art like, for example by contacting these with an organic or inorganic acid or base in a solvent or dispersant, or by anion exchange or cation exchange with other salts. The present invention also includes all salts of the compounds of the formula (I) or (Ia) which, owing to low physiological compatibility, are not directly suitable for use in pharmaceuticals but which can be used, for example, as intermediates for chemical reactions or for the preparation of pharmaceutically acceptable salts.

The present invention provides compounds of general formula (I) or (la) or their prodrugs as DPP-IV inhibitors. DPP-IV is a cell surface protein that has been implicated in a wide range of biological functions. It has a broad tissue distribution (intestine, kidney, liver, pancreas, placenta, thymus, spleen, epithelial cells, vascular endothelium, lymphoid and myeloid cells, serum), and distinct tissue and cell-type expression levels. DPP-IV is identical to the T cell activation marker CD26, and it can cleave a number of immunoregulatory, endocrine, and neurological peptides *in vitro.* This has suggested a potential role for this peptidase in a variety of disease processes.

DPP-IV related diseases are described in more detail in WO-A-03/181 under the paragraph "Utilities".

Accordingly, the present invention provides compounds of formula (I) or (Ia) or their prodrugs or pharmaceutically acceptable salt thereof for use as a medicament.

Furthermore, the present invention provides the use of compounds of formula (I) or (Ia) or their prodrugs or a pharmaceutically acceptable salt thereof for the manufacture of a medicament for the treatment or prophylaxis of non-insulin dependent (Type II) diabetes mellitus; hyperglycemia; obesity; insulin resistance; lipid disorders; dyslipidemia; hyperlipidemia; hypertriglyceridemia; hypercholestrerolemia; low HDL; high LDL; atherosclerosis; growth hormone deficiency; diseases related to the immune response; HIV infection; neutropenia; neuronal disorders; anxiety; depression; tumor metastasis; benign prostatic hypertrophy; gingivitis; hypertension; osteoporosis; diseases related to sperm motility; low glucose tolerance; insulin resistance; ist sequelae; vascular restenosis; irritable bowel syndrome; inflammatory bowel disease; including Crohn's disease and ulcerative colitis; other inflammatory conditions; pancreatitis; abdominal obesity; neurodegenerative disease; retinopathy, nephropathy; neuropathy; Syndrome X; ovarian hyperandrogenism (polycystic ovarian syndrome; Type n diabetes; or growth hormone deficiency. Preferred is non-insulin dependent (Type II) diabetes mellitus and obesity.

The present invention provides pharmaceutical compositions comprising a compound of formula (I) or (Ia), or a prodrug compound thereof, or a pharmaceutically acceptable salt thereof as active ingredient together with a pharmaceutically acceptable carrier.

"Pharmaceutical composition" means one or more active ingredients, and one or more inert ingredients that make up the carrier, as well as any product which results, directly or indirectly, from combination, complexation or aggregation of any two or more of the ingredients, or from dissociation of one or more of the ingredients, or from other types of reactions or interactions of one or more of the ingredients. Accordingly, the pharmaceutical compositions of the present invention encompass any composition made by admixing a compound of the present invention and a pharmaceutically acceptable carrier.

A pharmaceutical composition of the present invention may additionally comprise one or more other compounds as active ingredients like one or more additional compounds of formula (I) or (Ia), or a prodrug compound or other DPP-IV inhibitors.
Other active ingredients are disclosed in WO-A-03/181 under the paragraph "Combination Therapy". Accordingly, other active ingredients may be insulin sensitizers; PPAR agonists; biguanides; protein tyrosinephosphatase-IB (PTP-1 B) inhibitors; insulin and insulin mimetics; sulfonylureas and other insulin secretagogues; a-glucosidase inhibitors; glucagon receptor antagonists; GLP-1, GLP-1 mimetics, and GLP-1 receptor agonists; GIP, GIP mimetics, and GIP receptor agonists; PACAP, PACAP mimetics, and PACAP receptor 3 agonists; cholesterol lowering agents; HMG-CoA reductase inhibitors; sequestrants; nicotinyl alcohol; nicotinic acid or a salt thereof; PPARa agonists; PPARoly dual agonists; inhibitors of cholesterol absorption; acyl CoA : cholesterol acyltransferase inhibitors; anti-oxidants; PPARo agonists; antiobesity compounds; an ileal bile acid transporter inhibitor; or anti-inflammatory agents or pharmaceutically acceptable salts of these active compounds.

The term "pharmaceutically acceptable salts" refers to salts prepared from pharmaceutically acceptable non-toxic bases or acids, including inorganic bases or acids and organic bases or acids.

The compositions include compositions suitable for oral, rectal, topical, parenteral (including subcutaneous, intramuscular, and intravenous), ocular (ophthalmic), pulmonary (nasal or buccal inhalation), or nasal administration, although the most suitable route in any given case will depend on the nature and severity of the conditions being treated and on the nature of the active ingredient. They may be conveniently presented in unit dosage form and prepared by any of the methods well-known in the art of pharmacy.

In practical use, the compounds of formula (I) or (Ia) can be combined as the active ingredient in intimate admixture with a pharmaceutical carrier according to conventional pharmaceutical compounding techniques. The carrier may take a wide variety of forms depending on the form of preparation desired for administration, *e*.*g*., oral or parenteral (including intravenous). In preparing the compositions for oral dosage form, any of the usual pharmaceutical media may be employed, such as, for example, water, glycols, oils, alcohols, flavoring agents, preservatives, coloring agents and the like in the case of oral liquid preparations, such as, for example, suspensions, elixirs and solutions; or carriers such as starches, sugars, microcrystalline cellulose, diluents, granulating agents, lubricants, binders, disintegrating agents and the like in the case of oral solid preparations such as, for example, powders, hard and soft capsules and tablets, with the solid oral preparations being preferred over the liquid preparations.

Because of their ease of administration, tablets and capsules represent the most advantageous oral dosage unit form in which case solid pharmaceutical carriers are obviously employed. If desired, tablets may be coated by standard aqueous or nonaqueous techniques. Such compositions and preparations should contain at least 0.1 percent of active compound. The percentage of active compound in these compositions may, of course, be varied and may conveniently be between about 2 percent to about 60 percent of the weight of the unit. The amount of active compound in such therapeutically useful compositions is such that an effective dosage will be obtained. The active compounds can also be administered intranasally as, for example, liquid drops or spray.

The tablets, pills, capsules, and the like may also contain a binder such as gum tragacanth, acacia, com starch or gelatin; excipients such as dicalcium phosphate; a disintegrating agent such as com starch, potato starch, alginic acid; a lubricant such as magnesium stearate; and a sweetening agent such as sucrose, lactose or saccharin. When a dosage unit form is a capsule, it may contain, in addition to materials of the above type, a liquid carrier such as a fatty oil.

Various other materials may be present as coatings or to modify the physical form of the dosage unit. For instance, tablets may be coated with shellac, sugar or both. A syrup or elixir may contain, in addition to the active ingredient, sucrose as a sweetening agent, methyl and propylparabens as preservatives, a dye and a flavoring such as cherry or orange flavor.

Compounds of formula (I) or (Ia) may also be administered parenterally. Solutions or suspensions of these active compounds can be prepared in water suitably mixed with a surfactant such as hydroxy-propylcellulose. Dispersions can also be prepared in glycerol, liquid polyethylene glycols and mixtures thereof in oils. Under ordinary conditions of storage and use, these preparations contain a preservative to prevent the growth of microorganisms.

The pharmaceutical forms suitable for injectable use include sterile aqueous solutions or dispersions and sterile powders for the extemporaneous preparation of sterile injectable solutions or dispersions. In all cases, the form must be sterile and must be fluid to the extent that easy syringability exists. It must be stable under the conditions of manufacture and storage and must be preserved against the contaminating action of microorganisms such as bacteria and fungi. The carrier can be a solvent or dispersion medium containing, for example, water, ethanol, polyol (e.g., glycerol, propylene glycol and liquid polyethylene glycol), suitable mixtures thereof, and vegetable oils.
Any suitable route of administration may be employed for providing a mammal, especially a human, with an effective dose of a compound of the present invention. For example, oral, rectal, topical, parenteral, ocular, pulmonary, nasal, and the like may be employed. Dosage forms include tablets, troches, dispersions, suspensions, solutions, capsules, creams, ointments, aerosols, and the like. Preferably compounds of formula (I) or (la) are administered orally.

The effective dosage of active ingredient employed may vary depending on the particular compound employed, the mode of administration, the condition being treated and the severity of the condition being treated. Such dosage may be ascertained readily by a person skilled in the art.

When treating or preventing diabetes mellitus and/or hyperglycemia or hypertriglyceridemia or other diseases for which compounds of Formula I are indicated, generally satisfactory results are obtained when the compounds of the present invention are administered at a daily dosage of from about 0.1 milligram to about 100 milligram per kilogram of animal body weight, preferably given as a single daily dose or in divided doses two to six times a day, or in sustained release form. For most large mammals, the total daily dosage is from about 1.0 milligrams to about 1000 milligrams, preferably from about 1 milligrams to about 50 milligrams. In the case of a 70 kg adult human, the total daily dose will generally be from about 7 milligrams to about 350 milligrams. This dosage regimen may be adjusted to provide the optimal therapeutic response.

The compounds of formula (I) of the present invention can be prepared from beta amino acid intermediates such as those of formula (IV) and substituted amine intermediates such as those of formula (III), using standard peptide coupling conditions. The preparation of these intermediates is described in the following schemes.

**Some abbreviations that may appear in this application are as follows.**

### ABBREVIATIONS

### Designation

- bs: Broad singlet
- bm: Broad multiplet
- Boc (or BOC): *tert*-Butoxycarbonyl
- CDI: *N*,*N*-Carbonyldiimidazole
- DCE: 1,2-Dichloroethane
- DCM: Dichloromethane
- DIEA: Diisopropylethylamine
- DMF: *N,N*-Dimethylformamide
- EDC: 1-Ethyl-3-(3-dimethylaminopropyl)carbodiimide hydrochloride
- Et₃N: Triethylamine
- Fmoc: 9-Fluorenylmethoxycarbonyl
- HATU: *O*-(7-Azabenzotriazol-1-yl)-*N*,*N*,*N'*,*N'*-tetramethyluronium hexafluorophosphate
- HCl: Hydrogen chloride
- HOBt: 1-Hydroxybenzotriazole
- HPLC: High pressure liquid chromatography
- M. P.: Melting point
- NMR: Nuclear Magnetic Resonance
- PG: Protecting group
- rt: Retention time
- ^{t}BuOH: *tert-*Butanol
- TFA: Trifluoroacetic acid
- TLC: Thin Layer Chromatography

Available starting materials may be amines having the formula (III).

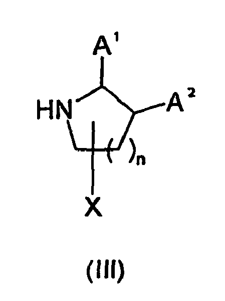

They may be purchased from commercially available sources such as Acros, Astatech, Array, Sigma-Aldrich, Fluka, ABCR or be synthesized by one skilled in the art. Common reactions between compounds containing amino groups and carboxyl, sulfonyl or isocyanate functionalities may be employed for their synthesis with suitable functionalized starting materials. Nucleophilic substitution reactions between compounds containing a suitable leaving group (e.g., halogenide, mesylate, tosylate) and nucleophiles (e.g., amines) may be also employed. The conversion of diverse functional groups (such as esters, alcohols, amides, nitriles, azides) may allow the synthesis of some intermediates or final compounds.
Schemes A through G outline general procedures for the synthesis of some compounds described below. Unless otherwise indicated in the schemes, the variables have the same meaning as described above.

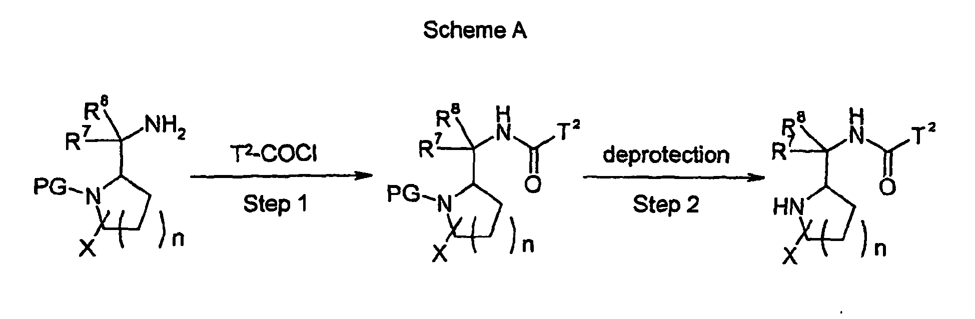

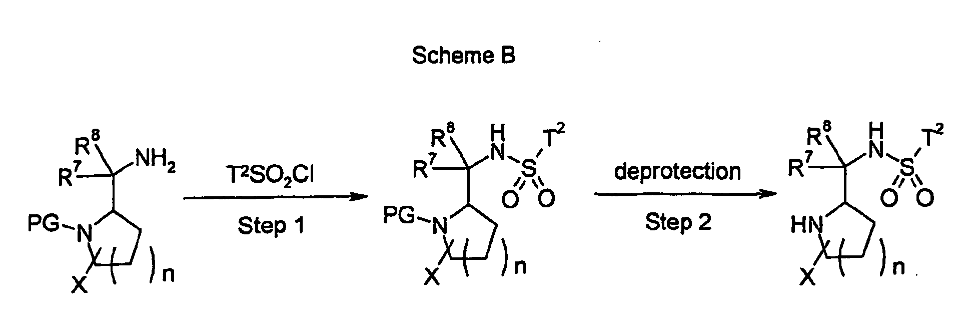

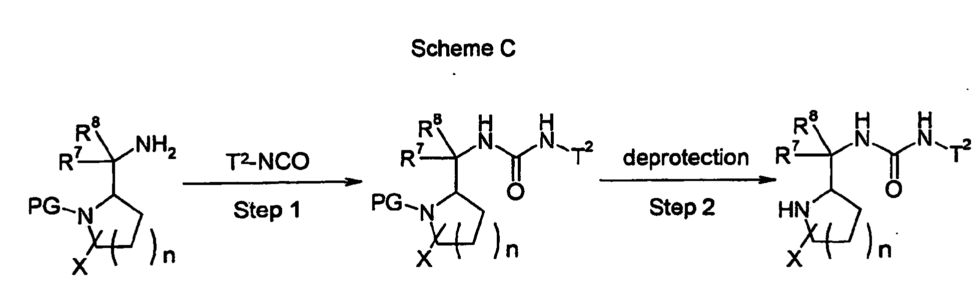

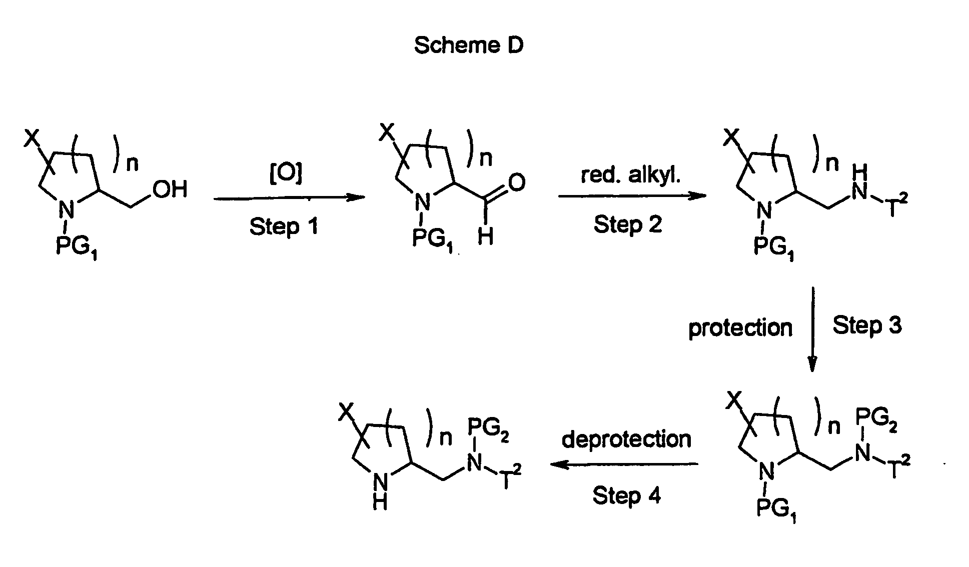

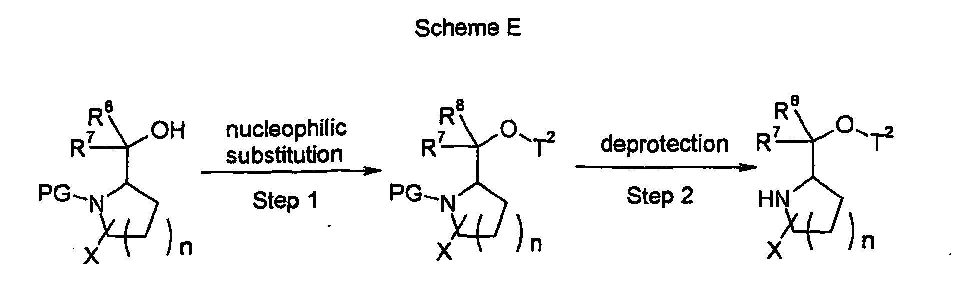

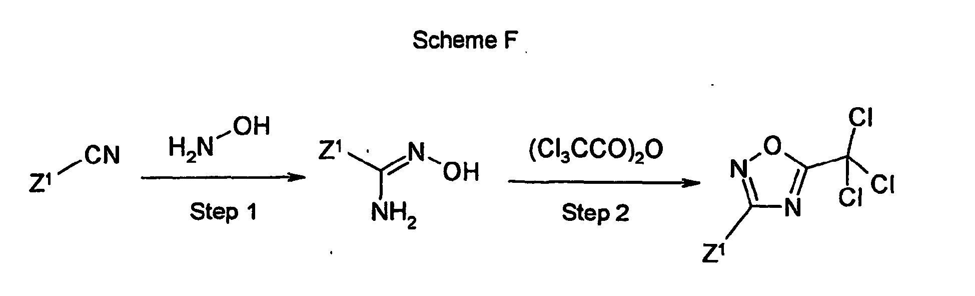

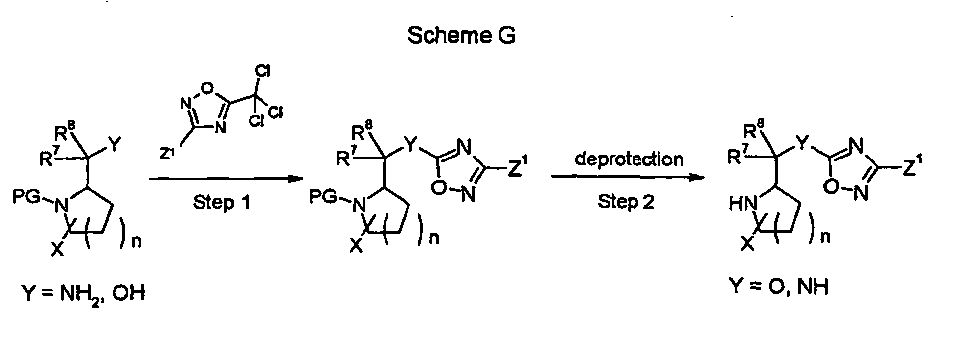

Enantiomerically pure beta amino acids having the formula (IV) 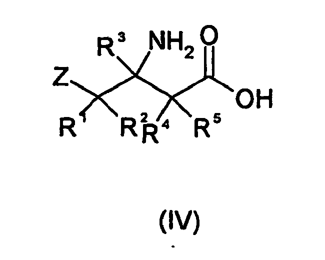 may be commercially available, known in the literature or may be conveniently synthesized using one of the methods already published and reviewed in e.g., Cole, Tetrahedron, 32, 9517 (1994), Juaristi et al., Aldrichimica Acta, 27, 3, 1994, or Juaristi, Enantioselective Synthesis of β-Amino Acids, Ed. Wiley-VCH, New York, 1997.
In particular, 3-amino-4-(2,4,5-trifluoro-phenyl)-butyric acid may be synthesized by a variety of methods as reported in the patent applications WO 2004069162, WO 2004064778, WO 2004037169, WO 2004032836 and in the articles JACS, 126, 3048 (2004) and JACS, 126, 9918 (2004).

Unless otherwise noted, all non-aqueous reactions were carried out under argon atmosphere with commercial dry solvents. Compounds were purified using flash column chromatography using Merck silica gel 60 (230-400 mesh) or reverse phase preparative HPLC using a Reprosil-Pur ODS3, 5 µm, 20 x 125 mm column with Shimadzu LC8A-Pump and SPD-10Avp UV/Vis diode array detector. The ¹H-NMR spectra were recorded on a Varian VXR-S (300 MHz for ¹H-NMR) using d₆-dimethylsulfoxide as solvent; chemical shifts are reported in ppm relative to tetramethylsilane. Analytical LC/MS was performed using Reprosil-Pur ODS3, 5 µM, 1 x 60 mm columns with a linear gradient from 5% to 95% acetonitrile in water (0.1% TFA) at a flow rate of 250 µl/min; retention times are given in minutes. Methods are:
(I) runs on a LC10Advp-Pump (Shimadzu) with SPD-M10Avp UV/Vis diode array detector and QP2010 MS-detector in ESI+ modus with UV-detection at 214, 254 and 275 nm, 10 min. linear gradient; (II) idem but 5 min. linear gradient; (III) runs on a LC10Advp-Pump (Shimadzu) with SPD-10Avp dual wavelength UV-detector and QP2010 MS-detector in ESI+ modus with UV-detection at 214 and 254 nm, 10 min. linear gradient; (IV) idem but 5 min. linear gradient; (V) runs on a LC10Advp-Pump (Shimadzu) with SPD-M10Avp UV/Vis diode array detector and QP2010 MS-detector in ESI+ mode with UV-detection at 214, 254 and 275 nm, with a linear gradient different from 5% to 95% acetonitrile in water (0.1 % TFA or formic acid). In this case the data will be reported as follows:
   LC/MS (V) (5-90%, 5 min): rt 1.60, m/z 171 (M+H)⁺ ; (VI) runs on a LC10Advp-Pump (Shimadzu) with SPD-10Avp dual wavelength UV-detector and QP2010 MS-detector in ESI+ modus with UV-detection at 214 and 254 nm, with a linear gradient different from 5% to 95% acetonitrile in water (0.1% TFA or formic acid). In this case the data will be reported as follows: LC/MS (VI) (5-90%, 5 min): rt 1.60, m/z 171 (M+H) ⁺; method (VII) is run on a LiChroCART 30-4 Purospher STAR RP-18, endcapped, 3 µm (Merck) column. Gradient elution using eluent (A): acetonitrile/water (5:95) with a 20 mM HCO₂NH₄/NH₄OH buffer at pH 7.4. Eluent (B): acetonitrile/water (80:20) with a 20 mM HCO₂NH₄/NH₄OH buffer at pH 7.4. Gradient: 0 minutes 70:30 (%A:%B); 2.5 minutes 5:95 (%A:%B); 4.3 minutes 5:95 (%A:%B); 4.4 minutes 70:30 (%A:%B); 5 minutes 70:30 (%A:%B). Flow rate 1.5 mL/minute; UV-detection 220 nM.

### General procedure for making compounds of the invention

In general, compounds having the formula (I) 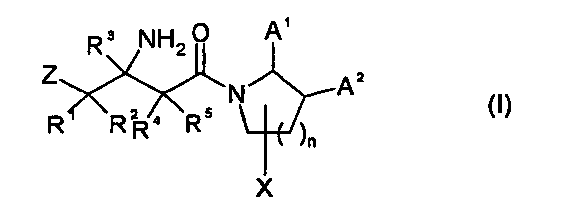 wherein the variables have the above described meanings, may be prepared using standard peptide coupling conditions, reagents and protective groups. For example, it may be possible to use 1-ethyl-3-(3-dimethylaminopropyl)carbodiimide hydrochloride (EDC) in combination with 1-hydroxybenzotriazole (HOBt) and a base (triethylamine or diisopropylethylamine) or O-(7-azabenzotriazol-1-yl)-N,N,N',N'-tetramethyluronium hexafluorophosphate (HATU) in the presence of a base, in solvents such as methylene chloride or N,N-dimethylformamide.

Scheme H outlines a procedure for using the amines formed according to Schemes A through G to synthesize compounds that are embodiments of the invention.

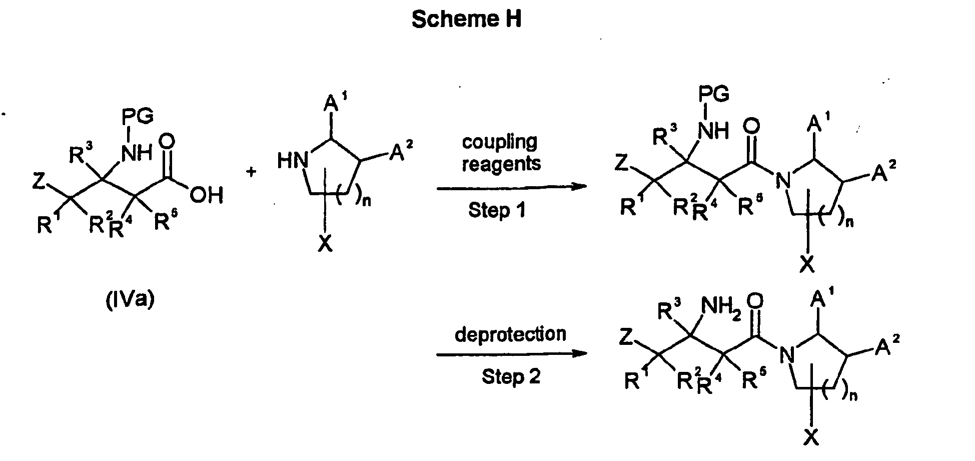

The protective group may be removed with, for example, diethylamine in dichloromethane in the case of 9-fluorenylmethoxycarbonyl or using acidic conditions (such as trifluoroacetic acid in dichloromethane or hydrochloric acid in dioxane) in the case of tert-butoxycarbonyl, as described in Protective Groups in Organic Synthesis 3rd ed., Ed. Wiley-VCH, New York; 1999.
For the purification of intermediates or end products, flash chromatography on silica gel may be suitable for the free amines whereas the use of preparative HPLC leads to the isolation of the corresponding trifluoroacetic acid or formate salts.

### EXAMPLES

The following examples are provided so that the invention might be more fully understood. These examples are illustrative only and should not be construed as limiting the invention in any way.

### PREPARATIONS

### Example 1

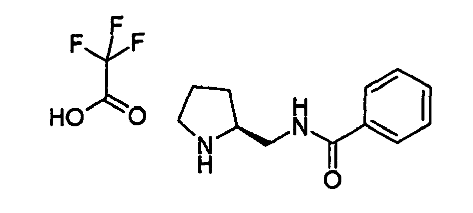

### Step 1

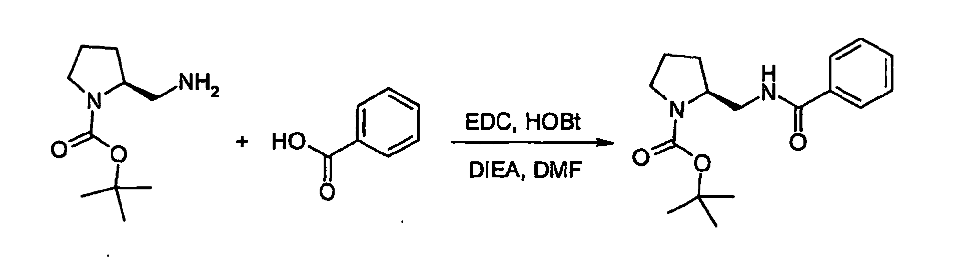

### (2S)-(Benzoylamino-methyl)-pyrrolidine-1-carboxylic acid tert-butyl ester.

A mixture of 127 mg (1.04 mmol) of benzoic acid, 219 mg (1.14 mmol) of 1-ethyl-3-(3-dimethylaminopropyl)carbodiimide hydrochloride (EDC), 154 mg (1.14 mmol) of 1-hydroxybenzotriazole (HOBt) and 271 µl (1.56 mmol) of diisopropylethylamine (DIEA) in 2 mL of *N,N*-dimethylformamide is stirred at room temperature for 10 minutes, before a solution of 250 mg (1.24 mmol) of (2S)-2-aminomethyl-pyrrolidine-1-carboxylic acid *tert*-butyl ester in 2 mL of *N,N*-dimethylformamide is added and stirring continued overnight The solution is diluted with 50 mL of ethyl acetate, washed sequentially with 5% citric acid aqueous solution, saturated aqueous sodium bicarbonate solution, and brine, dried over sodium sulphate and the solvent is removed under reduced pressure. Purification of the crude product by flash chromatography (silica gel, eluent: 0% to 10 % of ethyl acetate in cyclohexane) gives the title compound.
¹H-NMR δ (ppm) = 1.40 (s, 9H), 1.75-1.88 (m, 4H), 3.39-3.50 (m, 1H), 3.90-3.98 (m, 1H), 7.41-7.47 (m, 4H), 7.78-7.81 (m, 1H), 8.34-8.39 (m, 1H).
LC/MS (IV) rt 2.79, m/z 368 (M+Na+CH₃CN)⁺.

### Step 2

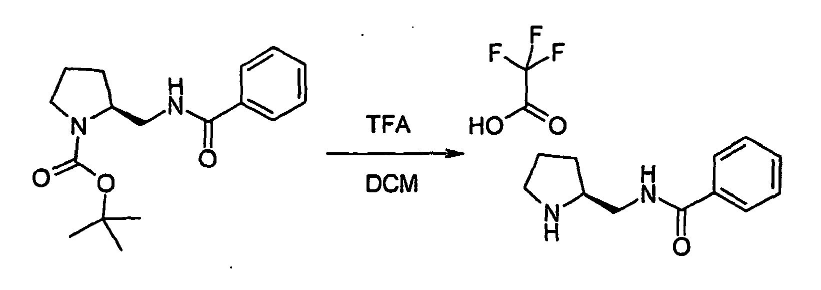

### N-Pyrrolidin-(2S)-ylmethyl-benzamide (TFA salt).

A solution of 20.0 mg (0.07 mmol) of *(2S)*-(benzoylamino-methyl)-pyrrolidine-1-carboxylic acid *tert*-butyl ester (step 1) in 1.0 mL of dichloromethane and 0.5 mL of trifluoroacetic acid is stirred at room temperature for 30 minutes and then evaporated under reduced pressure to give the title compound.

The intermediates in Table 1 are synthesized according to the procedure shown for Example 1.

**TABLE 1**

| Example | Structure | LC-MS | NMR |
|---|---|---|---|
| 2 | 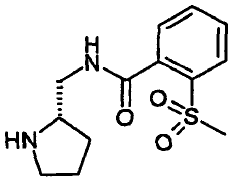 | LC/MS (V) (5-90%, 5 min): rt 2.53, m/z 405 (M+H+Na+AcCN)⁺. | |
| 3 | 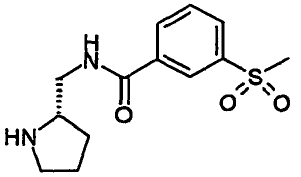 | LC/MS (V) (5-90%, 5 min): rt 2.57, m/z 405 (M+H+Na) ⁺. | |
| 4 | 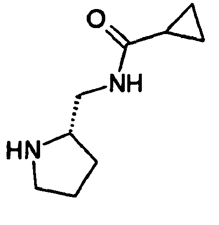 | LC/MS (II) rt 0.90 and 1.20, m/z 169 (M+H)⁺. | Boc-protected compound (Step 1): ¹H-NMR δ (ppm) = 0.61-0.65 (m, 4H), 1.40 (s, 9H), 1.49- 1.61 (m, 1H), 1.63-1.85 (m, 4H), 2.85-3.10 (m, 1H), 3.15-3.30 (m, 2H), 3.39-3.50 (m, 1H). 3.68-3.78 (m, 1 H), 8.08 (bs, 1 H). |
| 5 | 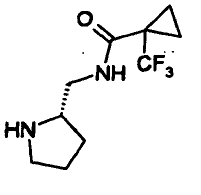 | LC/MS (II) rt 1.80 m/z 237 (M+H)⁺. | Boc-protected compound (Step 1): 1.19 (m, 2H), 1.21 (m, 2H), 1.40 (s, 9H), 1.60-1.85.(m, 4H), 2.99-3.22 (m, 3H), 3.38-3.52 (m, 1 H), 3.73-3.90 (m, 1H). 7.91 (bs, 1H). |
| 6 | 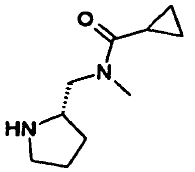 | LC/MS (11) rt 1.30 m/z 183 (M+H)⁺. | |
| 8 | 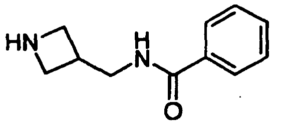 | LC/MS (II) rt 1.58 m/z 191 (M+H)⁺ | |

### Example 10

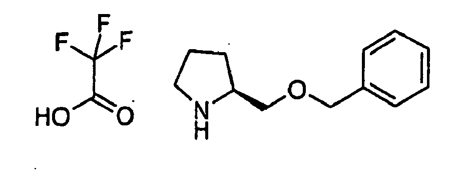

### Step 1

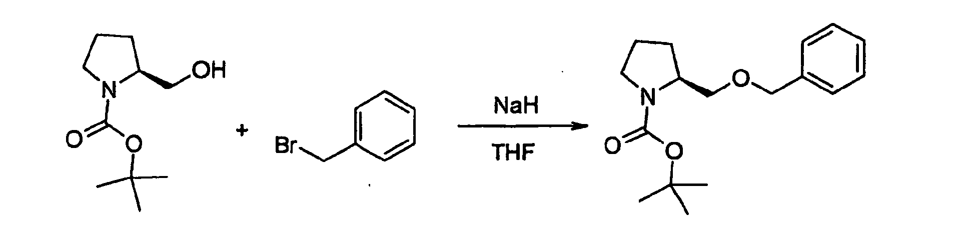

*(2S)*-Benzyloxymethyl-pyrrolidine-1-carboxylic acid *tert-*butyl ester. (For the synthesis see also J. Med. Chem.; 42; 4; 1999; 677-690)

A solution of 500 mg (2.48 mmol) of *(2S)*-hydroxymethyl-pyrrolidine-1-carboxylic acid *tert-*butyl ester in 2 mL of tetrahydrofuran is added dropwise to a slurry of 119.2 mg (60% dispersion in oil, 2.98 mmol) of sodium hydride in 2 mL of tetrahydrofuran at 0 °C and the mixture stirred for 5 minutes. 325 µL (119 mg, 2.73 mmol) of benzylbromide is added and the reaction is allowed to warm to room temperature and stirred overnight. Water and 1 N hydrochloric acid solution are added and the mixture is extracted with ethyl acetate. The collected organic phases are washed sequentially with a saturated aqueous sodium bicarbonate solution, brine and water, then dried over sodium sulphate and evaporated under reduced pressure. The crude mixture is purified using flash chromatography (silica gel, eluent: 0% to 10% ethyl acetate in cyclohexane) to afford the title compound.
LC/MS (IV) rt 3.41, m/z 233 (M+H-Boc+AcCN)⁺.

### Step 2

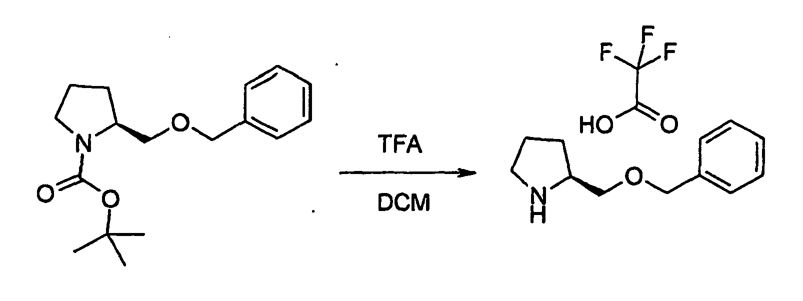

### (2S)-Benzyloxymethyl-pyrrolidine, (TFA salt).

A solution of 300 mg (1.03 mmol) of *(2S)*-benzyloxymethyl-pyrrolidine-1-carboxylic acid *tert-*butyl ester (step 1) in 1.5 mL of dichloromethane and 1.5 mL of trifluoroacetic acid is stirred at room temperature for 1 h and then evaporated under reduced pressure. The crude mixture is diluted in 5 mL of dichloromethane and stirred for 1 h with 1.43 g (4.12 mmol) of (polystyrylmethyl)trimethylammonium bicarbonate, then filtered and evaporated under reduced pressure to give the title compound.
¹H-NMR δ (ppm) = 1.34-1.42 (m, 1H), 1.59-1.81 (m, 3H), 2.76-2.86 (m, 2H), 3.27-3.33 (m, 4H), 4.47 (s, 2H), 7.29-7.24 (m, 5H).
LC/MS (III) rt 2.62, m/z 192 (M+H)⁺.

### Example 11

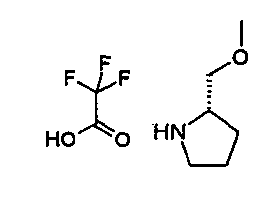

### Step 1

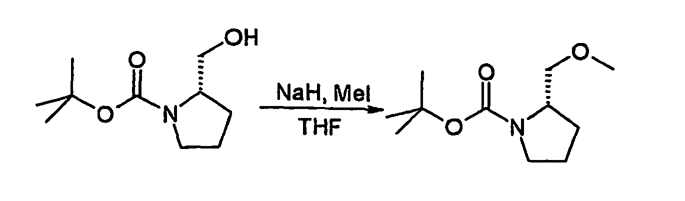

### 2-Methoxymethyl-pyrrolidine-1-carboxylic acid tert-butyl ester

A solution of 150 mg (0.75 mmol) of N-Boo-prolinol and 33 mg (0.82 mmol) of sodium hydride in 1 mL of tetrahydrofuran is stirred for 10 minutes at room temperature. 128 mg (0.90 mmol) of methyliodide in 0.5 mL THF are added and the reaction is stirred 1h. Methanol is added and the solvent is evaporated under reduced pressure. To the crude material 1N hydrochloric acid solution is added and the mixture is extracted with ethyl acetate. The collected organic phases are washed with brine and water, then dried over sodium sulphate and evaporated under reduced pressure. The crude mixture is purified using flash chromatography (silica gel) to afford the title compound.
LC/MS (II) rt 4.46, m/z 201 (M+H-CH₃)⁺.

### Step 2

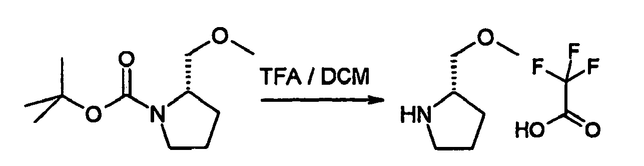

### 2-Methoxymethyl-pyrralidine, (TFA salt).

A solution of 51 mg (0.24 mmol) of the product from step 1 in 1 mL of trifluoroacetic acid and 2 mL of dichloromethane is stirred at room temperature for 1 h and then evaporated under reduced pressure. The product is isolated in the form of a TFA-salt. ¹H-NMR δ (ppm) = 1.50-1.65 (m, 1 H), 1.80-2.10 (m, 3H), 3.05-3.25 (m, 2H), 3.30 (s, 3H), 3.40-3.46 (m, 1H), 3.51-3.56 (m, 1 H), 3.60-3.75 (m, 1H), 8.55 (s, 3H), 9.18 (s, 3H).

### Example 12

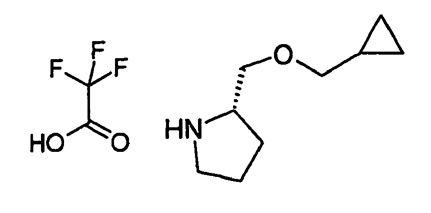

### Step 1

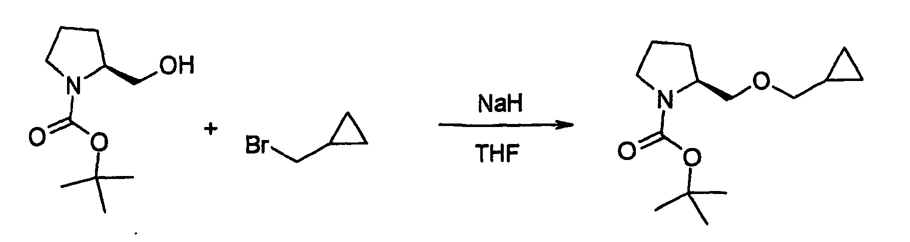

### 2-Cyclopropylmethoxymethyl-pyrrolidine-1-carboxylic acid tert-butyl ester

To a solution of 100 mg (0.50 mmol) of (2S)-hydroxymethyl-pyrrolidine-1-carboxylic acid *tert*-butyl ester in 500 µL tetrahydrofuran is added sodium hydride (40 mg, 60% dispersion in oil, 0.99 mmol) and the mixture is stirred for 10 minutes. (Bromomethyl)cyclopropane (202 mg, 1.49 mmol) is added and the reaction is heated in the microwave for 10 minutes at 100 °C. Ethyl acetate is added and the mixture is washed with brine (3x), then dried over sodium sulphate and evaporated under reduced pressure. The crude mixture is purified using flash chromatography (silica gel, eluent: 10% ethyl acetate in cyclohexane) to afford the title compound.
LC/MS (II) rt 4.55, m/z 256 (M+H-CH₃)⁺.

### Step 2

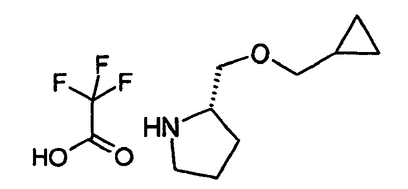

### 2-Cyclopropylmethoxymethyl-pyrrolidine, (TFA salt)

Obtained from the product of step 1 according to the procedure described for steps 2 in Example 1.

### Example 13

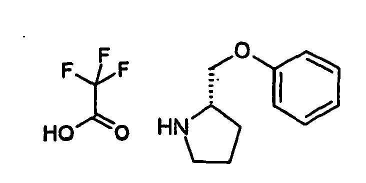

### Step 1

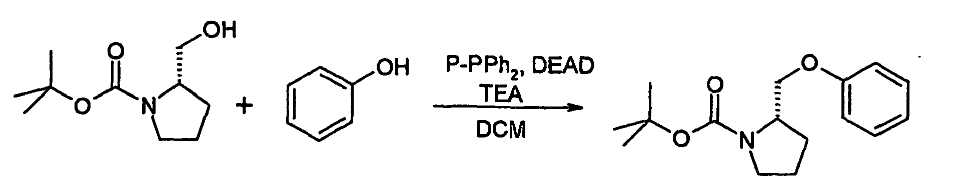

### 2-Phenoxymethyl-pyrrolidine-1-carboxylic acid tert-butyl ester

To 1.02 g (1.12 mmol) of polymer bound triphenylphosphine in 5 mL dichloromethane 156 mg (0.89 mmol), diethylazodicarboxylate (DEAD) is added at 0 °C and the mixture is stirred for 5 minutes. To the mixture a solution of 150 mg (0.75 mmol) of Boc-L-prolinol, 70 mg (0.75 mmol) of phenol and 116 µl (1.13 mmol) of triethylamine in 2 mL dichloromethane are added and the reaction is allowed to warm to room temperature and stirred over 60 h. The polymer is filtered off and the solution is evaporated under reduced pressure. The crude mixture is purified using flash chromatography (silica gel, eluent: 0% to 20% ethyl acetate in cyclohexane) to afford the title compound.
LC/MS (III) rt 5.68, m/z 263 (M+H-CH₃)⁺.

### Step 2

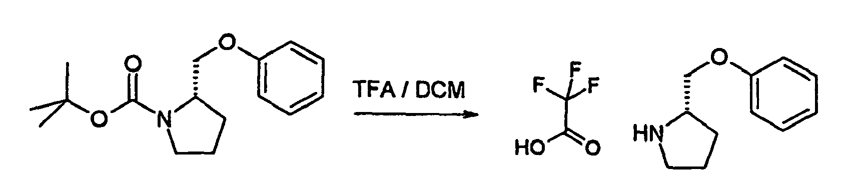

### 2-Phenoxymethyl-pyrrolidine (TFA salt)

Obtained from the product of step 1 according to the procedure described for steps 2 in Example 1.
¹H-NMR δ (ppm) = 1.65-1.80 (m, 1H), 1.86-2.03 (m, 2H), 2.07-2.18 (m, 1H), 3.05-3.15 (m, 2H), 3.90 (bs, 1H), 4.07 (dd, 1H), 4.23 (dd, 1H), 6.93-6.97 (m, 3H), 7.26-7.32 (m, 2H), 8.72 (bs, 1 NH), 9.27 (bs, 1 NH).
LC/MS (III) rt 2.77, m/z 178 (M+H)⁺.

The compounds in Table 2 are synthesized according to the procedure shown for example 13.

**TABLE 2**

| Example | Structure | LC-MS | NMR |
|---|---|---|---|
| 14 | 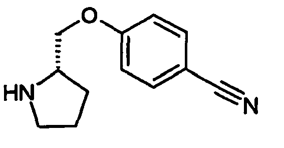 | LC/MS (II) rt 1.88, no mass detected. | ¹H-NMR δ (ppm) = 1.68-1.82 (m, 1 H), 1.86-2.03 (m, 2H), 2.08-2.21 (m, 1H), 3.16-3.30 (m, 2H), 3.87-4.01 (m, 2H), 4.17 (dd, 1 H), 4.34 (dd, 1H), 7.11 (d, 2H), 7.76 (d, 2H), 8.70-8.89 (br s, 1 NH), 9.23-9.43 (br s, 1 NH). |
| 15 | 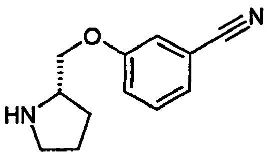 | LC/MS (II) rt 1.75, m/z 203 (M+H)⁺. | ¹H-NMR δ (ppm) = 1.66-1.81 (m, 1 H), 1.82-2.04 (m, 2H), 2.06-2.21 (m, 1H), 3.16-3.30 (m, 2H), 3.85-4.02 (m, 2H), 4.14 (dd, 1 H), 4.32 (dd, 1 H), 7.05-7.56 (m, 4H), 8.71-8.87 (brs, 1NH), 9.20-9.39 (br s, 1NH). |
| 16 | 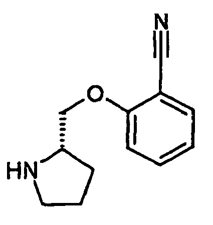 | LC/MS (II) rt 1.70, m/z 203 (M+H)⁺. | ¹H-NMR δ (ppm) = 1.77-1.97 (m, 2H), 1.99-2.23 (m, 2H), 3.19-3.32 (m, 2H), 3.94-4.09 (m, 2H), 4.29 (dd, 1 H), 4.38 (dd, 1 H), 7.12 (t, 1 H), 7.23 (d, 1 H), 7.60-7.77 (m, 2H), 8.75-8.95 (br s, 1 NH), 9.17-9.33 (br s, 1NH). |
| 17 | 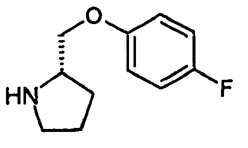 | LC/MS (II) rt 1.85, m/z 196 (M+H)⁺. | ¹H-NMR δ (ppm) = 1.66-1.81 (m, 1H), 1.84-2.03 (m, 2H), 2.06-2.20 (m, 1 H), 3.22-3.28 (m, 2H), 3.77-3.97 (m, 2H), 4.05 (dd, 1 H), 4.20 (dd, 1H), 6.93-7.00 (m, 2H), 7.07-7.16 (m, 2H), 8.64-8.80 (br s, 1NH), 9.17-9.32 (br s, 1NH). |
| 18 | 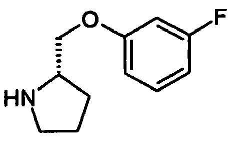 | LC/MS (II) rt 1.79, m/z 196 (M+H)⁺. | ¹H-NMR δ (ppm) = 1.63-1.81 (m, 1 H), 1.84-2.03 (m, 2H), 2.06-2.18 (m, 1H), 3.22-3.31 (m, 2H), 3.74-3.98 (m, 2H), 4.08 (dd, 1 H), 4.25 (dd, 1 H), 6.74-6.87 (m, 3H), 7.32 (q, 1 H), 8.70-8.82 (br s, 1 NH), 9.16-9.35 (br s, 1NH). |
| 19 | 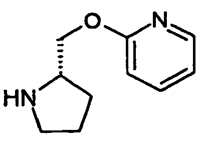 | LC/MS (II) rt 1.45, no mass detected. | ¹H-NMR δ (ppm) = 1.68-1.80 (m, 1 H), 1.86-2.03 (m, 2H), 2.06-2.20 (m, 1 H), 3.15-3.26 (m, 2H), 3.84-3.99 (m, 2H), 4.33 (dd, 1 H), 4.50 (dd, 1 H), 6.83 (d, 1 H), 7.00 (dd, 1 H), 7.70- 7.77 (m, 1 H), 8.14 (dd, 1H), 8.60-8.77 (br s, 1 NH), 9.07-9.27 (br s, 1NH). |

### Example 20

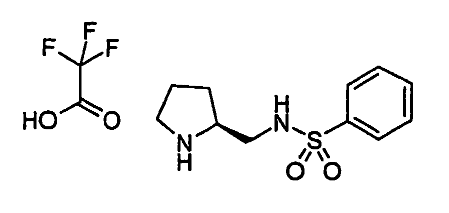

### Step 1

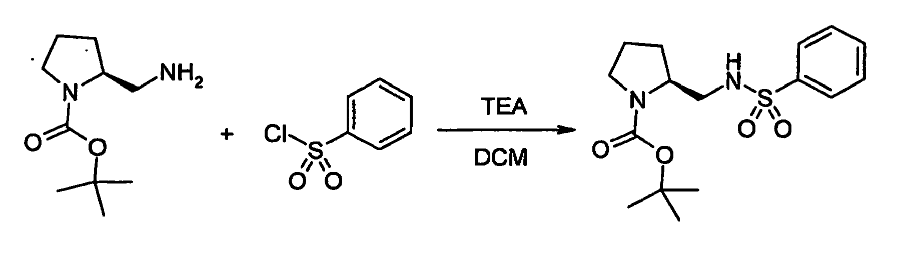

### (2S)-(Benzenesulfonylamino-methyl)-pyrrolidine-1-carboxylic acid tert-butyl ester.

To a solution of 150 mg (0.75 mmol) of (2*S*)-aminomethyl-pyrrolidine-1-carboxylic acid *tert-*butyl ester and 117 µL (90.0 mg, 0.90 mmol) of triethylamine in 3 mL of dichloromethane is added 62 µL (85.7 mg, 0.80 mmol) of benzenesulfonylchloride at 0 °C. The mixture is stirred for 1.5 h at room temperature and then evaporated under reduced pressure to give a crude mixture containing approx. 70% of the title compound, which is taken directly to the next step.
LC/MS (I) rt 4.34, m/z 241 (M-+H-Boc)⁺.

### Step 2

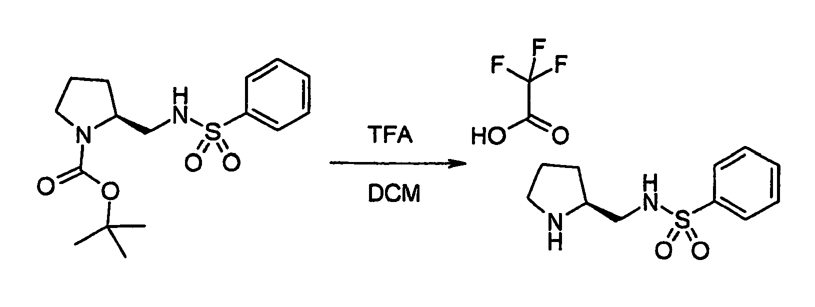

### N-Pyrrolidin-(2S)-ylmethyl-benzensulfonamide (TFA salt).

A solution of 231 mg (approx. 70% purity, 0.47 mmol) of (2*S*)-(benzenesulfonylaminomethyl)-pyrrolidine-1-carboxylic acid *tert*-butyl ester (Step 1) in 1.5 mL of dichloromethane and 0.5 mL of trifluoroacetic acid is stirred at room temperature for 2 h and then evaporated under reduced pressure. The oily product is diluted in 5 mL of dichloromethane and filtered through aluminium oxide (eluent 0% to 10% methanol in dichloromethane). The collected fractions are concentrated to give the title compound.
¹H-NMR δ (ppm) =1.53-1.65 (m, 1H), 1.81-2.05 (m, 3H), 2.91-3.16 (m, 5H), 3.50-3.55 (m, 1H), 7.27-7.29 (m, 1H), 7.58-7.60 (m, 2H), 7.78-7.80 (m, 2H), 7.99 (t, *J*=7.6 Hz, 1H), 9.15 (bs, 1H).
LC/MS (I) rt 2.11, m/z 241 (M+H)⁺.

The compounds in Table 3 are synthesized according to the procedure shown for Example 20.

**TABLE 3**

| Example | Structure | LC-MS | NMR |
|---|---|---|---|
| 21 | 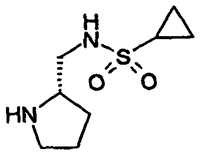 | LC/MS (II) rt 1.60, m/z 206 (M+H)⁺. | |
| 22 | 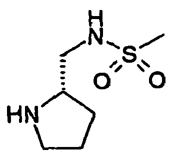 | LC/MS (II) rt.0.40, m/z 196 (M+H)⁺. | |
| 23 | 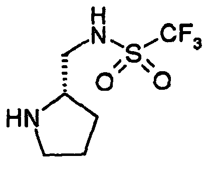 | Boc-protected compound (step 1) : LC/MS (II) rt 4.30, m/z 318 (M+H-CH₃)⁺. | Boc-protected compound (step 1) : ¹H-NMR δ (ppm) = 1.39 (s, 9H), 1.73-1.92 (m, 4H), 3.02-3.18 (m, 1 H), 3.20-3.25 (m, 2H), 3.39-3.50 (m, 1H), 3.70-3.80 (m, 1H), 9.50 (bs, 1 H). |
| | | | |
| 24 | 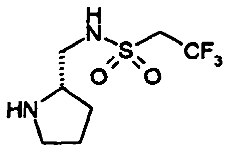 | Boc-protected compound (step 1) : LC/MS (II) rt 3.90, m/z 332 (M+H-CH₃)⁺. | Boc-protected compound: 1H-NMR δ(ppm)= 1.40 (s, 9H), 1.73-1.86 (m, 4H), 2.83-2.96 (m, 1H), 3.18-3.21 (m, 3H), 3.67-3.78 (m, 1H), 4.32-4.38 (m, 2H), 7.88 (bs, 1 H). |

### Example 26

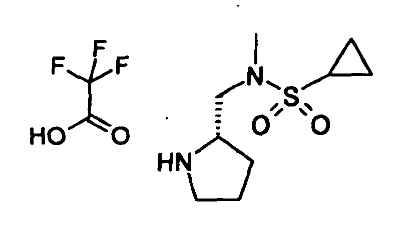

### Step 1

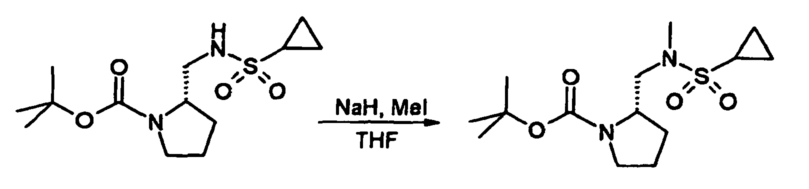

### 2-[(Cyclopropanesulfonyl-methyl-amino)-methyl]-pyrrolidine-1-carboxylic acid tert-butyl ester

To a solution of 45 mg (0.15 mmol) of 2-(cyclopropanesulfonylamino-methyl)-pyrrolidine-1-carboxylic acid tert-butyl ester (step 1, example 18) in 1 mL of tetrahydrofuran, 7.1 mg (0.30 mmol) of sodium hydride in 0.5 mL THF is added and the reaction is stirred 5 minutes. 14 µl (0.22 mmol) of methyliodide are added slowly and reaction is stirred overnight. The solvent is evaporated under reduced pressure, the crude material is dissolved in ethyl acetate and washed sequentially with 5% aqueous citric acid solution and saturated aqueous sodium bicarbonate solution, and brine, dried over sodium sulphate and the solvent is removed under vacuum. The crude material is used without further purification In the next step.
LC/MS (V) (5-90%, 5 min): rt 2.85, m/z 382 (M++Na+AcCN)⁺.

### Step 2

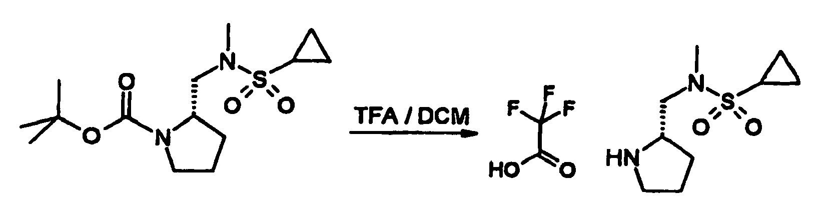

### Cyclopropanesulfonic acid methyl-pyrrolidin-2-ylmethyl-amide (TFA salt).

Obtained from the product of step 1 according to the procedure described for steps 2 in Example 1.
LC/MS (V) (5-90%. 5 min): rt 0.22, m/z 241 (M++Na)⁺.

### Example 27

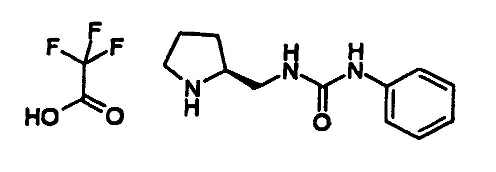

### Step 1

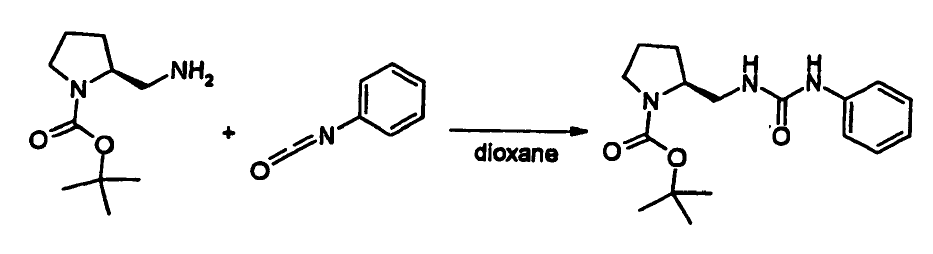

### (2S) [(3-Phenyl-ureido)-methyl]-pyrrolidine-1-carboxylic acid tert-butyl ester.

A solution of 150 mg (0.75 mmol) of (2*S*)-aminomethyl-pyrrolidine-1-carboxylic acid *tert*-butyl ester and 86 µL (93.7 mg, 0.79 mmol) of phenyl isocyanate in 3 mL of dioxan is stirred at 90 °C for 5 h. After evaporation of the solvent under reduced pressure, the crude mixture (approx. 50% content of title product) is used in the next step without further purification.
LC/MS (III) rt 4.18, m/z 320 (M+H)⁺.

### Step 2

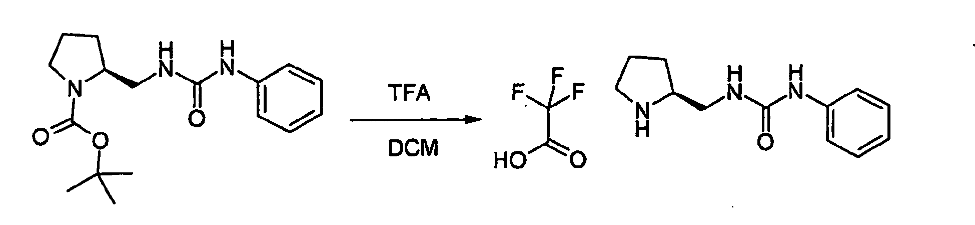

### 1-Phenyl-3-pyrrolidin-(2S)-ylmethyl-urea (TFA salt).

A solution of 253 mg (approx. 0.37 mmol) of (2S)-[(3-phenyl-ureido)-methyl]-pyrrolidine-1-carboxylic acid *tert*-butyl ester (step 1) in 0.5 mL of trifluoroacetic acid and 1.0 mL of dichloromethane is stirred at room temperature for 2 h and then evaporated , under reduced pressure. The crude mixture is dissolved in 2 mL of a 1M ammonia solution in methanol, concentrated under reduced pressure and then purified using flash chromatography (aluminium oxide, eluent: 0% to 10% methanol in dichloromethane containing 0.1% of ammonia) to give the title compound.
¹H-NMR δ (ppm) = 1.35-1.40 (m, 1H), 1.78-1.81 (m, 5H), 2.85-2.84 (m, 2H), 3.02-3.22 (2H), 4.35 (bs, 2H), 6.38 (bs, 1H), 6.83 (t, J=10.0 Hz, 1H), 7.16 (t, J=10.0 Hz, 2H), 7.35 (d, J=10.0 Hz, 2H), 8.65 (bs, 0.1H), 8.77 (bs, 0.9H).
LC/MS (I) rt 1.88, m/z 220 (M+H)⁺.

### Example 28

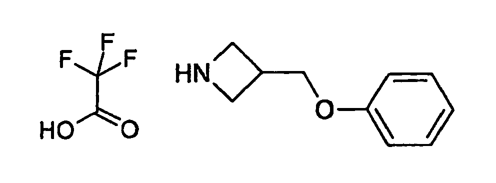

### Step 1

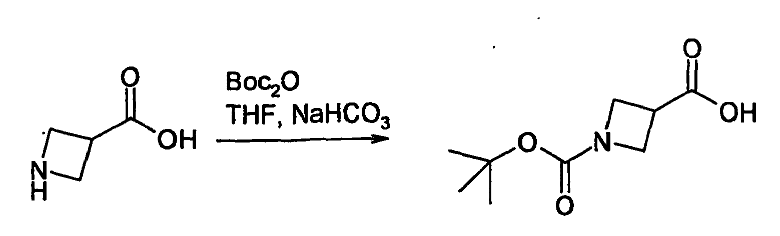

### Boc-azetidine-3-carboxylic acid

To a solution of 100 mg (0.99 mmol) 3-azetidine carboxylic acid in 15 mL THF is added 5 mL of saturated sodium bicarbonate solution and 238 mg (1.09 mmol) di-*tert-*butyl dicarbonate. The mixture is stirred overnight at room temperature, acidified with 5% aqueous hydrochloric acid and extracted three times with ethyl acetate. The combined organic layers are washed with brine and dried over sodium sulphate. Removal of the solvent in vacuum yields the product that was used without further purification for the next step.
LC/MS (II) rt 2.08, m/z 187 (M+H-CH₃)⁺.

### Step 2

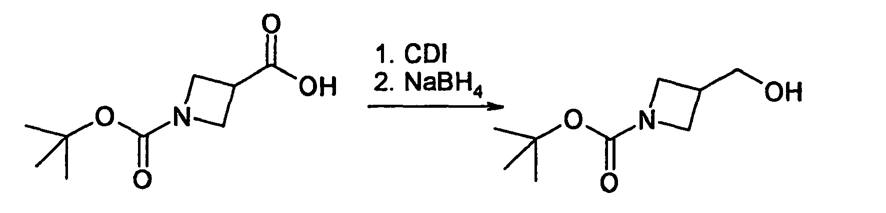

### 3-Hydroxymethyl-azetidine-1-carboxylic acid tert-butyl ester

A mixture of 212 mg (0.99 mmol) of the crude material from step 1 and 241 mg (1.49 mmol) CDI in 15 mL dry tetrahydrofurane is stirred for 2 h at room temperature, then cooled to 0°C and a suspension of 56 mg (1.49 mmol) sodium borohydride in water added quickly. After another 1 h at 0°C acetone is added, the mixture allowed to warm to room temperature and the solvent removed. The remaining material is dissolved in ethyl acetate and water, the layers separated and the organic layer washed with 5 % citric acid, saturated sodium bicarbonate solution and brine. Drying over sodium sulphate and removal of the solvent affords the alcohol.
LC/MS (II) rt 1.81, m/z 173 (M+H-CH₃)⁺.

### Step 3

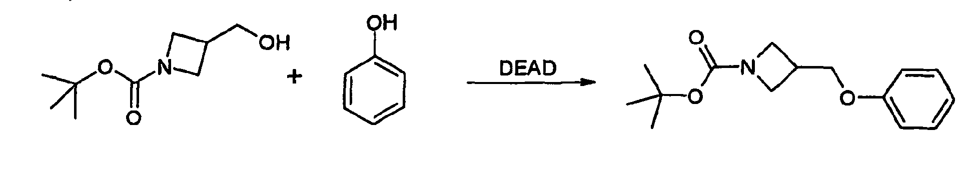

### 3-Phenoxymethyl-azetidine-1-carboxylic acid tert-butyl ester

To a solution of 94 mg (0.5 mmol) Boc protected hydroxymethyl azetidine (step 2) in 5 mL of THF was added 354 mg (0.5 mmol) fluorous triphenyl phosphine and 47 mg (0.5 mmol) phenol. The mixture was cooled down to 0 °C and 405 mg (0.5 mmol) fluorous diethyl azodicarboxylate (DEAD) was added and allowed for warm up to room temperature. The reaction was stirred for 3 days, evaporated to dryness over 1 g of alumina. Alumina containing the reaction product was placed over fluorous silica cartridge and washed with methanol:water 4:1 eluent (4 x 1 mL). The filtrate was concentrated under reduced pressure and subjected to preparative TLC (silica, hexanes: ethyl acetate 1:1) to afford the title product.
LC/MS (11) rt 1.89, m/z 164 (M+H-Boc)⁺.

### Step 4

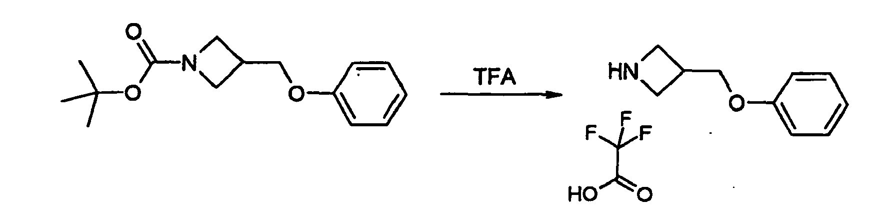

### 3-Phenoxymethyl-azetidine CTFA salt)

A solution of 34.0 mg (0.13 mmol) of 3-phenoxymethyl-azetidine-1-carboxylic acid tert-butyl ester (step 3) in 300 µL of trifluoroacetic acid and 300 µL of dichloromethane is stirred at room temperature for 30 minutes and then evaporated under reduced pressure to give the title compound.

### Example 29

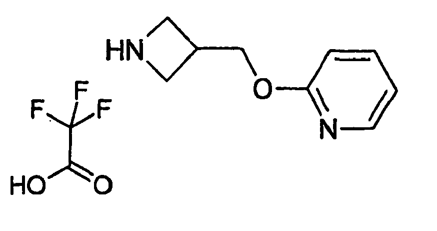

### 2-(Azetidin-3-ylmethoxy)-pyridine (TFA salt).

Obtained from 3-hydroxymethyl-azetidine-1-carboxylic acid tert-butyl ester and pyridin-2-ol according to the procedure described for steps 3 and 4 in Example 28.
LC/MS (II) rt 0.25, m/z 165 (M+H)⁺.

### Example 30

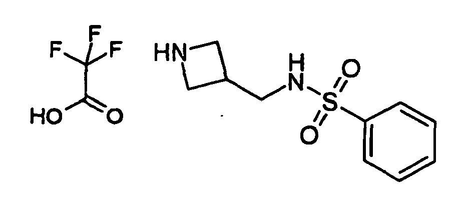

### Step1

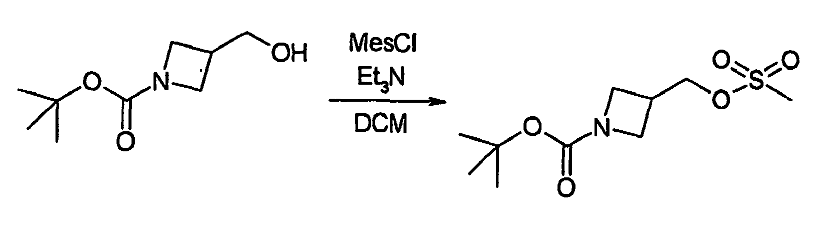

### 3-Methanesulfonyloxymethyl-azetidine-1-carboxylic acid tert-butyl ester

To 170 mg (0.90 mmol) of 3-hydroxymethyl-azetidine-1-carboxylic acid tert-butyl ester in 10 mL dry dichloromethane 155 µl (1.08 mmol) triethylamine and 75 µl (0.99 mmol) methanesulfonic acid chloride are added at 0°C. After 4 h at 0°C dichloromethane (50 mL) is added and the organic layer washed twice with brine. The organic layer is dried over sodium sulphate and the solvent removed, yielding crude material that is directly taken to the next step.
LC/MS (II) rt 2.35, m/z 251 (M+H-CH₃)⁺.

### Step 2

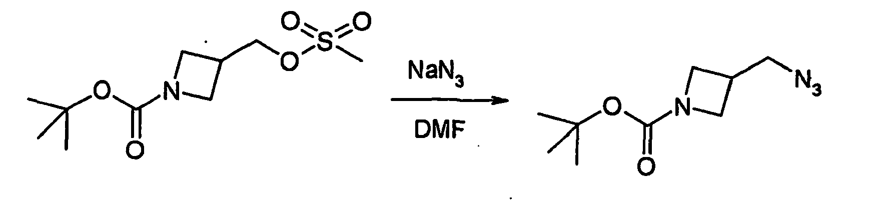

### 3-Azidomethyl-azetidine-1-carboxylic acid tert-butyl ester

A mixture of 3-methanesulfonyloxymethyl-azetidine-1-carboxylic acid tert-butyl ester (266 mg, 0.90 mmol, step 1) and 176 mg (2.70 mmol) sodium azide in 10 mL dry *N,N-*dimethylformamide is heated to 90 °C for 1 h. For workup 60 mL of ethyl acetate are added and the organic layer is washed thoroughly with brine (3x), dried over sodium sulphate and concentrated under reduced pressure. Purification by flash chromatography on silica gel (cyclohexane to 20% ethylacetate in cyclohexane) yields the azide.
LC/MS (II) rt 2.57, m/z 198 (M+H-CH₃)⁺.

### Step 3

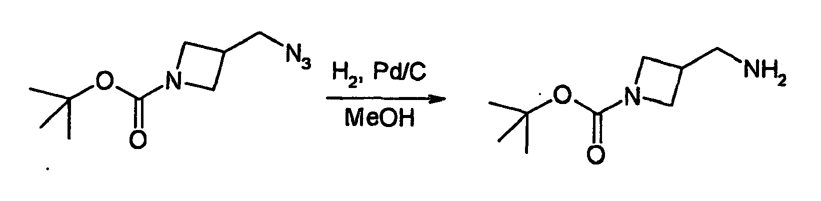

### 3-Aminomethyl-azetidine-1-carboxylic acid tert-butyl ester

73 mg (0.35 mmol) of 3-azidomethyl-azetidine-1-carboxylic acid tert-butyl ester (step 2) dissolved in 20 mL methanol, 1 mL ammonia (2M in MeOH) and Pd/C (5% with 50% water) added and the mixture stirred at 1 atm H₂ for 1 h. Filtration over Celite and evaporation of the solvent affords the crude amine that is taken directly to the next step.
LC/MS (IV) rt 1.75, m/z 172 (M+H- CH₃)⁺.

### Step 4

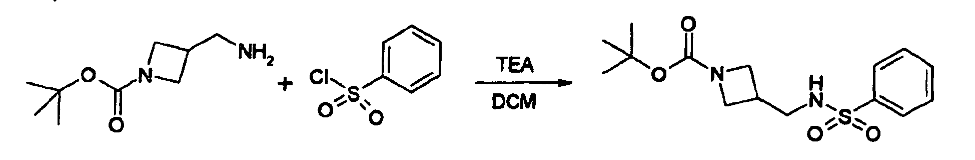

### 3-(Benzenesufonylamine-methyl)-azetidine-1-carboxylic acid tert-butyl ester

39 mg (0.21 mmol) of 3-aminomethyl-azetidine-1-carboxylic acid tert-butyl ester (step 3) and 32µl (0.25 mmol) triethylamine are dissolved in dichloromethane and 17 µl (0.23 mmol) of benzenesulfonylchloride added at 0°C**.** The reaction mixture is subsequently stirred for 1h and diluted with dichloromethane. The organic layer is washed with 5% citric acid, saturated sodium bicarbonate solution and brine and dried over sodium sulphate. The crude product is purified by flash chromatography on silica gel (cyclohexane to 20% ethyl acetate in cyclohexane).
LC/MS (IV) rt 2.64, m/z 312 (M+H-CH₃)⁺.

### Step 5

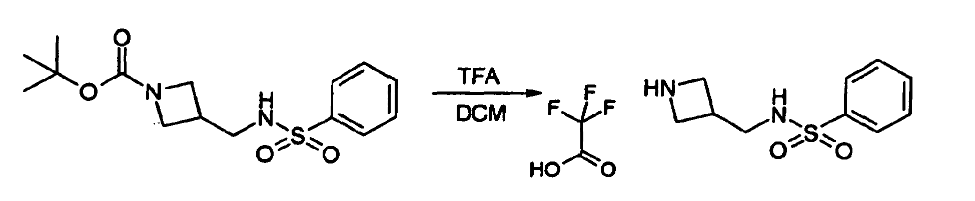

### N-Azetidin-3-ylmethyl-benzenesulfonamide (TFA salt).

Obtained from the product of step 4 according to the procedure described for step 2 in example 1.
LC/MS (IV) rt 1.73, m/z 227 (M+H)⁺.

### Example 32

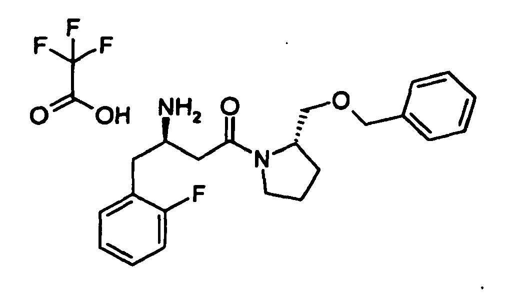

### Step 1

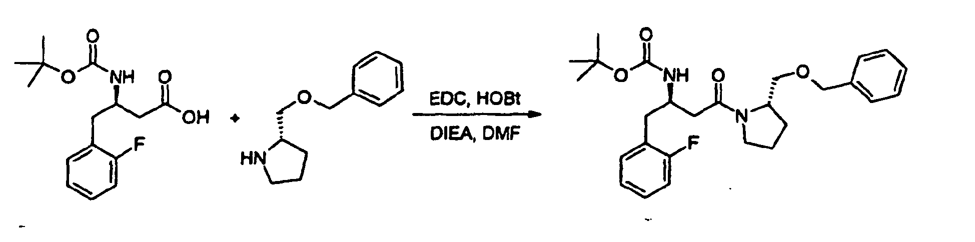

### {(3R)-[(2S)-Benzyloxymethyl-pyrrolidin-1-yl]-1-(2-fluoro-benzyl)-3-oxo-proryl}-carbamic acid tert-butyl ester.

A mixture of 44.8 mg (0.15 mmol) of (*3R*)-*tert*-butoxycarbonylamino-4-[2-fluoro-phenyl]-butyric acid, 28.3 mg (0.21 mmol) of 1-hydroxybenzotriazole (HOBt), 39.9 mg (0.21 mmol) of 1-ethyl-3-(3-dimethylaminopropyl)carbodiimide hydrochloride (EDC) and 100 µL (98.2 mg, 0.76 mmol) of diisopropylethylamine in 2.5 mL of *N*,*N*-dimethylformamide is stirred for 5 minutes. After addition of 50.0 mg (0.17 mmol) of (*2S*)-benzyloxymethylpyrrolidine (Example 10) in 0.5 mL of *N*,*N*-dimethylfonnamide, the mixture is stirred for further 16 h. The solution is diluted with 5 mL of 1N hydrochloric acid solution and extracted twice with 10 mL of dichloromethane. The collected organic phases are washed with brine and water, dried over sodium sulphate and evaporated under reduced pressure. The residue is purified using flash chromatography (silica gel, eluent: 0% to 10% methanol in dichloromethane) to afford the title compound.
LC/MS (I) rt 5.68, m/z 471 (M+H)⁺.

### Step 2

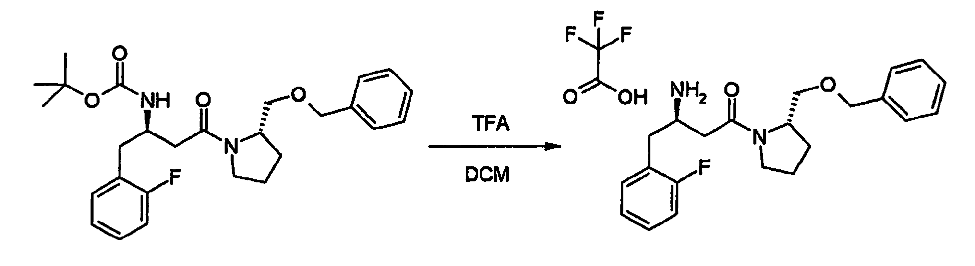

### (3R)-Amino-1-[(2S)-benzyloxymethyl-pyrrolidin-1-yl]-4-(2-fluoro-phenyl)-butan-1-one (TFA salt).

A solution of 8.00 mg (0.017 mmol) of {*(3R)*-[*(2S)*-benzyloxymethyl-pyrrolidin-1-yl]-1-(2-fluoro-benzyl)-3-oxo-propyl}]-carbamic acid *tert-*butyl ester (Step 1) in 0.5 mL of trifluoroacetic acid and 1 mL of dichloromethane is stirred at room temperature for 1 h and then evaporated under reduced pressure. The crude mixture is purified using HPLC (eluent: 5% to 95% acetonitrile in water with 0.1 % of trifluoroacetic acid) to afford the title compound.
¹H-NMR δ (ppm) = 1.79-1.87 (m, 3H), 2.85-2.92 (m, 1H), 2.98-3.05 (m, 1H), 3.21-3.32 (m, 5H), 3.43-3.47 (m, 1H), 3.69 (bs), 3.93-3.95 (m, 0.3H), 4.05-4.10 (m, 0.7H), 4.41-4.45 (m, 3H), 7.11-7.18 (m, 2H), 7.21-7.32 (m, 7H), 7.94 (bs, 2H).
LC/MS (I) rt 3.60, m/z 371 (M+H)⁺.

The compounds in Table 4 are synthesized according to the procedure shown for example 32.

**TABLE 4**

| Example | Structure | LC-MS | NMR |
|---|---|---|---|
| 33 | 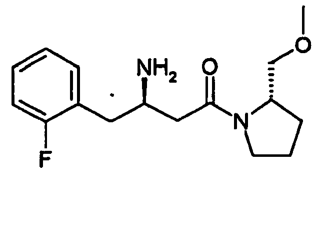 | LC/MS (I) rt 2.78, m/z 295 (M+H)⁺. | ¹H-NMR δ (ppm) = 1.68-1.94 (m, 4H), 2.49-2.58 (m, 1 H), 2.62-3.06 (m, 3H), 3.13-3.45 (m, 7H), 3.65-3.79 (m, 1 H), 3.93-4.08 (m.1H), 7.10-7.19 (m, 2H), 7.26-7.36 (m, 2H), 7.90-8.02 (br s, 3H). |
| 34 | 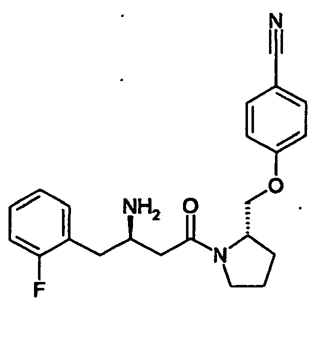 | LC/MS (II) rt 2.20, m/z 382 (M+H)⁺. | ¹H-NMR δ (ppm) = 1.81-2.06 (m, 4H), 2.52-2.66 (m, 1H), 2.71-3.09 (m, 3H), 3.30-3.43 (m, 2H), 3.67-3.83 (m, 1 H), 3.85-4.10 (m, 2H), 4.13-4.27 (m, 1 H), 7.02-7.12 (m, 4H), 7.23-7.33 (m, 2H), 7.67-7.78 (m, 2H), 7.90-8.06 (br s, 3H). |
| 35 | 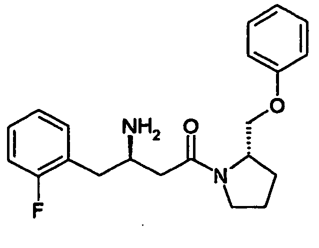 | LC/MS (III) rt 3.74, m/z 357 (M+H)⁺. | ¹H-NMR δ (ppm) = 1.76-2.06 (m, 4H), 2.50-2.61 (m, 1 H), 2.69-3.07 (m, 3H), 3.20-3.40 (m, 2H), 3.65-4.11 (m, 3H), 4.15-4.27 (m, 1H), 6.85-6.98 (m, 3H), 7.10-7.42 (m, 6H), 7.98 (br s, 3H). |
| 36 | 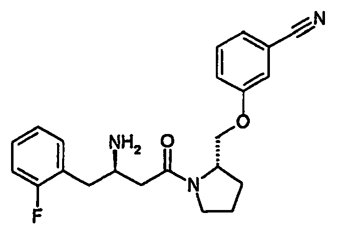 | LC/MS (IV) rt 2.38, m/z 382 (M+H)⁺. | ¹H-NMR δ (ppm) = 1.83-2.07 (m, 4H), 2.48-2.58 (m, 1 H), 2.70-3.08 (m, 3H), 3.24-3.45 (m, 2H), 3.70-3.80 (m, 1 H), 3.85-4.08 (m, 2H), 4.15-4.29 (m, 1H), 7.08-7.20 (m, 2H), 7.22-7.50 (m, 6H), 7.89- 8.05 (br s, 3H). |
| 37 | 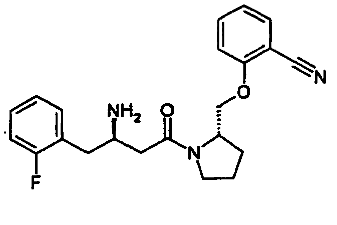 | LC/MS (IV) rt 2.31, m/z 382 (M+H)⁺. | ¹H-NMR δ (ppm) = 1.88-2.07 (m, 4H), 2.48-2.59 (m, 1H), 2.68-3.08 (m, 3H), 3.28-3.48 (m, 2H), 3.67-3.79 (m, 1H), 3.98-4.30 (m, 3H), 6.99-7.38 (m, 6H), 7.53-7.72 (m, 2H), 7.87-8.00 (br s, 3H). |
| 38 | 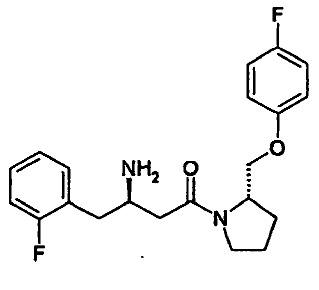 | LC/MS (IV) rt 2.45, m/z 375 (M+H)⁺. | ¹H-NMR δ (ppm) = 1.76-2.06 (m, 4H), 2.49-2.57 (m, 1H), 2.68-3.08 (m, 3H), 3.22-3.42 (m, 2H), 3.70-4.00 (m, 3H), 4.11-4.27 (m, 1 H), 6.85-6.94 (m, 2H), 6.99-7.20 (m, 4H), 7.24-7.33 (m,2H), 7.92-8.04 (br s, 3H). |
| 39 | 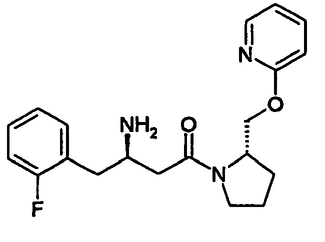 | LC/MS (IV) rt 1.98, m/z 358 (M+H)⁺. | ¹H-NMR δ (ppm) = 1.85-1.97 (m, 4H), 2.74-2.99 (m, 4H), 3.25-3.40 (m, 2H), 3.66-3.81 (m, 1 H), 4.11-4.34 (m, 3H), 6.72-6.82 (dd, 1H), 6.88-7.00 (m, 1 H), 7.08-7.19 (m, 2H), 7.24-7.36 (m. 2H), 7.60-7.72 (m. 1H). 7.89-8.01 (br s, 3H), 8.04-8.13 (m, 1 H). |
| 40 | 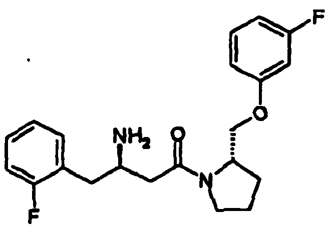 | LC/MS (IV) rt 2.49, m/z 375 (M+H)⁺. | ¹H-NMR δ (ppm) = 1.78-2.07 (m, 4H), 2.49-2.58 (m, 1H), 2.69-3.08 (m, 3H), 3.21-3.43 (m, 2H), 3.89-4.05 (m, 3H), 4.12-4.27 (m, 1H), 6.64-8.80 (m, 3H), 7.08-7.36 (m. 5H). 7.92-8.04 (br s, 3H). |
| 41 | 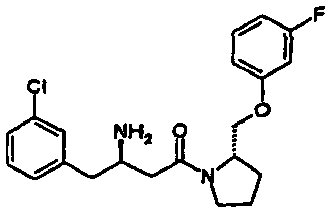 | LC/MS (II) rt 2.67, m/z 391 (M+H)⁺. | ¹H-NMR δ (ppm) = 1.79-2.09 (m, 4H), 2.51-2.54 (m, 1H), 2.60-3.01 (m. 3H), 3.21-3.47 (m, 2H). 3.69-3.91 (m, 2H), 4.00-4.05 (m, 1 H), 4.17-4.28 (m, 1 H), 6.64-6.84 (m. 3H), 7.13-7.41 (m, 5H). 7.90 (bs, 3H. |
| 42 | 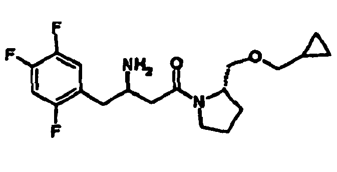 | LC/MS (VI) (10-80%, 10 min): rt 4.51, m/z 393 (M++Na)⁺. | ¹H-NMR δ (ppm) = 0.12-0.16 (m, 2H), 0.41-0.47 (m, 2H); 0.95 (m, 1H), 1.81 (m, 4H), 2.33 (m. 2H). 2.72 (m, 2H3.17-3.43 (m, 7H), 4.01 (m, 1H). 7.46-7.53 (m, 2H). 8.20 (s, 1H). |

Using a procedure similar to those outlined for example 32, the following compounds were prepared.

### Example 43

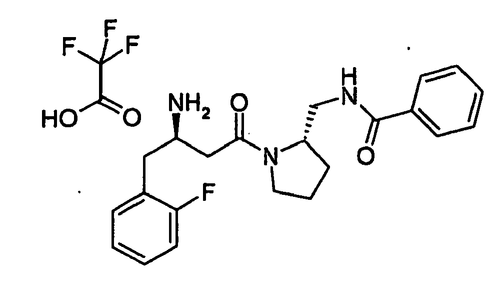

### Step 1

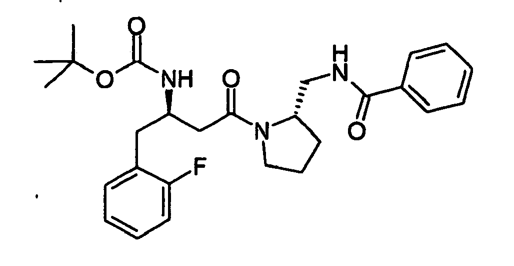

### {(3R)-[(2S)-(Benzoylamino-methyl)-pyrrolidin-1-yl]-1-(2-fluoro-benzyl)-3-oxo-propyl}carbamic acid tert-butyl ester.

Obtained from (*3R*)-tert-butoxycarbonylamino-4-(2-fluoro-phenyl)-butyric acid and *N-*pyrrolidin-(*2S*)-ylmethyl-benzamide (Example 1) according to the procedure described for step 1 in example 32.
LC/MS (II) rt 2.99, m/z 506 (M+Na)⁺.

### Step 2

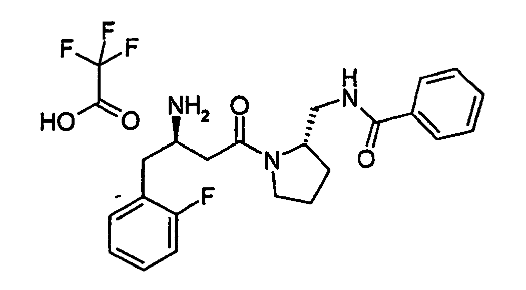

### {(3R)-[(2S)-(Benzoylamino-methyl)-pyrrolidin-1-yl]-1-(2-fluoro-benzyl)-3-oxo-propyl}-carbamic acid tert-butyl ester (TFA salt).

Obtained from the product of step 1 according to the procedure described for step 2 in example 32.
¹H-NMR δ (ppm) = 1.75-1.95 (m, 5H), 2.78-3.20 (m, 3H), 3.32-3.37 (m, 2H), 3.69-3.80 (m, 0.5H), 3.90-3.97 (m, 0.2H), 4.18-4.20 (m, 0.4H), 7.12-7.19 (m, 2H), 7.28-7.33 (m, 5H), 7.37-7.52 (m, 2H), 7.73-7.76 (m, 3H), 7.92 (bs, 2H), 8.38-8.66 (m, 0.7H), 8.62-8.66 (m, 0.3H).
LC/MS (II) rt 2.02, m/z 384 (M+H)⁺.

The compounds in Table 5 are synthesized according to the procedure shown for Example 43.

**TABLE 5**

| Example | Structure | LC-MS | NMR |
|---|---|---|---|
| 44 | 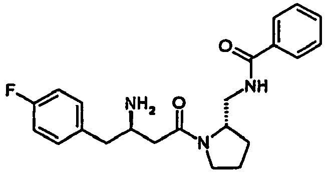 | LC/MS (IV) rt 2.17, m/z 384 (M+H)⁺. | ¹H-NMR δ (ppm) = 1.74-2.01 (m, 4H), 1 H overlaps with the DMSO signal, 2.73-3.19 (m, 3H), (m, 3H), 3.21-3.43 (m, 4H), 3.61-3.77 (m, 1 H), 3.87-3.99 (m, 0.3 H), 4.17-4.27 (m, 0.5 H), 7.06-7.16 (m, 2H), 7.21-7.31 (m, 2H), 7.35-7.55 (m, 2H), 7.71 7.95 (m, 4H), 8.37-8.4, (m, 0.5H), 8.62-8.72 (m 0.3H). |
| 45 | 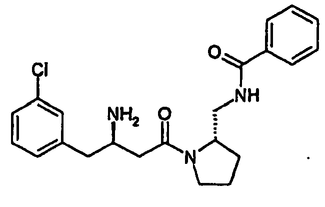 | LC/MS (IV) rt 2.26, m/z 400 (M+H)⁺. | ¹H-NMR δ (ppm) = 1.76 2.01 (m, 4H), 1 H overlaps with the DMSO signal 2.77-3.18 (m, 3H), 3.24 3.44 (m, 4H), 3.66-3.82 (m, 1H), 3.87-4.01 (m, 0.4 H), 4.13-4.27 (m, 0.6 H), 7.15-7.54 (m, 5H), 7.71. 7.96 (m, 6H), 8.38-8.46 (m, 0.5H), 8.62-8.70 (m, 0.3H). |
| 46 | 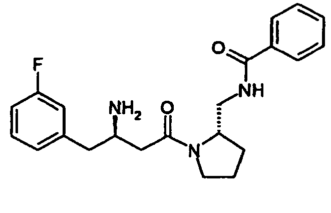 | LC/MS (IV) rt 2.21, m/z 384 (M+H)⁺. | ¹H-NMR δ (ppm) = 1.68-2.00 (m, 4H), 1 H overlaps with the DMSO signal, 2.73-3.09 (m, 3H), 3.22-3.45 (m, 5H), 3.94-4.02 (m, 0.4 H), 4.18-4.27 (m, 0.6 H), 7.08-7.20 (m, 2H), 7.23-7.43 (m, 2H), 7.51-7.64 (m, 1 H), 7.85-8.05 (m, 5H), 8.54-8.65 (m, 1 H), 8.74-8.85 (m, 0.5 H), 8.95-9.05 (m, 0.3 H). |
| 47 | 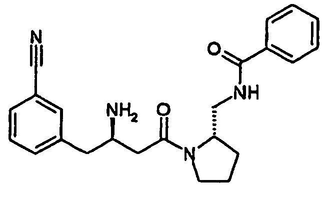 | LC/MS (II) rt 1. 96, m/z 391 (M+H)⁺. | ¹H-NMR δ (ppm) = 1.72-1.98 (m, 4H), 2.51-2.57 (m, 1 H), 2.78-3.15 (m, 3H), 3.22-3.44 (m, 4H), 3.69-3.84 (m, 1H), 3.89-4.02 (m, 0.4 H), 4.14-4.25 (m, 0.6 H), 7.34-7.64 (m, 6H), 7.65-7.77 (m, 3H), 7.81-8.01 (m, 3H), 8.38-8.46 (m, 0.6 H), 8.62-8.72 (m, 0.3 H). |
| 48 | 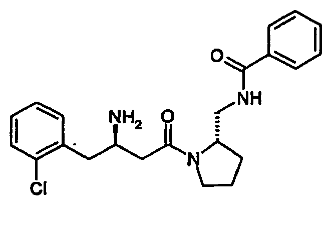 | LC/MS (II) rt 2.06, m/z 400 (M+H)⁺. | ¹H-NMR δ (ppm) = 1.72-1.97 (m, 4H), 2.52-2.58 (m, 1 H), 2.70-2.91 (m, 1 H), 2.97-3.19 (m, 3H), 3.23-3.41 (m, 3H), 3.74-3.88 (m, 1 H), 3.88-3.95 (m, 0.4 H), 4.15-4.22 (m, 0.6 H), 7.25-7.31 (m, 2H), 7.34-7.54 (m, 5H), 8.01 (bs, 3H), 8.38-8.44 (m, 0.5H), 8.63-8.71 (m, 0.3H). |
| 49 | 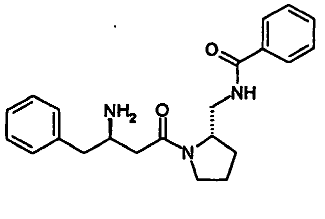 | LC/MS (IV) rt 2.10, m/z 366 (M+H)⁺. | ¹H-NMR δ (ppm) = 1.74-1.98 (m, 4H), 1 H overlaps with the DMSO signal, 2.76-2.88 (m, 2H), 2.93-3.18 (m, 1 H), 3.21-3.42 (m, 4H), 3.67-3.76 (m, 1H), 3.85-3.95 (m, 0.4 H), 4.14-4.26 (m, 0.6 H), 7.17-7.36 (m, 5H), 7.37-7.53 (m, 3H), 7.73-7.93 (m, 5H), 8.37-8.43 (m, 0.7 H), 8.60-8.68 (m, 0.3 H). |
| 50 | 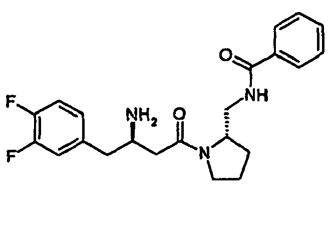 | LC/MS (IV) rt 2.19, m/z 402 (M+H)⁺. | ¹H-NMR δ (ppm) = 1.75-2.01 (m, 4H), 1H overlaps with the DMSO signal, 2.72-3.16 (m, 3H), 3.26-3.44 (m, 4H), 3.66-3.80 (m, 1H), 3.92-3.99 (m, 0.4 H), 4.16-4.22 (m, 0.6 H), 7.03-7.13 (m, 1H), 7.28-7.54 (m, 5H), 7.71-7.93 (m, 5H), 8.37-8.47 (m, 0.7 H), 8.62-8.70 (m, 0.3 H). |

### Example 51

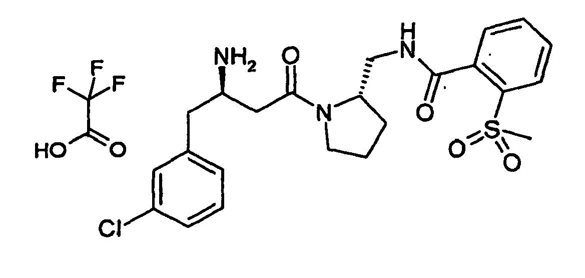

### Step 1

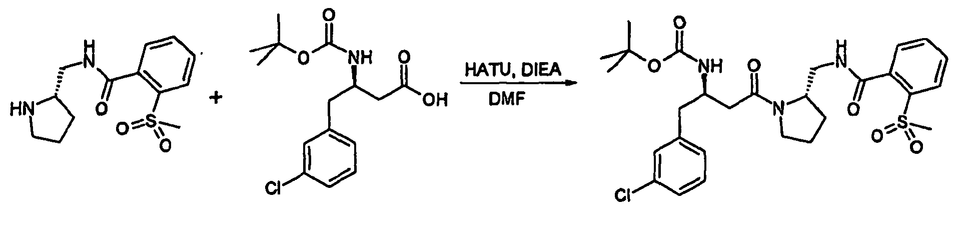

### (1-(3-Chloro-benzyl)-3-{2-[(2-methanesulfonyl-benzoylamino)-methyl]-pyrrolidin-1-yl}-3-oxo-propyl)-carbamic acid tert-butyl ester

A mixture of 23 mg (0.08 mmol) of (*3R*)-*tert*-butoxycarbonylamino-4-[3-chloro-phenyl]-butyric acid, 34.2 mg (0.09 mmol) O-(7-Azabenzotriazol-1-yl)-*N*,*N*,*N',N'-*tetramethyluronium hexafluorophosphate (HATU), and 39.3 µL (0.22 mmol) of diisopropylethylamine in 2.5 mL of *N*,*N*-dimethylformamide is stirred for 10 minutes at room temperature. 25.4 mg (0.09 mmol) of 2-methanesulfonyl-*N*-pyrrolidin-2-ylmethylbenzamide (example 2) and 19.6 µL (0.11 mmol) of diisopropylethylamine in 1 mL of *N*,*N*-dimethylformamide is added to the solution and the mixture is stirred overnight. The solvent is evaporated under reduced pressure. The crude material is dissolved in ethyl acetate and washed sequentially with 5% citric acid aqueous solution and saturated aqueous sodium bicarbonate solution, dried over sodium sulphate and the solvent is removed under reduced pressure. The crude product is used in the next step without further purification.

### Step 2

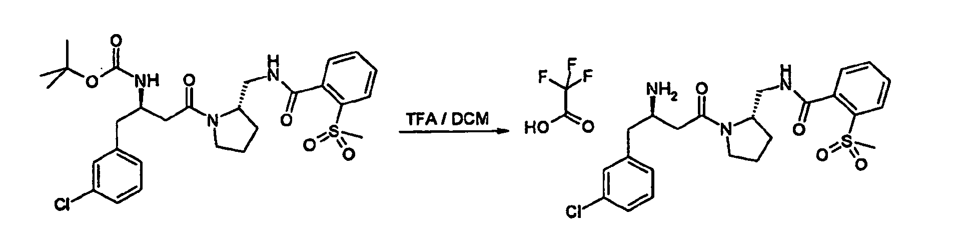

### N-{1-[3-Amino-4-(3-chloro-phenyl)-butyryl]-pyrrolidin-2-ylmethyl}-2-methanesulfonylbenzamide

Obtained from the product of step 1 according to the procedure described for steps 2 in Example 32.
LC/MS (8 min 10-70%) rt 3.13, m/z 478 (M+H)⁺.

The compounds in Table 6 are synthesized according to the procedure shown for Example 51.

**TABLE 6**

| Example | Structure | LC-MS | NMR |
|---|---|---|---|
| 52 | 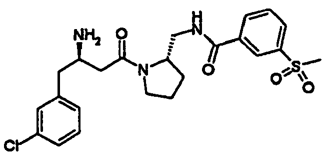 | LC/MS (VI) (10-70%, 8 min): rt 3.22, m/z 478 (M+H)⁺. | ¹H-NMR δ (ppm) = 1.77-1.97 (m, 4H), 2.51-2.57 (m, 1H), 2.79-3.04 (m, 2H), 3.22 (s, 3H), 3.28-3.43 (m, 3H), 3.68-3.82 (m, 1H), 3.90-3.98 (m, 0.3 H), 4.19-4.26 (m, 0.7 H), 7.16-7.38 (m, 4H), 7.67-7.78 (m, 1H), 7.84-7.93 (m, 3H), 8.01-8.20 (m, 2H), 8.29 (m, 0.7H), 8.35 (m, 0.3H), 8.69-8.75 (m, 0.7H), 8.93-9.01 (m, 0.3H). |
| 53 | 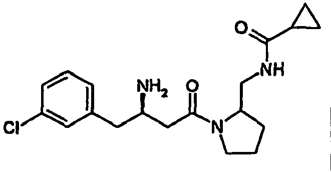 | LC/MS (V) (15-50%, 10 min): rt 3.01, m/z 364 (M+H)⁺. | ¹H-NMR δ (ppm) = 0.57-0.76 (m, 4H), 1.45-1.58 (m, 1H), 1.73-1.92 (m, 4H), 2.70-2.72 (m, 1H), 2.79-2.99 (m, 3H), 3.11-3.39 (m, 4H), 3.66-3.83 (m, 1H), 3.94-4.20 (m, 1H), 7.16-7.21 (m, 1H), 7.27-7.39 (m, 3H), 7.79-7.90 (bs, 3H), 7.95-8.00 (m, 0.7H), 8.19-8.28 (m, 0.3H). |

### Example 54

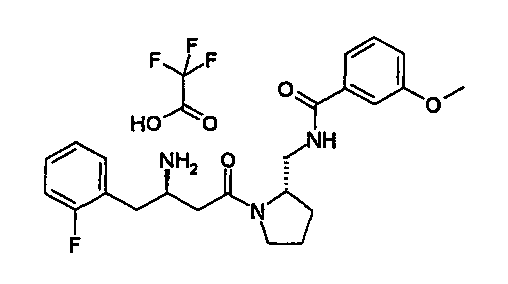

### Step 1

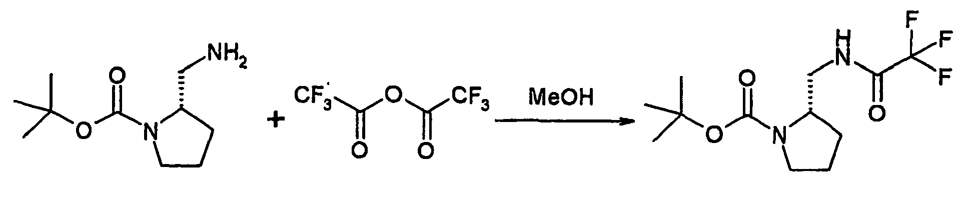

### 2-[(2,2,2-Trifluoro-acetylamino)-methyl]-pyrrolidine-1-carboxylic acid tert-butyl ester

2-Aminomethyl-pyrrolidine-1-carboxylic acid tert-butyl ester (200 mg, 1.00 mmol) is dissolved in 1 mL methanol. Triethylamine (113 µl, 1.10 mmol) and trifluoroacetic acid anhydride (210 mg, 0.99 mmol) are added sequentially and the reaction is stirred at room temperature overnight. The solvent is evaporated under reduced pressure and the crude material is purified by flash chromatography (silica gel, eluent: 0% to 30% ethyl acetate in cyclohexane) to afford the title compound.
LC/MS (IV) rt 2.81, m/z 282 (M+H-CH₃)⁺.

### Step 2

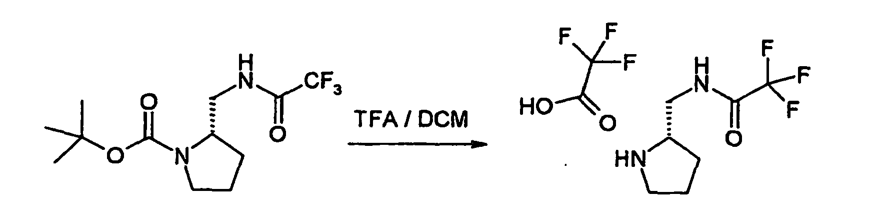

### 2,2,2-Trifluoro-N-pyrrolidin-2-yimethyl-acetamide (TFA salt).

Obtained from the product of step 1 according to the procedure described for step 2 in example 1.
¹H-NMR δ (ppm) = 1.62-1.68 (m, 1H), 1.82-2.10 (m, 3H), 3.13-3.35 (m, 2H), 3.43-3.65 (m, 3H), 8.50 (bs, 1NH), 9.11 (bs, 1NH), 9.60 (bs, 1 H).
LC/MS (IV) rt 1.15, m/z 197 (M+H)⁺.

### Step 3

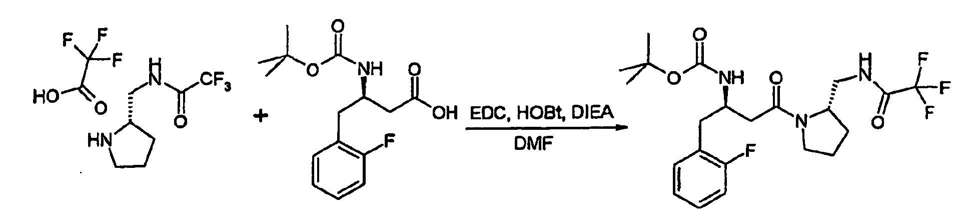

### (1-(2-Fluoro-benzyl)-3-oxo-3-{2-[(2,2,2-trifluoro-acetylamino)-methyl]-pyrrolidin-1-yl}-propyl)-carbamic acid tert-butyl ester

Obtained from the product of step 3 and 3-tert butoxycarbonylamino-4-(2-fluorophenyl)-butyric acid according to the procedure described for step 1 in example 32.
LC/MS (IV) rt 3.05, m/z 498 (M+Na)⁺.

### Step 4

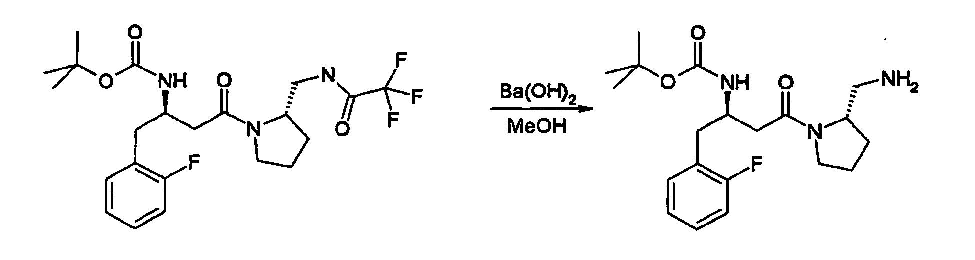

### [3-(2-Aminomethyl-pyrrolidin-1-yl)-1-(2-fluoro-benzyl)-3-oxo-propyl]-carbamic acid tert-butyl ester

(1-(2-Fluoro-benzyl)-3-oxo-3-{2-[(2,2.2-trifluoro-acetylamino)-methyl]-pyrrolidin-1-yl}-propyl)-carbamic acid tert-butyl ester (Step 3, 155 mg, 0.33 mmol) is dissolved in 1 mL methanol and 2 mL of a 0.4 N barium hydroxide solution are added. The reaction is stirred overnight at room temperature. The solvent is evaporated under reduced pressure, water is added and the crude material is extracted with dichloromethane. The solvent is evaporated and the crude material is redissolved in a mixture methanol/dichloromethane, dried over sodium sulphate and the solvent is evaporated under reduced pressure. The crude material is used in the next step without further purification.
¹H-NMR δ (ppm) = 1.28 (m, 9H), 1.70-1.95 (m, 4H), 2.32-2.50 (m, 2H), 2.60-2.90 (m, 3H), 3.10.3.50 (m, 4H), 4.00-4.15 (m, 1H). 6.63 (bs, 1H), 7.03-7.08 (m, 2H), 7.18-7.22 (m, 2H).
LC/MS (IV) rt 2.27, m/z 380 (M+H)⁺.

### Step 5

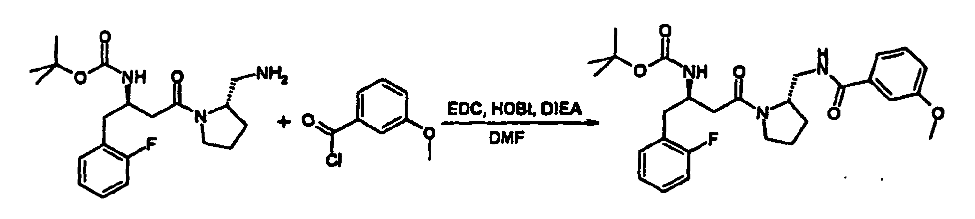

### (1-(2-Fluoro-benzyl)-3-{2-[(3-methoxy-benzoylamino)-methyl]-pyrrolidin-1-yl]-3-oxopropyl)-carbamic acid tert-butyl ester

Obtained from the product of step 4 and 3-methoxy-benzoyl chloride according to the procedure described for steps 1 In Example 32.
LC/MS (II) rt 2.97, m/z 536 (M++Na)⁺.

### Step 6

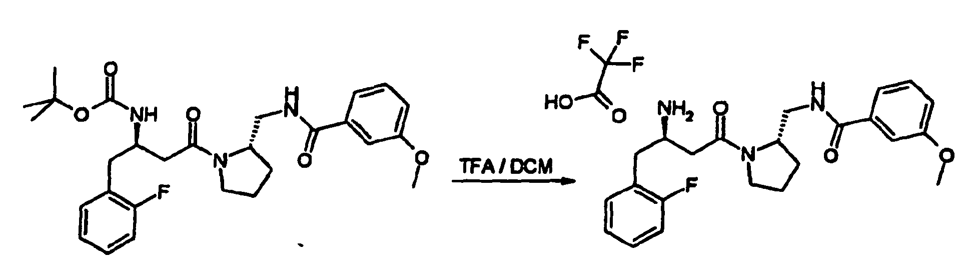

### N-{1-[3-Amino-4-(2-fluoro-phenyl)-butyryl]-pyrrolidin-2-ylmethyl}-3-methoxybenzamide (TFA salt).

Obtained from the product of step 5 according to the procedure described for steps 2 In Example 1.
LC/MS (II) rt 2.09, m/z 414 (M+H)⁺.

The compounds in Table 7 are synthesized according to the procedure shown for Example 54.

**TABLE 7**

| Example | Structure | LC-MS | NMR |
|---|---|---|---|
| 55 | 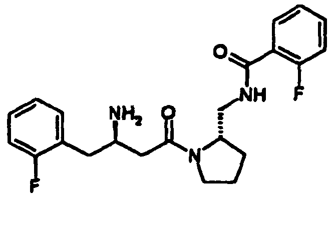 | LC/MS (II) rt 2.07, m/z 402 (M+H)⁺. | ¹H-NMR δ (ppm) = 1.75-1.90 (m, 4H), 2.76-3.41 (m, 8H), 2H overlap with the water signal, 7.11-7.32 (m, 6H), 7.43-7.60 (m, 2H), 7.95 (bs, 3H), 8.24 (bs, 0.7 H), 8.47 (bs, 0.3H). |
| 56 | 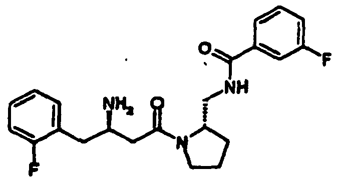 | LC/MS (II) rt 2.12, m/z 402 (M+H)⁺. | |
| 57 | 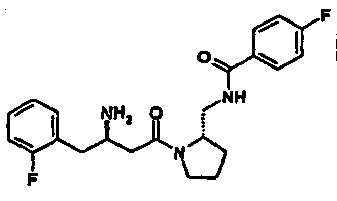 | Boc-protected compound (Step 5): LC/MS (IV) rt 3.05, m/z 524 (M+Na)⁺. | |
| 58 | 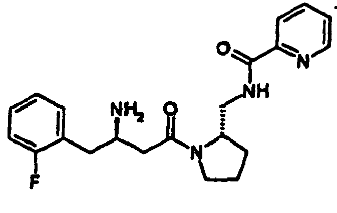 | Boc-protected compound (Step 5): LC/MS (IV) rt 2.82, m/z 485(M+H)⁺. | ¹H-NMR δ (ppm) = 1.68-2.03 (m, 4H), 2.69-3.16 (m, 4H), 3.24-3.41 (m, 4H), 3.85-3.84 (m, 1H), 3.87-3.99 (m, 0.3 H), 4.11-4.28 (m, 0.7 H), 7.08-7.21 (m, 2H), 7.25-7.52 (m, 3H), 7.69-7.80 (m, 2H), 7.84-8.03 (m, 3H), 8.85-8.80 (m, 2.5 H), 8.95-9.00 (m, 0.3H). |
| 59 | 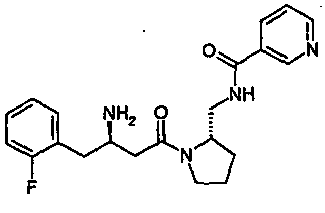 | LC/MS (IV) rt 1.62, m/z 385(M+H)⁺. | ¹H-NMR δ (ppm) = 1.71-2.03 (m, 4H), 1H overlaps with the DMSO signal, 2.67-3.08 (m, 3H), 3.20-3.40 (m, 4H), 2H overlap with the water signal 7.09-7.21 (m, 2H), 7.26-7.39 (m, 2H), 7.41-7.54 (m, 1H), 7.86-8.02 (m, 3H), 8.06-8.20 (m, 1 H), 8.56-8.64 (m, 0.5H), 8.64-8.72 (m, 1H), 8.80-8.87 (m, 0.3H), 8.89-9.01 (m, 1 H). |
| 60 | 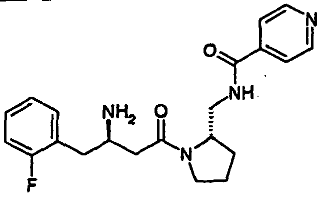 | LC/MS (IV) rt 1.79, m/z 385 (M+H)⁺. | |
| 61 | 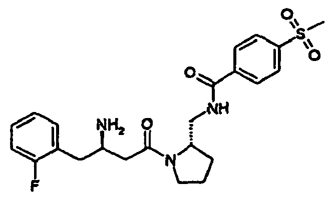 | LC/MS (IV) rt 2.03, m/z 462 (M+H)⁺. | |
| 62 | 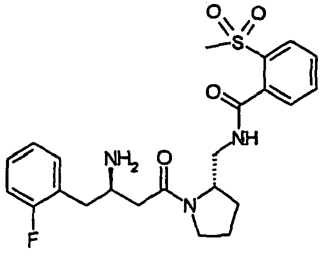 | LCIMS (IV) rt 2.05, m/z 462 (M+H)⁺. | ¹H-NMR δ (ppm) = 1.72-2.02 (m, 4H), 1 H overlaps with the DMSO signal, 2.68-3.12 (m, 3H), 3.22-3.45 (m, 7H), 3.65-4.26 (m, 2H), 7.09-7.21 (m, 2H), 7.23-7.34 (m, 2H), 7.44-7.58 (m, 1H), 7.61-7.77 (m, 2H), 7.89-8.01 (m, 3H), 8.46-8.53 (m, 0.5 H), 8.71-8.80 (m, 0.2H). |

### Example 63

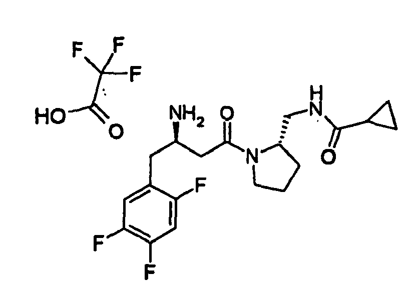

### Step 1

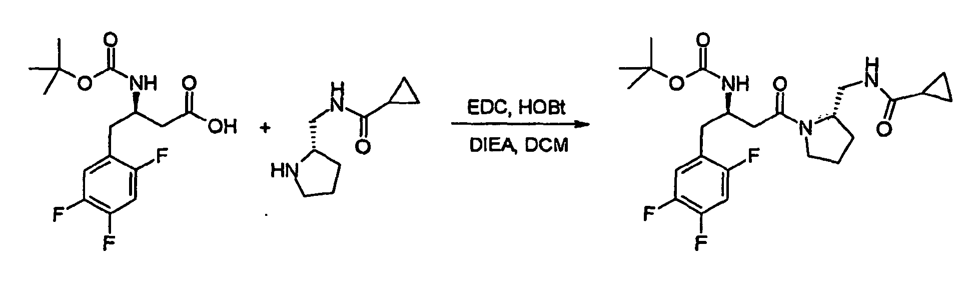

### [3-{2-[(Cyclopropanecarbonyl-amino)-methyl]-pyrrolidin-1-yl}-3-oxo-1-(2,4,5-trifluorobenzyl)-propyl-carbamic acid tert-butyl ester

A mixture of 70.0 mg (0.21 mmol) of (*3R*)-*tert*-butoxycarbonylamino-4-[2-fluoro-phenyl]-butyric acid, 31.3 mg (0.23 mmol) of 1-hydroxybenzotriazole, 45.0 mg (0.23 mmol) of 1-ethyl-3-(3-dimethylaminopropyl)carbodiimide hydrochloride and 56 µL (0.31 mmol) of diisopropylethylamine in 1 mL of dichloromethane is stirred for 30 minutes at 0 °C. After addition of 87.0 mg (0.26 mmol) of cyclopropanecarboxylic acid (pyrrolidin-2-ylmethyl)-amide (Example 4) in 1 mL of dichloromethane and another 56 µL (0.31 mmol) of diisopropylethylamine, the mixture is stirred overnight at room temperature. The solution is diluted with dichloromethane, washed sequentially with 5% citric acid aqueous solution, saturated aqueous sodium bicarbonate solution, and brine, dried over sodium sulphate and the solvent is removed under vacuum. Purification of the crude product by flash chromatography (silica gel, eluent: 5% to 10 % of ethyl acetate in cyclohexane) gives the title compound.

### Step 2

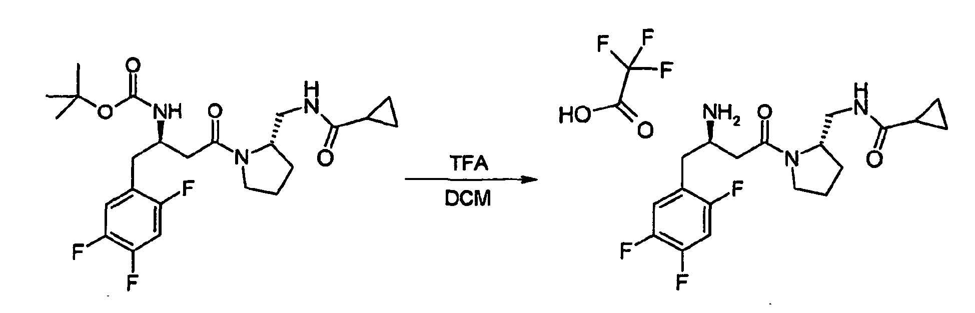

### (3R)-Amino-1-[(2S)-benzyloxymethyl-pyrrolidin-1-yl]-4-(2-fluoro-phenyl)-butan-1-one (TFA salt)

A solution of the product from step 1 in 30% trifluoroacetic acid in dichloromethane is stirred at 0 °C for 1 h and then 1 mL methanol is added. The solvent is evaporated under reduced pressure. The crude mixture is dissolved in dichloromethane and the solvent is removed under reduced pressure. This procedure is repeated 3-4 times. The crude material is purified using HPLC (eluent: 5% to 95% acetonitrile in water with 0.1 % of trifluoroacetic acid) to afford the title compound.
¹H-NMR δ (ppm) = 0.60-0.66 (m, 4H), 1.45-1.54 (m, 1 H), 1.70-1.90 (m, 4H), 2.75 (m, 1H), 2.81-3.00 (m, 2H), 3.12-3.21 (m, 2H), 3.45-3.49 (m, 3H), 3.68-3.81 (m 1.5H), 3.98 (m, 0.7H), 7.43-7.62 (m, 2H), 8.10 (bs, 0.6H), 8.14 (s, 0.7H), 8.22 (s, 0.2H), 8.38 (bs, 0.4H)
LC/MS (10 min, 1-30%) rt 6.81, m/z 384 (M+H)⁺.

The compounds in Table 8 are synthesized according to the procedure shown for example 63.

**TABLE 8**

| Example | Structure | LC-MS | NMR |
|---|---|---|---|
| 64 | 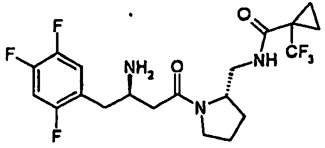 | LC/MS(VI) (10-60%, 10 min): rt 4.32, m/z 452 (M+H)⁺. | ¹H-NMR δ (ppm)= 1.15-1.30 (m, 4H), 1.64-1.88 (m, 4H), 2.42 (m, 1H), 2.61-2.68 (m, 1H), 2.82 (m, 2H), 2.90-3.00 (m, 1H), 3.12-3.38 (m, 3H), 3.52-3.58 (m, 1.3H), 3.86 (m 0.5H), 4.10 (m, 0.8H), 7.43-7.55 (m, 2H), 7.95 (bs, 0.6H), 8.10 (bs, 0.4H), 8.22 (s, 1H). |
| 65 | 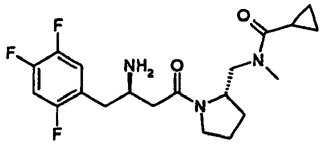 | LC/MS(VI) (10-60%, 10 min): rt 4.32, m/z 452 (M+H)⁺. | ¹H-NMR δ (ppm) = 0.61-0.76 (m, 4H), 1.66-2.10 (m, 5H), 2.54 (m, 2H), 2.75-2.85 (m, 4H), 3.13 (m, 3H), 3.52 (m, 3H), 4.20 (m, 1H), 7.44-7.55 (m, 2H), 8.17 (s, 0.7H). |

### Example 66

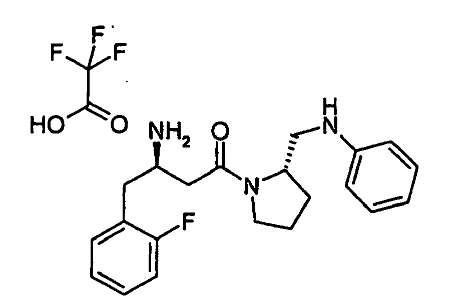

### Step 1

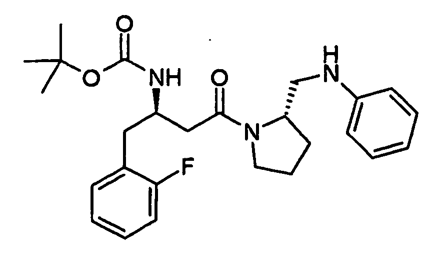

### {(3R)-[(2S)-(Benzoylamino-methyl)-pyrrolidin-1-yl]-1-(2-fluoro-benzyl)-3-oxo-propyl}-carbamic acid tert-butyl ester.

Obtained from (*3R*)-*tert*-butoxycarbonylamino-4-(2-fluoro-phenyl)-butyric acid and phenyl-pyrrolidin-(*2S*)-ylmethyl-amine according to the procedure described for step 1 in example 32.
LC/MS (III) rt 4.16, m/z 455 (M+H)⁺

### Step 2

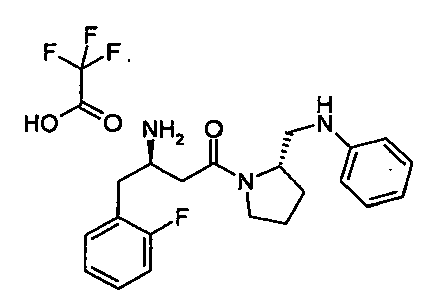

### (3R)-Amino-4-(2-fluoro-phenyl)-1-[(2S)-phenylaminomethyl-pyrrolidin-1-yl]-butan-1-one (TFA salt).

Obtained from the product of step 1according to the procedure described for step 2 in example 32.
¹H-NMR δ (ppm) = 1.76-1.90 (m, 6H), 2.77-3.35 (m, 10H), 3.70-3.79 and 4.10-4.15 (2m, 1H), 4.90 (bs), 6.42-6.47 (2m, 1H), 6.54-6.61 (m, 2H), 6.95-7.03 (m, 1H), 7.13-7.20 (m, 2H), 7.30-7.35 (m, 2H), 7.96 (bs, 2H).
LC/MS (III) rt 2.84, m/z 354 (M+H)⁺ . The compounds in Table 9 are synthesized according to the procedure shown for Example 66.

**TABLE 9**

| Example | Structure | LC-MS | NMR |
|---|---|---|---|
| 67 | 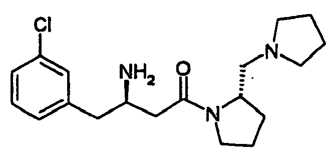 | LC/MS(VI) (5-90%, 5 min): rt 1.73, m/z 350 (M+H)⁺. | ¹H-NMR δ (ppm) = 1.75-2.09 (m, 8H), 2.52-2.67 (m, 2H), 2.62-3.16 (m, 6H), 3.29-3.43 (m, 2H), 2.52-3.83 (m, 3H), 4.17-4.30 (m, 1H), 7.17-7.22 (m, 1H), 7.27-7.41 (m, 3H), 7.95 (bs, 3H), 8.30 (bs, 1H). |

### Example 68

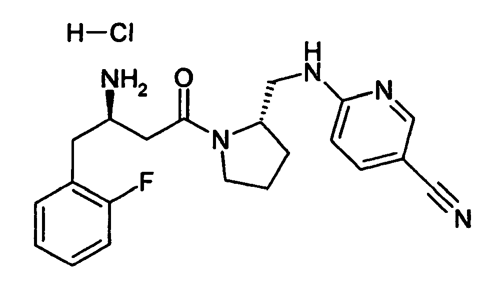

### Step 1

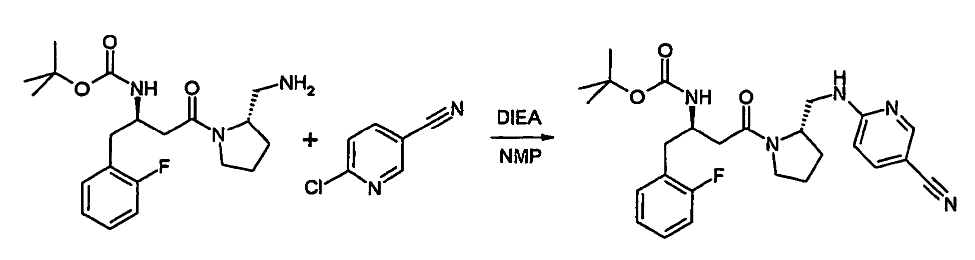

### 3-{2-[(5-Cyano-pyridin-2-ylamino)-methyl]-pyrrolidin-1-yl}-1-(2-fluoro-benzyl)-3-oxopropyl]-carbamic acid tert-butyl ester

20 mg (0.05 mmol) [3-(2-aminomethyl-pymolidin-1-yl)-1-(2-fluoro-benzyl)-3-oxo-propyl]-carbamic acid tert-butyl ester (step 4, example 54) and 25 µl (0.16 mmol) diisopropylamine are dissolved In 1 mL NMP. 21 mg (0.16 mmol) of 6-chloronicotinonitrile are added at room temperature. The reaction mixture is stirred for 3 hours at room temperature and for 3 hours at 80 °C. After cooling to room temperature the solvent is evaporated under reduced pressure and the crude product is purified by flash chromatography on silica gel (2% methanol in dichloromethane).
LC/MS (II) rt 2.85, m/z 482 (M+H)⁺.

### Step 2

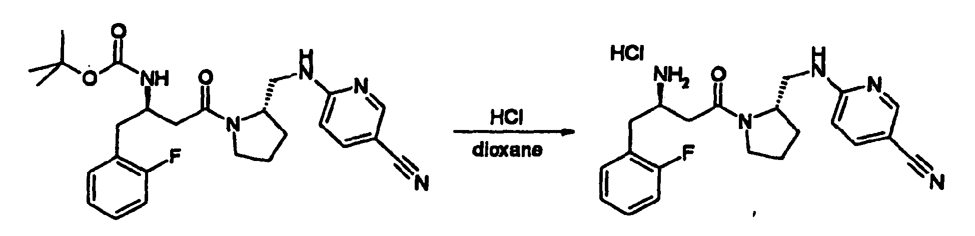

### 6-({1-[3-Amino-4-(2-fluoro-phenyl)butyryl]-pyrrolidin-2-ylmethyl]-amino)nicotinonitrile (HCI salt).

The product of step 1 is dissolved In 1 mL of 4N hydrochloric acid in dioxane. The solution is stirred for 1 hour at room temperature and the solvent is evaporated under reduced pressure. The crude material Is redissolved in methanol and the solvent Is evaporated under reduced pressure to give the title compound.
LC/MS (II) rt 2.09, m/z 382 (M+H)⁺.

The compounds in Table 10 are synthesized according to the procedure shown for Example 68.

**TABLE 10**

| Example | Structure | LC-MS | NMR |
|---|---|---|---|
| 69 | 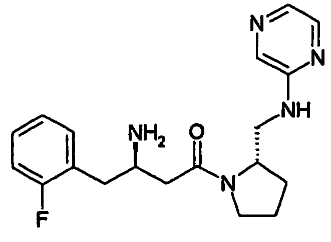 | LC/MS (II) rt 1.87, m/z 358 (M+H)⁺. | ¹H-NMR δ (ppm) = 1.79-2.01 (m, 4H), 2.34-2.43 (m, 2H), 2.80-2.88 (m, 1H), 2.92-3.02 (m, 2H), 3.20-3.38 (m,2H), 3.46-3.53 (m, 1 H), 3.63-3.76 (m, 1H). 3.95-4.05 (m, 1H), 7.12-7.18 (m, 2H), 7.25-7.34. (m, 2H), 7.68-7.75 (m, 1 H), 7.95-8.02 (m, 4H), 8.06-8.10 (m, 1 H), 8.20-8.32 (bs, 2H). |

### Example 70

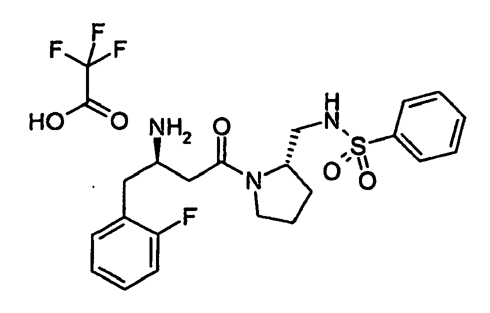

### Step 1

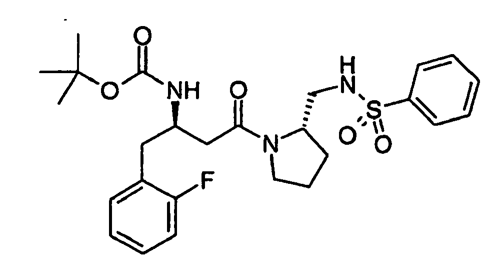

### [(3R)-[(2S)-(Benzenesulfonylamino-methyl)-pyrrolidin-1-yl]-1-(2-fluoro-benzyl)-3-oxopropyl]-carbamic acid tert-butyl ester.

Obtained from (3*R*)-*tert*-butoxycarbonylamino-4-(2-fluoro-phenyl)-butyric acid and *N-*pyrrolidin-(*2S*)-ylmethyl-benzensulfonamide (Example 20) according to the procedure described for step 1 in example 32.
LC/MS (III) rt 4.98, m/z 542 (M+Na)⁺.

### Step 2

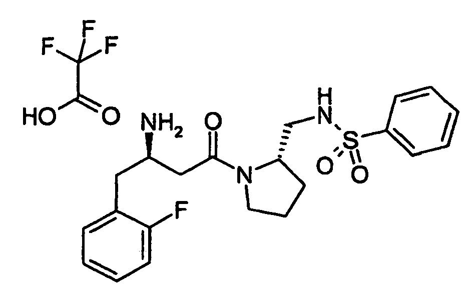

### N-{1-[(3R)-Amino-4-(2-fluoro-phenyl)-butyryl]-pyrrolidin-(2S)-ylmethyl}-benzenesulfonamide (TFA salt).

Obtained from the product of step 1 according to the procedure described for step 2 in example 32.
¹H-NMR δ (ppm) = 1.75-1.85 (m, 4H), 2.48-2.49 (m, 1H), 2.62-2.72 (m, 1H), 2.72-3.04 (m, 3H), 3.22-3.28 (m, 2H), 3.64-3.75 (m, 1H), 3.90-3.94 (m, 0.7H), 4.70 (bs), 7.09-7.15 (m, 2H), 7.21-7.31 (m, 2H), 7.50-7.66 (m, 4H), 7.70-7.77 (m, 2H), 7.89 (bs, 1H).
LC/MS (III) rt 3.16, m/z 442 (M+Na)⁺.

The compounds in Table 11 are synthesized according to the procedure shown for Example 70.

**TABLE 11**

| Example | Structure | LC-MS | NMR |
|---|---|---|---|
| 71 | 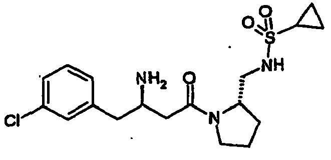 | LC/MS (VI) (10-60%, 8 min): rt 3.10, m/z 414 (M+H)⁺. | |
| 72 | 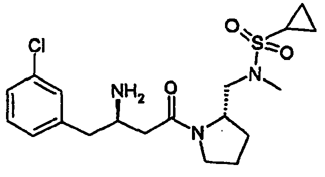 | LC/MS (VI) (10-60%, 8 min): rt 3.82, m/z 400 (M+H) ⁺. | |
| 73 | 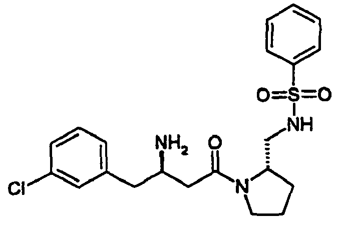 | LC/MS (II) rt 2.29, m/z 436 (M+H)*. | ¹H-NMR δ (ppm) = 1.70-1.91 (m, 4H), 2.64-3.05 (m, 3H), 3.42-3.55 (m, 2H), 3.63-3.71 (m, 3H), 3.86-4.11 (m, 1.5 H), 4.50-4.61 (m, 1H), 7.12-7.36 (m, 4H), 7.47-7.62 (m, 3H), 7.64-7.79 (m, 2H), 7.91-8.30 (bs, 2H). |
| 74 | 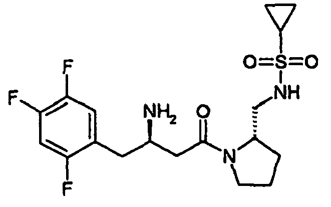 | LC/MS (VI) (1-30%, 10 min): rt 5.27, m/z 420 (M+H) ⁺. | ¹H-NMR δ (ppm)= 0.90-0.96 (m, 4H), 1.76-1.94 (m, 5H), 2.40 (m, 1 H), 2.74-2.80 (m, 2H), 2.82-2.98 (m, 1 H), 3.17-3.36 (m, 5H), 3.87-4.00 (m, 1H), 7.08 (bs, 0.7H), 7.39-7.84 (m, 2H), 8.22 (bs, 1H). |
| 75 | 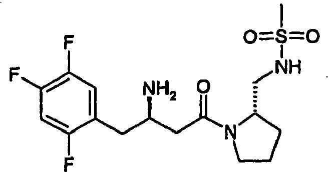 | LC/MS (VI) (1-30%, 10 min): rt 5.77, m/z 394 (M+H)⁺. | ¹H-NMR δ (ppm)= 1.76-1.92 (m, 4H), 2.38 (m, 1 H), 2.74 (m, 2H), 2.91 (m, 3H), 3.10-3.50 (m, 4H), 3.87-3.97 (m, 3H), 7.05 (bs, 0.6H), 7.38-7.47 (m, 2H), 8.24 (bs, 0.7H). |
| 76 | 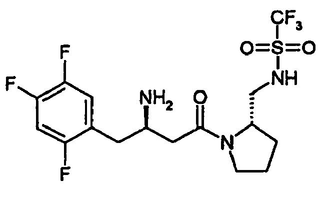 | LC/MS (VI) (1-30%, 10 min): rt 8.10, m/z 489(M+H+AcCN)⁺. | ¹H-NMR δ (ppm)= 1.82-1.87 (m, 4H), 2.46-2.51 (m, 1H), 2.82-3.10 (m, 3H), 3.22-3.50 (m, 4H), 3.64-3.74 (m, 1H), 3.88-3.99 (m, 1H), 7.49-7.59 (m, 2H), 8.16 (s, 0.4H). |
| 77 | 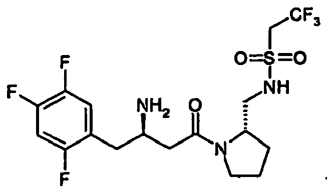 | LC/MS (VI) (1-30%, 10 min): rt 7.03, m/z 484 (M+Na)⁺. | ¹H-NMR δ (ppm)= 1.82-1.88 (m, 4H), 2.42 (m, 1H), 2.82 (m, 1 H), 3.00 (m, 1 H), 3.12-3.58 (m, 6H), 3.86-3.99 (m, 1 H), 4.32-4.45 (m, 2H), 7.42-7.59 (m, 2H), 8.18 (s, 0.4H). |

### Example 78

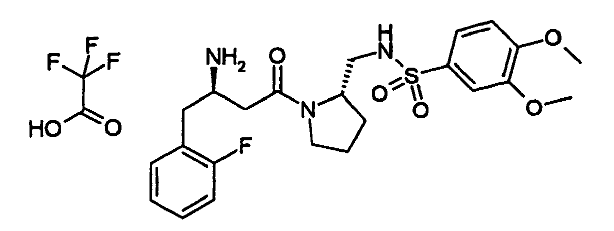

### Step 1

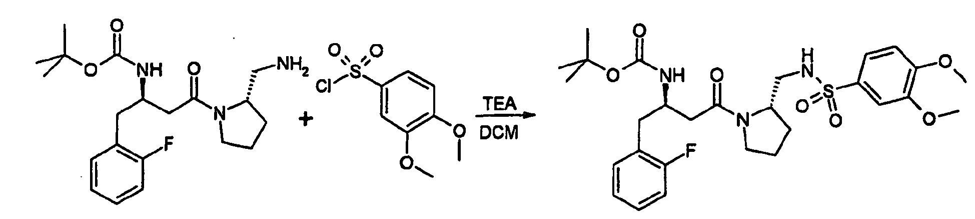

### [3-{2-[(3,4-Dimethoxy-benzenesulfonylamino)-methyl]-pyrrolidin-1-yl}-1-(2-fluorobenzyl)-3-oxo-propyl-carbamic acid tert-butyl ester

15 mg (0.04 mmol) [3-(2-aminomethyl-pyrrolidin-1-yl)-1-(2-fluoro-benzyl)-3-oxo-propyl]-carbamic acid tert-butyl ester (step 4, example 51) and 8 µL (0.06 mmol) triethylamine are dissolved in 1 µL dichloromethane. 11 mg (0.05 mmol) of 3,4-dimethoxybenzenesulfonyl chloride are added at room temperature. The reaction mixture is stirred overnight at room temperature and the solvent is evaporated under reduced pressure and the crude product is used in the next step without further purification.

### Step 2

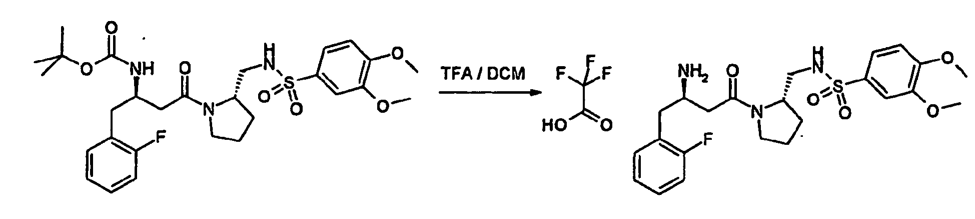

### N-{1-[3-Amino-4-(2-fluoro-phenyl)-butyryl]-pyrrolidin-2-ylmethyl]-3,4-dimethoxybenzenesulfonamide (TFA salt).

Obtained from the product of step 1 according to the procedure described for step 2 in Example 32.
LC/MS (IV) rt 2.21, m/z 480 (M+H)⁺.

The compounds in Table 12 are synthesized according to the procedure shown for example 78.

**TABLE 12**

| Example | Structure | I LC-MS | NMR |
|---|---|---|---|
| 79 | 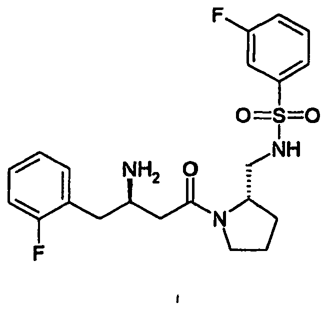 | LC/MS (IV) rt 2.31, m/z 438 (M+H)⁺. | ¹H-NMR δ (ppm) = 1.66-1.90 (m, 4H), 2.58-3.15 (m, 5H), 3.15-3.35 (m, 2H), 3.62-3.76 (m, 2H), 1 H overlaps with the water signal, 7.03-7.13 (m, 2H), 7.17-7.35 (m, 2H), 7.42-7.65 (m, 4H), 7.82-7.89 (m, 0.7H), 8.05-8.22 (bs, 3H). |
| 80 | 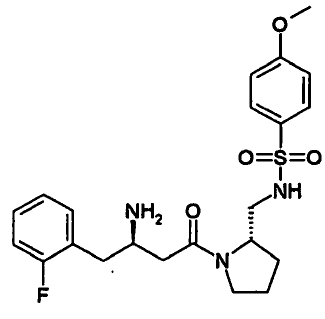 | LC/MS (IV) rt 2.28, m/z 450 (M+H)⁺. | |
| 81 | 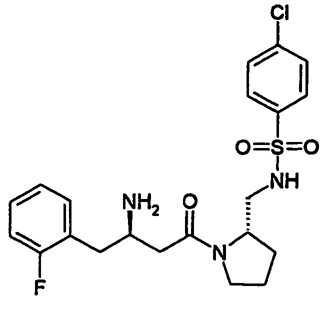 | LC/MS (IV) rt 2.35, m/z 454 (M+H)⁺. | |
| 82 | 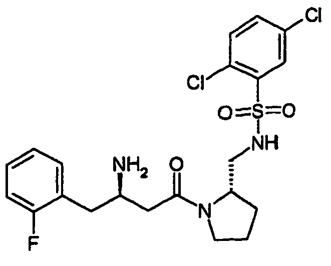 | LC/MS (IV) rt 2.37, m/z 488 (M+H)⁺. | ¹H-NMR δ (ppm) = 1.73-1.90 (m, 4H), 2.72-2.91 (m, 3H), 2.95-3.13 (m, 3H), 3.14-3.31 (m, 1H). 3.62-3.77 (m, 2H), 1 H overlaps with the water signal, 7.03-7.16 (m, 2H), 7.18-7.34 (m, 2H), 7.50-7.73 (m, 3H), 7.80-7.97 (m, 3H), 8.07-8.12 (bs, 3H). |
| 83 | 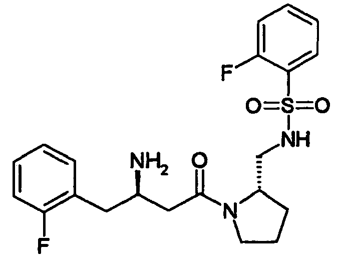 | LC/MS (IV) rt 2.28, m/z 438 (M+H)⁺. | ¹H-NMR δ (ppm) = 1.68-1.90 (m, 4H), 1 H overlaps with the water signal, 2.59-2.76 (m, 1H), 2.82-3.09 (m, 3H), 3.16-3.40 (m, 2H), 3.61-3.80 (m, 2H), 3.86-3.95 (m, 1H), 7.09-7.18 (m, 2H), 7.21-7.52 (m, 5H), 7.66-8.01 (m, 5H). |
| 84 | 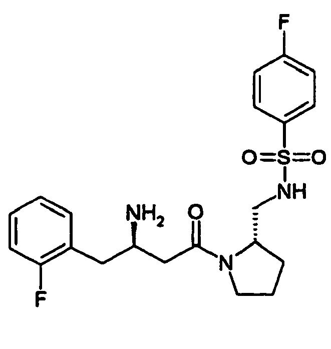 | LC/MS (IV) rt 2.23, m/z 438 (M+H)⁺. | ¹H-NMR δ (ppm) = 1.68-1.90 (m, 4H), 1 H overlaps with the water signal, 2.72-2.89 (m, 2H), 2.92-3.09 (m, 2H), 3.16-3.33 (m, 2H), 3.65-3.82 (m, 2H), 3.85-3.97 (m, 1 H), 7.03-7.17 (m, 2H), 7.20-7.47 (m, 5H), 7.58-8.02 (m, 5H). |
| 85 | 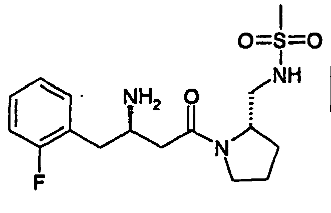 | LC/MS (IV) rt 1.95, m/z 3.58 (M+H)⁺. | |

### Example 86

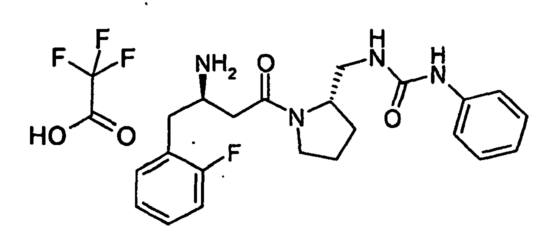

### Step 1

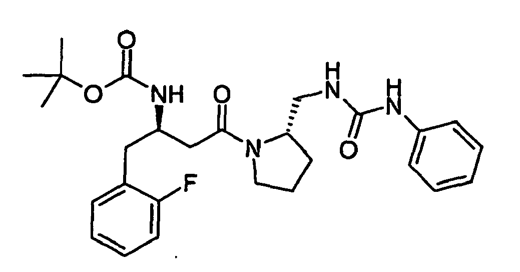

### (1-(2-Fluoro-benzyl)-3-oxo-(3R)-{(2S)-[(3-phenyl-ureido)-methyl]-pyrrolidin-1-yl}-propyl)-carbamic acid tert-butyl ester.

Obtained from (3R)-tert-butoxycarbonylamino-4-(2-fluoro-phenyl)-butyric acid and 1-phenyl-3-pyrrolidin-(2S)-ylmethyl urea (Example 27) according to the procedure described for step 1 in Example 32.
LC/MS (III) rt 4.79, m/z 521 (M+Na)⁺.

### Step 2

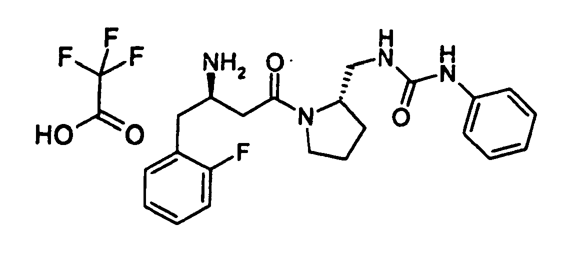

### 1-{1-[(3R)-Amino-4-(2-fluoro-phenyl)-butyryl]-pyrrolidin-(2S)-ylmethyl}-3-phenyl-urea.

Obtained from the product of step 1 according to the procedure described for step 2 in Example 32.
1H-NMR δ (ppm) = 1.79-1.83 (m, 4H), 2.27-2.31 (m, 1H), 2.67-2.69 (m, 2H), 3.29-3.37 (m, 5H), 3.87 and 3.97 (2m, 1H), 6.20 (m, 0.6H), 6.40 (m, 0.2H), 6.81-6.86 (m, 1 H), 7.06-7.39 (m, 8H), 8.41 (bs, 0.5H), 8.52 (bs, 0.2H).
LC/MS (III) rt 3.12, m/z 421 (M+Na)⁺.

### Example 90

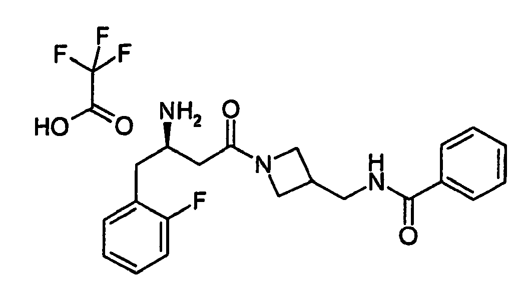

### N-{1-[(3R)-Amino-4-(2-fluoro-phenyl)-butyryl]-azetidin-3-ylmethyl}benzamide (TFA salt).

Obtained from (*3R*)-*tert*-butoxycarbonylamino-4-(2-fluoro-phenyl)-butyric acid and *N-*azetidin-3-yl-methyl-benzylamide (example 8) according the procedure described for example 32.
¹H-NMR δ (ppm) = 1.28 (m, 2H), 2.61-2.92 (m, 2H), 2.95-3.02 (m, 1H), 3.43-3.49 (m, 2H), 3.57-3.70 (m, 2H), 3.74-3.91 (m, 2H), 4.01-4.09 (m, 1H), 7.13-7.19 (m, 2H), 7.29-7.32 (m, 2H), 7.38-7.49 (m, 3H), 7.76-7.80 (m, 2H), 7.92 (bs, 3H), 8.51 (m, 1H).
LC/MS (II) rt 1.92, m/z 370 (M+H)⁺.

### Example 91

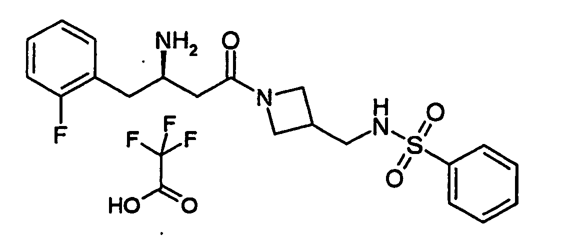

### N-{1-[3-Amino-4-(2-fluoro-phenyl)-butyryl]-azetidin-3-ylmethyl}-benzenesulfonamide (TFA salt).

Obtained from (*3R*)-*tert*-butoxycarbonylamino-4-(2-fluoro-phenyl)-butyric acid *N-*Azetidin-3-ylmethyl-benzenesulfonamide (Example 30) according to the procedure described for example 32.
¹H-NMR δ (ppm) = 1.20-2.29 (m, 2H), 2.52-2.64 (m, 1H), 2.82-3.03 (m, 4H), 3.39-3.49 (m, 1 H), 3.56-3.80 (m, 3H), 3.91-4.00 (m, 1 H), 7.08-7.18 (m, 2H), 7.26-7.33 (m, 2H), 7.52-7.64 (m, 3H), 7.74-7.77 (m, 3H), 8.04 (bs, 3H).
LC/MS (II) rt 1.99, m/z 406 (M+H)⁺.

### Example 92

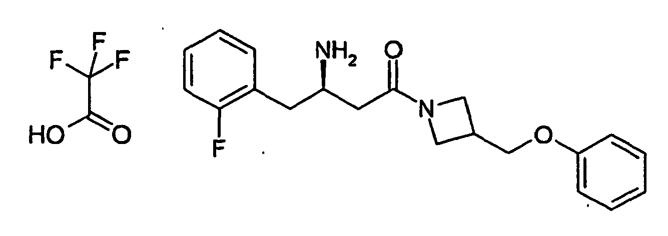

### Step 1

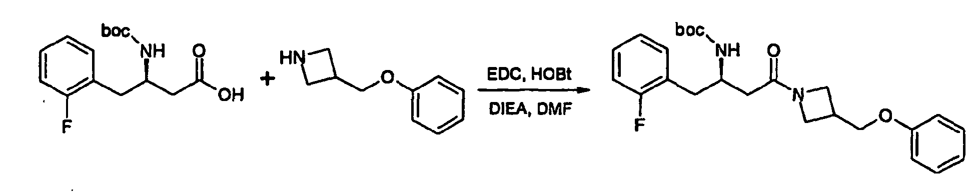

### 1-(2-Fluoro-benzyl)-3-oxo-3-(3-phenoxymethyl-azetidin-1-yl)-propyl]-carbamic acid tert-butyl ester

A mixture of 33.0 mg (0.11 mmol) of (3*R*)*-tert*-butoxycarbonylamino-4-[2-fluoro-phenyl]-butyric acid, 16.0 mg (0.21 mmol) of 1-hydroxybenzotriazole, 23.0 mg (0.12 mmol) of 1-ethyl-3-(3-dimethylaminopropyl)carbodiimide hydrochloride and 114 µL (0.65 mmol) of diisopropylethylamine in 2.5 mL of DCM is stirred for 5 minutes. After addition of 48.0 mg (0.11 mmol) of 3-phenoxymethyl-azetidine (Example 28), the mixture is stirred overnight The solution is diluted with dichloromethane, washed with a saturated aqueous bicarbonate solution and brine, dried over sodium sulphate and evaporated under reduced pressure. The residue is purified using flash chromatography (silica gel, eluent: 25% cyclohexane in ethyl acetate) to afford the title compound.
LC/MS (ll5) rt 3.21, m/z 443 (M+H)⁺.

### Step 2

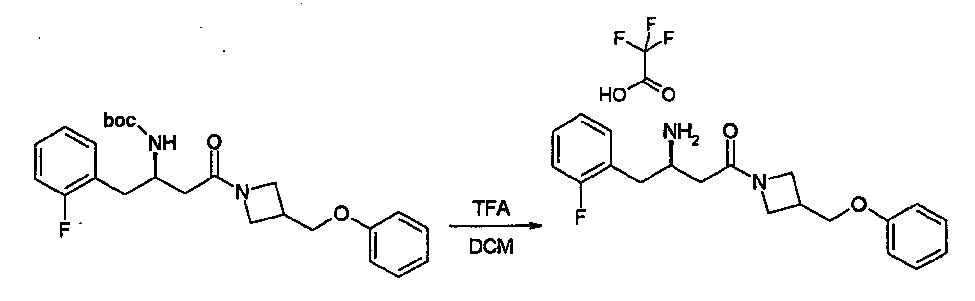

### 3-Amino-4-(2-fluoro-phenyl)-1-(3-phenoxymethyl-azetidin-1-yl)-butan-1-one (TFA salt).

A solution of 20.0 mg (0.045 mmol) of 1-(2-fluoro-benzyl)-3-oxo-3-(3-phenoxymethylazetidin-1-yl)-propyl]-carbamic acid tert-butyl ester in 300 µL of trifluoroacetic acid and 700 µL of dichloromethane is stirred at room temperature for 1 h and then evaporated under reduced pressure to give the title compound.
¹H-NMR δ (ppm) = 2.32 (d, 2H), 2.86-3.06 (m, 3H), 3.60-3.70 (m, 3H), 3.80-4.00 (m, 2H), 4.01-4.17 (m, 2H), 6.91 (t, 3H), 7.15 (t, 2H), 7.23-7.34 (m, 4H), 8.01 (bs, 3H).
LC/MS (II) rt 2.35, m/z 343 (M+H)⁺.

The compounds in Table 13 are synthesized according to the procedure shown for Example 92.

**TABLE 13**

| Example | Structure | LC-MS | NMR |
|---|---|---|---|
| 93 | 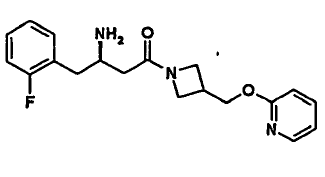 | LC/MS (II) rt 2.02, m/z 344 (M+H)⁺. | ¹H-NMR δ (ppm) = 2.31 (d, 2H), 2.85-3.04 (m, 3H), 3.50-4.05 (m, 4H), 4.12 (q, 1H), 4.37-4.39 (m, 2H), 6.77 (t, 3H), 6.94-6.98 (m, 1H), 7.13-7.18 (m, 2H), 7.31-7.34 (m, 2H), 7.64-7.71 (m, 1H), 7.96 (bs, 3H), 8.09-8.11 (m, 1H). |

### Example 95

Procedure for making an intermediate according to Scheme F.

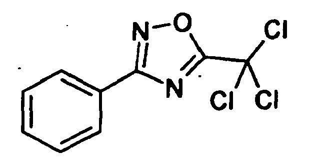

### Step 1

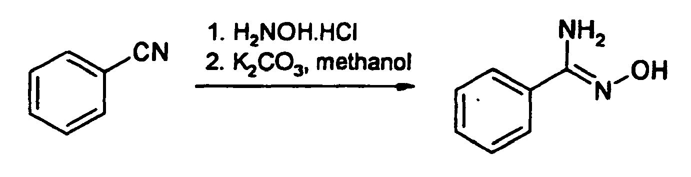

### N-Hydroxybenzamidine

To 10.31 g (0.10 mol) of benzonitrile dissolved in 40 mL methanol is added 20.73 g (0.15 mole) of finely powdered potassium carbonate. To this is added, in small portions with stirring, 13.89 g (0.20 mol) of hydroxylamine hydrochloride dissolved in 120 mL of methanol. The mixture is then refluxed for 5 hours and, after cooling to room temperature, the solvent is removed under reduced pressure. The residue is taken up in 50 mL of water and 200 mL of chloroform. The organic layer is separated, washed twice with 30 mL of water, and dried over magnesium sulphate. The mixture is then filtered and evaporated under reduced pressure. The residue is crystallised with diethyl ether to afford the title compound.
M.P.: 77-79 °C.

### Step 2

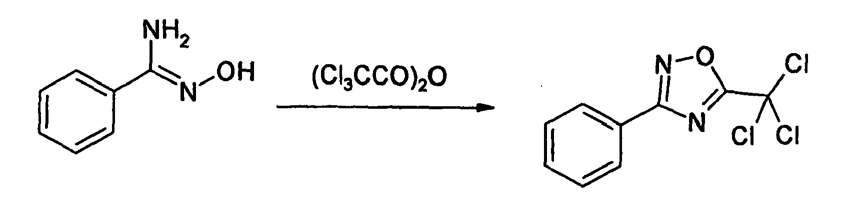

### 3-Phenyl-5-trichloromethyl-[1,2,4]oxadiazole

To 40.05 g (129.7 mmol; 23.7 mL) of trichloroacetic anhydride in a 250 mL round bottom flask protected with a calcium chloride drying tube is added portionwise, with stirring, at room temperature over 20 minutes, 8.82 g (64.80 mmol) of the product from step 1. When addition is complete, the mixture is heated to 90-120°C for 75 minutes, and the hot mixture is then poured into a stirred ice-water solution. The resulting solid is crystallised with hexane or diethylether to give the title compound.
¹H-NMR (300 MHz, CDCl₃) δ = 7.60-7.45 (m, 3H), 8.15 (m, 2H).

### Example 96

Prepared following the procedure outlined for Example 95 according to Scheme F.

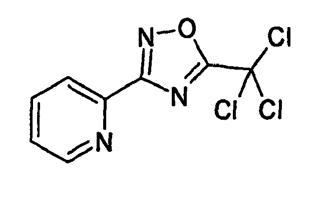

### Step 1

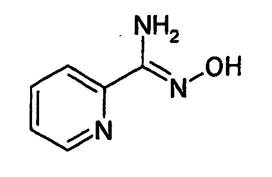

### N-Hydroxypyridine-2-carboxamide

Obtained from pyridine-2-carbonitrile and hydroxylamine hydrochloride according to Step 1 in Example 95.
M.P.: 115-117 °C

### Step 2

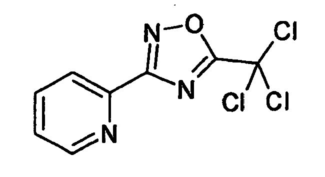

### 2-(5-Trichloromethyl-[1,2,4]oxadiazol-3-yl)-pyridine

Obtained from *N*-hydroxy-pyridine-2-carboxamide (Example 96, Step 1) according to Step 2 in Example 95.
¹H-NMR (300 MHz, DMSO-d₆) δ = 7.68 (2xdd, 1H), 8.07 (ddd, 1H), 8.14 (dd, 1H), 8.81 (m, 1 H).

### Example 97

Prepared following the procedure outlined for Example 95 according to Scheme F.

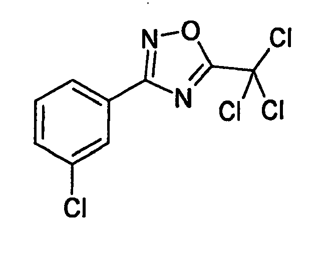

### Step 1

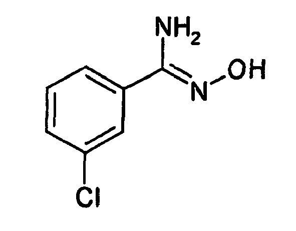

### 3-Chloro-N-hydroxy-benzamidine

Obtained from 3-chlorobenzonitrile and hydroxylamine hydrochloride according to Step 1 in Example 95.
M.P.: 115-118°C

### Step 2

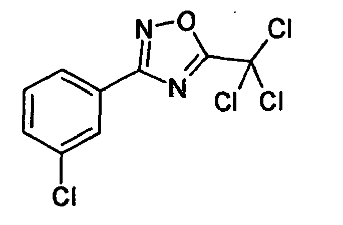

### 3-(3-Chlorophenyl)-5-trichloromethyl-[1,2,4]oxadiazole

Obtained from 3-chlaro-N-hydroxy-benzamidine (Example 97, Step 1) according to Step 2 in Example 95.
¹H-NMR (300 MHz, CDCl₃) δ = 7.44 (dd, 1H), 7.55 (ddd, 1H), 8.04 (ddd, 1H), 8.12 (dd, 1H).

### Example 98

Prepared following the procedure outlined for Example 95 according to Scheme F.

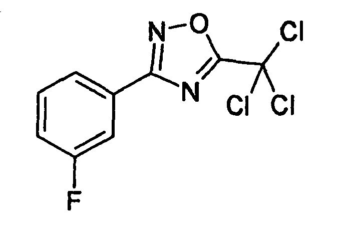

### Step 1

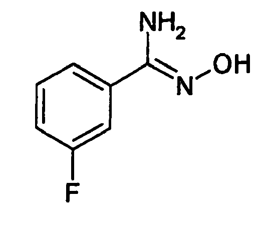

### 3-Fluoro-N-hydroxy-benzamidine

Obtained from 3-fluorobenzonitrile and hydroxylamine hydrochloride according to Step 1 in Example 95.
M.P.: 74-76°C

### Step 2

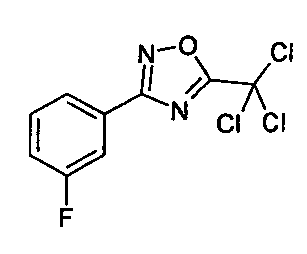

### 3-(3-Fluorophenyl)-5-trichloromethyl-[1,2,4]oxadiazole

Obtained from 3-fluoro-*N*-hydroxy-benzamidine (Example 98, Step 1) according to Step 2 in Example 95.
¹H-NMR (300 MHz, CDCl₃) δ = 7.26 (m, 1H). 7.51 (m, 1 H), 7.75 (m, 1H), 7.93 (m, 1 H).

### Example 99

Prepared following the procedure outlined for Example 95 according to Scheme F.

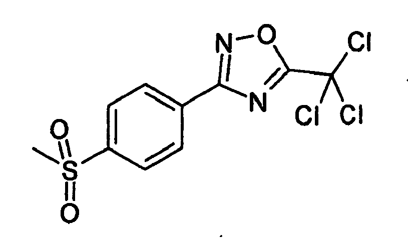

### Step 1

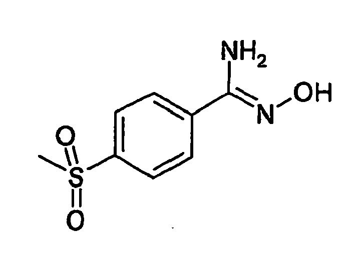

### N-Hydroxy-4-methanesulphonyl-benzamidine

Obtained from 4-methanesulphonyl-benzonitrile and hydroxylamine hydrochloride according to Step 1 in Example 95.
M.P.: 115-118°C

### Step 2

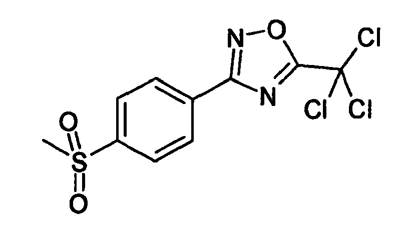

### 3-(4-Methanesulphonyl-phenyl)-5-trichloromethyl-[1,2,4]oxadiazole

Obtained from N hydroxy-4-methanesulphonyl-benzamidine (Example 99, Step 1) according to Step 2 in Example 95.
¹H-NMR (300 MHz, CDCI₃) δ = 3.13 (s, 3H), 8.12 and 8.38 (m, 4H).

### Example 100

Following examples are prepared according to Schemes G and H.

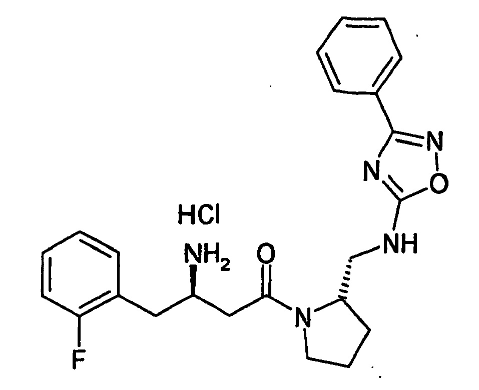

### Step 1

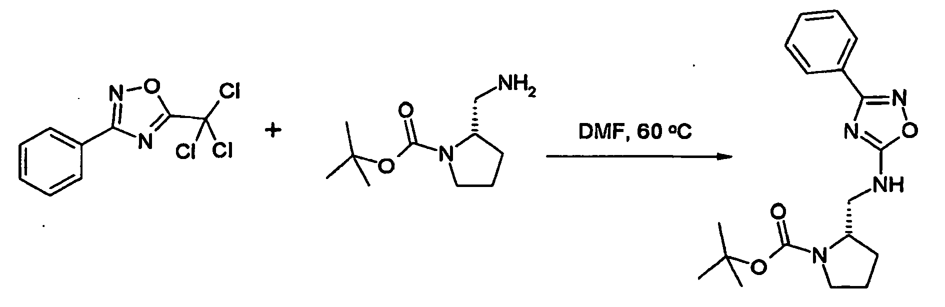

### (2S)-[3-Phenyl-[1,2,4]oxadiazol-5-ylamino)-methyl]pyrrolidine-1-carboxylic acid tert-butyl ester

164.40 mg (0.62 mmol) of 3-phenyl-5-trichloromethyl-[1,2,4]oxadiazole (Example 95) and 150.0 mg (0.75 mmol) of (2*S*)-aminomethyl-pyrrolidine-1-carboxylic acid tert-butyl ester is stirred in 5 mL of dry N,N-dimethylformamide at 60 °C for 12 hours. The progression of the reaction is monitored by TLC (Kieselgel Merck 5554 sheets, eluent: hexane-ethylacetate 2:1). The mixture is evaporated to dryness under reduced pressure and the residue is purified by preparative thin layer-chromatography using the same solvent system to afford the title compound.
1H-NMR (300 MHz, CDC13) δ = 1.45 (s, 9H), 1.62-2.16 (m, 4H), 3.28-3.70 (m, 4H,), 4.17 (m, 1 H), 7.30 (m, 1 H), 7.41 (m, 3H), 7.90 (m, 2H).

### Step 2

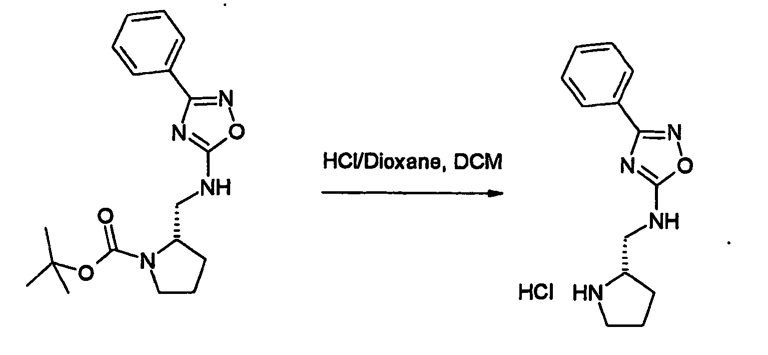

### (3-Phenyl-[1,2,4]oxadiazol-5-yl)pyn-olidin-(2S)-ylmethylamine hydrochloride

The product from Step 1, (2S)-[3-phenyl-[1,2,4]oxadiazol-5-ylamino)-methyl]pyrrolidine-1-carboxylic acid tert-butyl ester, is dissolved in 4 mL of dichloromethane then 8 mL of saturated HCl/dioxane solution is added. After the mixture is stirred for 2 hours the solvent is evaporated under reduced pressure to yield the title compound, which is used directly in the next step without further purification and characterisation.

### Step 3

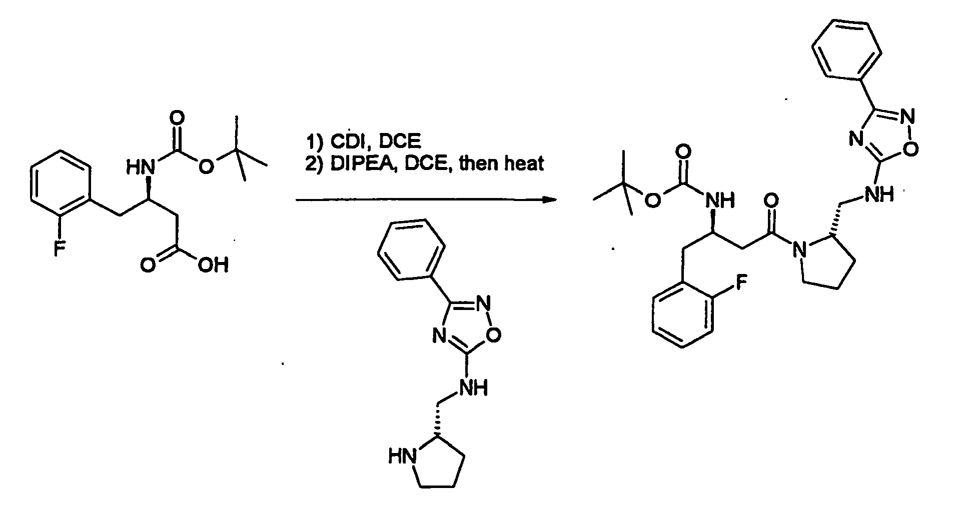

### (1R)-(2-Fluorobenzyl)-3-oxo-3-{(2S)-[(3-phenyl-[1,2,4]oxadiazol-5-ylaminomethyl]pyrrolidin-1-yl}-propyl)carbamic acid tert-butyl ester

In a 25 mL round-bottomed flask is stirred for 2 hours under nitrogen a mixture of 74.90 mg (0.38 mmol) of (3*R*)*-tert*-butoxycarbonylamino-4-(2-fluoro-phenyl)-butyric and 64.70 mg (0.40 mmol; 1.05 eq.) of 1,1'-carbonyldiimidazole in 5 mL of dry 1,2-dichloroethane. Separately, 106.70 mg, (0.38 mmol) of 3-phenyl-[1,2,4]oxadiazol-5-yl)pyrrolidin-(2*S*)-ylmethylamine hydrochloride (Example 100, Step 2) and 107.90 mg, (0.83 mmol; 145.0 µL; 2.2 eq.) of *N*,*N*-diisopropylethylamine is stirred in 4 mL of dry 1,2-dichloroethane for 15 minutes and this solution is poured into the butyric acid and 1,1'-carbonyldiimidazole reaction mixture prepared above. Stirring is continued overnight at room temperature, then the mixture is boiled for 5 hours. The solution is cooled to room temperature, washed successively with a 5 % citric acid solution, saturated sodium hydrogen carbonate solution, water and brine, dried over magnesium sulphate, filtered and the solvent is removed under reduced pressure. The residue is subjected to preparative thin layer chromatography on silica gel (eluent: dichloroethane/ethanol 5:1) to afford the title compound which is taken directly into the next step without further characterisation.

### Step 4

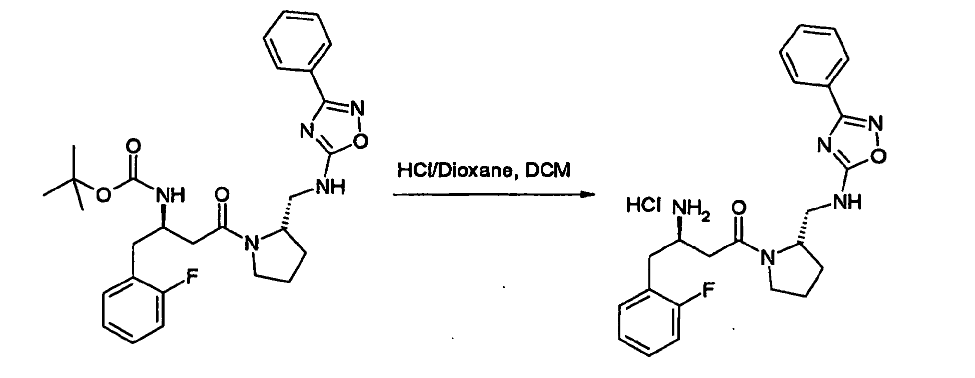

### (3R)-Amino-4-(2-fluorophenyl)-1-[(2S)-[(3-phenyl-[1,2,4]oxadiazol-5-ylamino)-methyl]-pyrrolidin-1-yl}-butan-1-one hydrochloride

(1R)-(2-fluorobenzyl)-3-oxo-3-{(2S)-[(3-phenyl-[1,2,4]oxadiazol-5-ylamino-methyl]-pyrrolidin-1-yl}-propyl)carbamic acid tert-butyl ester, the product from Step 3, is dissolved in 4 mL of dichloromethane then 10 mL of saturated HCl/dioxane solution is added. The mixture is stirred for 2 hours then is the solvent removed under reduced pressure to yield the title compound. If the residue is a solid it is taken up in diethyl ether and hexane and filtered. Otherwise, if the residue is an oil, this is taken up in 10 mL of dioxane and the solvent is evaporated to dryness. This procedure is repeated two times to afford the title compound.
1H-NMR (300 MHz, CDCl3) δ = 1.82-2.00 (m, 4H), 2.66-2.70 (m, 1H), 2.84 (bs, 1H), 3.21 (bs, 1H), 3.33-3.53 (m, 5H), 4.01 (bs, 1H), 4.39 (bs, 1H), 6.92-7.03 (m, 2H), 7.13-7.21 (m, 1H), 7.32-7.47 (m, 3H), 7.58 (bs, 1H), 7.88-7.90 (m, 2H), 7.94-8.06 (m, 1H), 8.66 (m, 3H).
LC/MS (Method VII) m/z 424 [M+H]⁺.

### Example 101

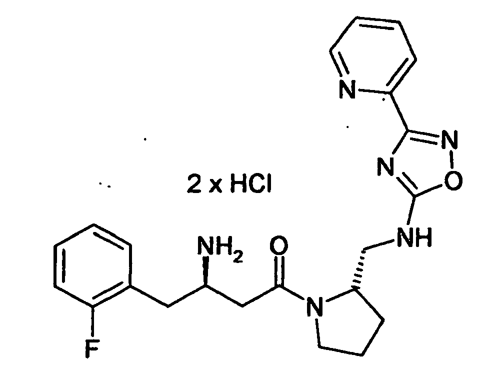

Prepared according to the procedure above outlined for Example 100 Steps 1 to 4, according to Schemes G and H.

### Step 1

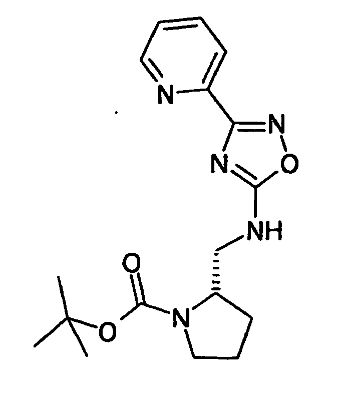

### (2S)-[(3-Pyridin-2-yl-[1,2,4]oxadiazol-5-ylamino)-methyl]-pyrrolidine-1-carboxylic acid tert-butyl ester

Obtained from 2-(5-trichloromethyl-[1,2,4]oxadiazol-3-yl)-pyridine (Example 96) and (2*S*)-aminomethyl-pyrrolidine-1-carboxylic acid *tert*-butyl ester, synthesised according to the procedure for Example 100, Step 1.
¹H-NMR (300 MHz, CDCl₃) δ = 1.45 (s, 9H), 1.60-2.20 (m, 4H), 3.20-3.45 (m, 4H), 4.15 (m, 1 H), 7.38 (m, 1 H), 7.50 (m, 1H), 7.79 (dd, 1 H), 8.08 (dd, 1 H), 8.78 (dd, 1H).

### Step 2

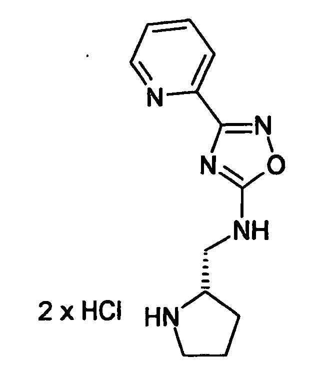

### (3-Pyridin-2-yl-[1,2,4]oxadiazol-5-yl)-pyrrolidin-(2S)-ylmethylamine di-hydrochloride

Obtained from (2S)-[(3-Pyridin-2-yl-[1,2,4]oxadiazol-5-ylamino)-methyl]-pyrrolidine-1-carboxylic acid *tert*-butyl ester (Example 101, Step 1), and synthesised according to the procedure for Example 100, Step 2.

### Step 3

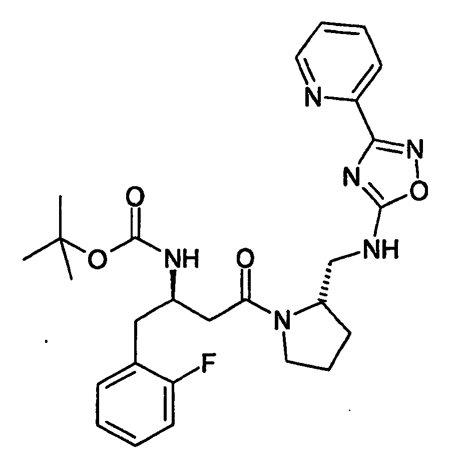

### (1R)-(2-Fluorobenzyl)-3-oxo-3-{(2S)-[(3-pyridin-2-yl-[1,2,4]oxadiazol-5-ylamino)-methyl]-pyrrolidin-1-yl}-propyl)-carbamic acid tert-butyl ester

Obtained from (3-pyridin-2-yl-[1,2,4]oxadiazol-5-yl)-pyrrolidin-(2S)-ylmethylamine di-hydrochloride (Example 101, Step 2) and (3R)-tert-butoxycarbonylamino-4-(2-fluorophenyl)-butyric acid, and synthesised according to Example 100, Step 3.

### Step 4

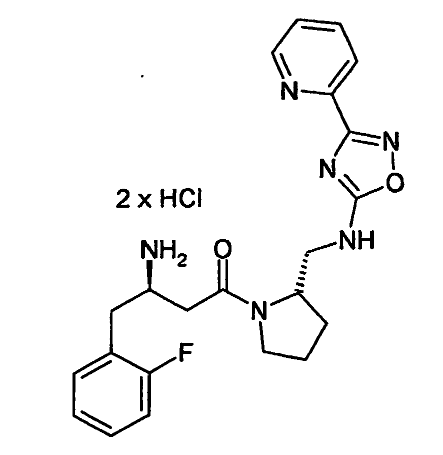

### (3R)-Amino-4-(2-fluorophenyl)-1-{(2S)-[(3-pyridin-2-yl-[1,2,4]oxadiazol-5-ylamino)-methyl]-pyrrolidin-1-yl}-butan-1-one. Di-hydrochloride

Obtained from (1*R*)-(2-fluorobenzyl)-3-oxo-3-{(2*S*)-[(3-pyridin-2-yl-[1,2,4]oxadiazol-5-ylamino)-methyl]-pyrrolidin-1-yl}-propyl)-carbamic acid *tert*-butyl ester (Example 101, Step 3), and synthesised according to Example 100, Step 4.
¹H-NMR (300 MHz, CDCl₃ + DMSO-d₆) δ = 1.88-1.99 (m, 4H), 2.50-2.58 (m, 1H), 2.62-. 2.68 (m, 1H), 2.97-3.03 (m, 1H), 3.12-3.17 (m, 1H), 3.33-3.40 (m, 3H), 3.50-3.54 (m, 1 H), 3.70-3.74 (m, 1 H), 4.25-4.27 (m, 1H). 7.04-7.16 (m, 2H), 7.24-7.35 (m, 2H), 7.53-7.58 (m, 1H). 7.95-8.06 (m, 2H), 8.19-8.24 (bs, 3H), 8.43-8.46 (m, 1 H), 8.70-8.76 (m, 1 H).
LC/MS (Method VII) m/z 425 [M+H]⁺

### Example 102

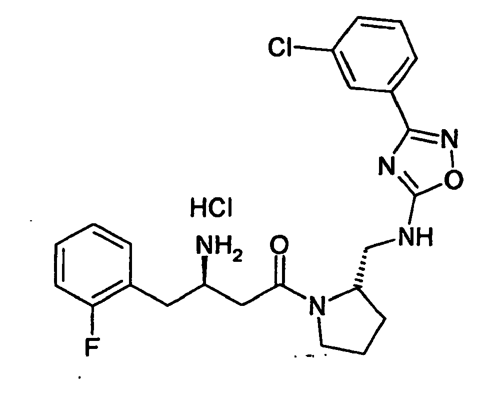

Prepared according to the procedure above outlined for Example 100, steps 1-4 according to Schemes G and H.

### Step 1

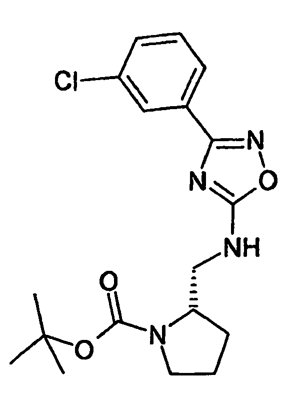

### (2S)-{[3-(3-Chlorophenyl)-[1,2,4]oxadiazol-5-ylamino]-methyl}-pyrrolidine-1-carboxylic acid tert-butyl ester

Obtained from 3-(3-Chlorophenyl)-5-trichloromethyl-[1,2,4]oxadiazole (Example 97) and (2*S*)-aminomethyl-pyrrolidine-1-carboxylic acid *tert*-butyl ester, synthesised according to the procedure for Example 100, Step 1.

### Step 2

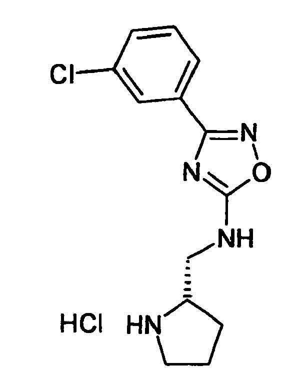

### [3-(3-Chlorophenyl)-[1,2,4]oxadiazol-5-yl]-pyrrolidin-(2S)-ylmethylamine hydrochloride

Obtained from (2*S*)-{[3-(3-chlorophenyl)-[1,2,4]oxadiazol-5-ylamino]-methyl}-pyrrolidine-1-carboxylic acid *tert*-butyl ester (Example 102, Step 1), and synthesised according to the procedure for Example 100, Step 2.
¹H-NMR (300 MHz, CDCl₃ + d₆-DMSO) δ = 1.75-2.20 (m, 4H), 3.20-3.30 (m, 2H), 3.71 (m, 2H), 3.81 (m, 1H), 7.40-7.50 (m, 2H), 7.85 (ddd, 1H), 7.88 (dd, 1H), 8.57 (m, 1H), 9.00 and 9.42 (bm, 2H).

### Step 3

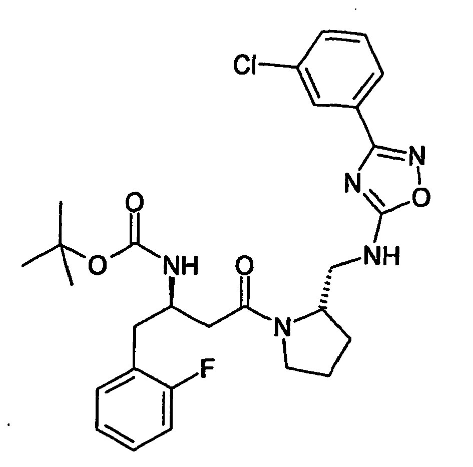

### [3-(2S)-{[3-(3-Chlorophenyl)-[1,2,4]oxadiazol-5-ylamino]-methyl}-pyrrolidin-1-yl)-(1R)-(2-fluorobenzyl)-3-oxo-propyl]-carbamic acid tert-butyl ester

Obtained from [3-(3-chlorophenyl)-[1,2,4]oxadiazol-5-yl-pyrrolidin-(2S)-ylmethylamine hydrochloride (Example 102, Step 2) and (3R)-tert-butoxycarbonylamino-4-(2-fluorophenyl)-butyric acid, and synthesised according to Example 100, Step 3.
1H-NMR (300 MHz, CDCl₃) δ = 1.38 (s, 9H), 1.80-2.10 (m, 4H), 2.50 and 2.90 (m, 2 x 2H), 3.20-3.70 (m, 4H), 4.23 (m, 1 H), 4.38 (m, 1H), 5.38 (m, 1 H), 7.00-7.25 (m, 4H), 7.32 (bm,), 7.35 (m, 1 H), 7.42 (m, 1 H), 7.87 (ddd, 1 H), 7.97 (dd, 1 H).

### Step 4

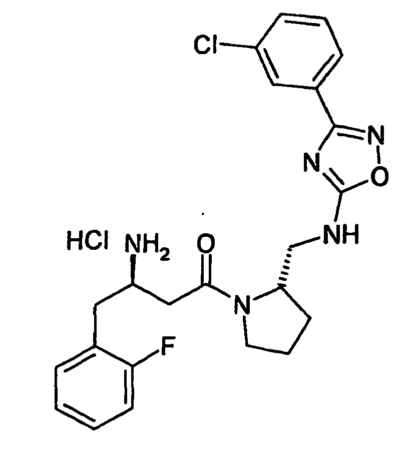

### (3R)-Amino-1-(2S)-{[3-(3-chlorophenyl)-[1,2,4]oxadiazol-5-ylamino]methyl}-pyrrolidin-1-yl)-4-(2-fluorophenyl)-butan-1-one hydrochloride

Obtained from [3-(2*S*)-{[3-(3-chlorophenyl)-[1,2,4]oxadiazol-5-ylamino]-methyl}pyrrolidin-1-yl)-(1*R*)-(2-fluorobenzyl)-3-oxo-propyl]-carbamic acid *tert*-butyl ester (Example 102, Step 3), and synthesised according to Example 100, Step 4.
¹H-NMR (300 MHz, CDCl₃) δ = 1.82-1.99 (m, 4H), 2.67-2.73 (m, 1H), 2.83-2.89 (m, 1H), 3.21-3.25 (m, 1H), 3.39-3.53 (m, 5H), 3.99-4.03 (m, 1H), 4.40-4.42 (m, 1H), 6.93-6.97 (m, 1H), 7.00-7.04 (m, 1 H), 7.14-7.19 (m, 1H), 7.21-7.40 (m 3H), 7.59 (bs, 1H), 7.76-7.78 (m, 1H), 7.87 (s, 1H), 8.66 (bs, 3H).
LC/MS (Method VII) m/z 458 [M+H]⁺

### Example 103

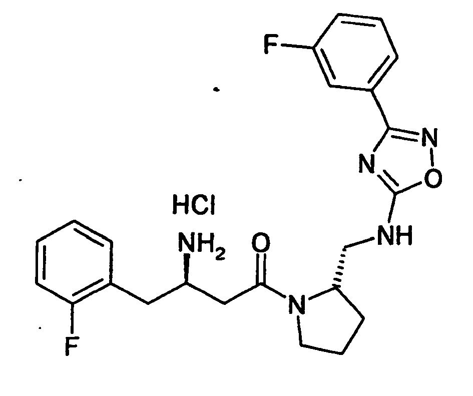

Prepared according to the procedure above outlined for Example 100, steps 1-4, according to Schemes G and H.

### Step 1

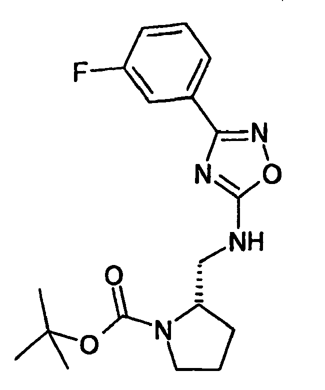

### (2S)-{[3-(3-Fluorophenyl)-[1,2,4]oxadiazol-5-ylamino]-methyl}-pyrrolidine-1-carboxylic acid tert-butyl ester

Obtained from 3-(3-fluorophenyl)-5-trichloromethyl-[1,2,4]oxadiazole (Example 98) and (2S)-aminomethyl-pyrrolidine-1-carboxylic acid *tert*-butyl ester, synthesised according to the procedure for Example 100, Step 1.

### Step 2

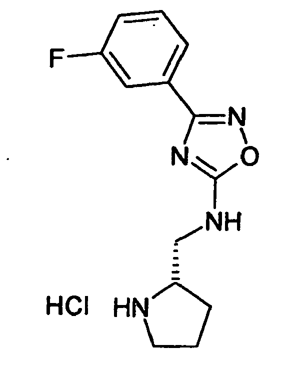

### [3-(3-Fluorophenyl)-[1,2,4]oxadiazol-5-yl]-pyrrolidin-(2S)-ylmethylamine hydrochloride

Obtained from (2*S*)-{[3-(3-Fluorophenyl)-[1,2,4]oxadiazol-5-ylamino]-methyl}pyrrolidine-1-carboxylic acid *tert*-butyl ester (Example 103, Step 1), and synthesised according to the procedure for Example 100, Step 2.
¹H-NMR (300 MHz, CDCl₃ + d₈-DMSO) δ = 1.75-2.20 (m, 4H), 3.10-3.38 (m, 2H), 3.69 (m, 2H), 3.81 (m, 1 H), 7.25 (m, 1 H), 7.48 (m, 1 H), 7.66 (ddd, 1 H), 7.78 (dd, 1 H), 8.62 (1H, m, NH), 8.90 and 9.42 (bm, 2H).

### Step 3

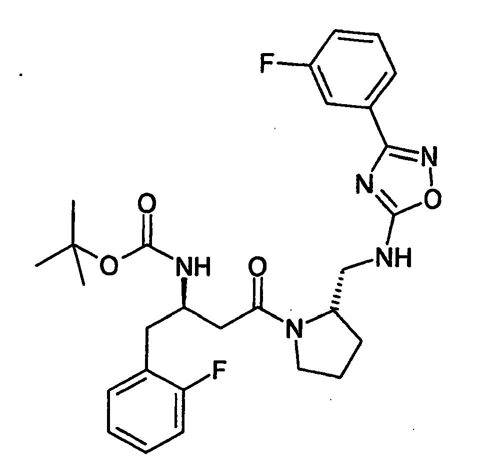

### [(1R)-(2-Fluorobenzyl)-3-((2S)-{[3-(3-fluorophenyl)-[1,2,4]oxadiazol-5-ylamino]-methyl}-pyrrolidin-1-yl)-3-oxo-propyl]-carbamic acid tert-butyl ester

Obtained from [3-(3-fluorophenyl)-[1,2,4]oxadiazol-5-yl]-pyrrolidin-(2S)-ylmethylamine hydrochloride (Example 103, Step 2) and (3R)-tert-butoxycarbonylamino-4-(2-fluorophenyl)-butyric acid, and synthesised according to Example 100, Step 3.

### Step 4

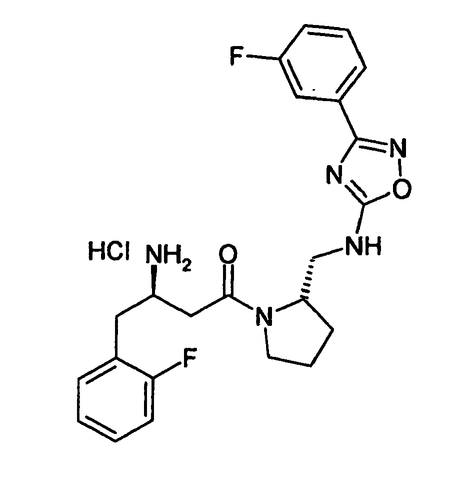

### (3R)-Amino-4-(2-fluorophenyl)-1-((2S)-{[3-(3-fluorophenyl)-[1,2,4]oxadiazol-5-ylaminol]-methyl}-pyrrolidin-1-yl)-butan-1-one hydrochloride

Obtained from [(1*R*)-(2-fluorobenzyl)-3-((2*S*)-{[3-(3-fluorophenyl)-[1,2,4]oxadiazol-5-ylamino]-methyl}-pyrrolidin-1-yl)-3-oxo-propyl]-carbamic acid *tert-butyl* ester (Example 103, Step 3), and synthesised according to Example 100, Step 4.
¹H-NMR (300 MHz, CDCl₃) δ = 1.79-2.06 (m, 4H), 2.73-2.83 (m, 2H), 3.21-3.27 (m, 1H), 3.38-3.54 (m, 3H), 3.58-3.72 (m, 2H), 3.97-4.05 (m, 1H), 4.42-4.49 (m, 2H), 6.93-6.97 (m, 1H), 7.01-7.04 (m, 1H), 7.12-7.17 (m, 2H), 7.30-7.41 (m, 2H), 7.62-7.65 (m, 1 H), 7.74-7.76 (d, 1 H), 8.32 (bs, 1 H), 8.62 (bs, 3H).
LC/MS (Method VII) m/z 442 [M+H]⁺

### Example 104

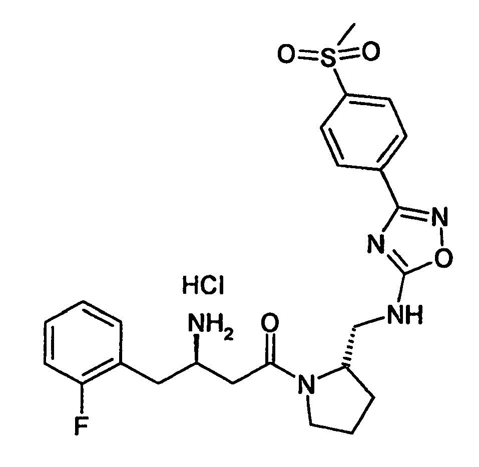

Prepared according to the procedure above outlined for Example 100, steps 1-4, according to Schemes G and H.

### Step 1

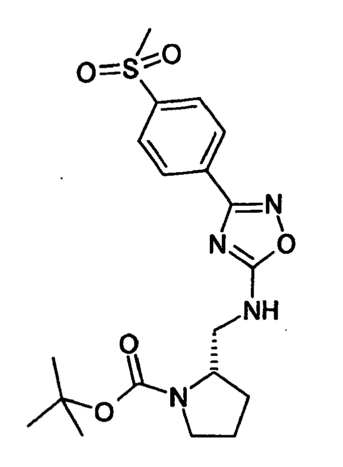

### (2S)-{[3-(4-Methanesulphonylphenyl)-[1,2,4]oxadiazol-5-ylamino]-methyl}-pyrrolidine-1-carboxylic acid tert-butyl ester

Obtained from 3-(4-methanesulphonyl-phenyl)-5-bichloromethyl-[1,2,4]oxadiazole (Example 99) and (2*S*)-aminomethyl-pyrrolidine-1-carboxylic acid *tert*-butyl, synthesised according to the procedure for Example 100, Step 1.
¹H-NMR (300 MHz, CDCl₃) δ = 1.45 (s, 9H), 1.60-2.18 (m, 4H), 3.05 (s, 3H), 3.25-3.70 (m, 4H), 4.17 (m, 1H), 7.46 (m, 1H), 8.00 (m, 2H), 8.20 (m, 2H).

### Step 2

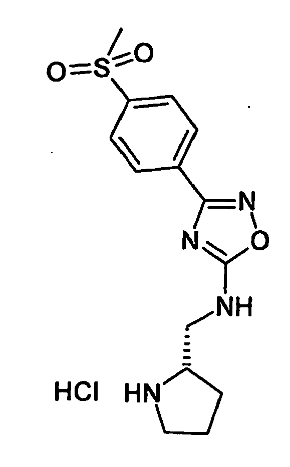

### [3-(4-Methanesulahonylphenyl)-[1,2,4]oxadiazol-5-yl]-pyrrolidin-(2S)-ylmethylamine hydrochloride

Obtained from (2S)-{[3-(4-methanesulphonylphenyl)-[1,2,4]oxadiazol-5-ylamino]-methyl}-pyrrolidine-1-carboxylic acid *tert-*butyl ester (Example 104, Step 1), and synthesised according to the procedure for Example 100, Step 2.

### Step 3

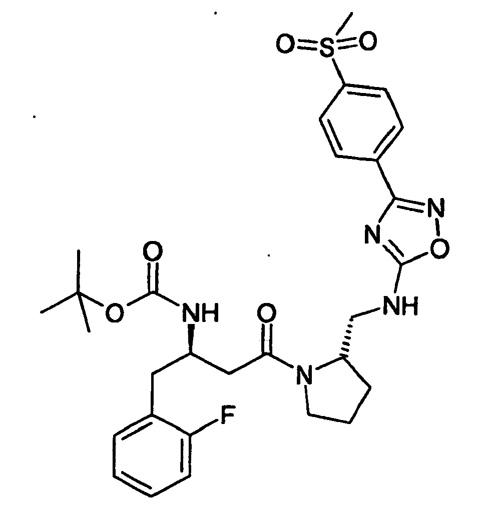

### [(1R)-(2-Fluorobenzyl)-3-((2S)-{[3-(4-methanesulphonylphenyl)-[1,2,4]oxadiazol-5-ylamino]-methyl}-pyrrolidin-1-yl)-3-oxo-propyl]-carbamic acid tert-butyl ester

Obtained from [3-(4-methanesulphonylphenyl)-[1,2,4]oxadiazol-5-yl]-pyrrolidin-(2S)-ylmethylamine hydrochloride (Example 104, Step 2) and (3R)-tert-butoxycarbonylamino-4-(2-fluoro-phenyl)-butyric acid, and synthesised according to Example 100, Step 3.

### Step 4

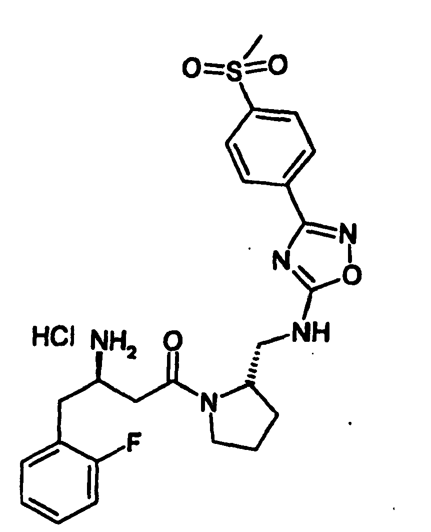

### (3R)-Amino-4-(2-fluorophenyl)-1-((2-S)-{[3-(4-methanesulphonylphenyl)-[1,2,4]oxadiazol-5-ylamino]-methyl-pyrrolidin-1-yl)-butan-1-one hydrochloride.

Obtained from [(1*R*)-(2-Fluorobenzyl)-3-((2*S*)-{[3-(4-methanesulphonylphenyl)-[1,2,4]oxadiazol-5-ylamino]-methyl]-pyrrolidin-1-yl)-3-oxo-propyl]-carbamic acid *tert-*butyl ester (Example 104, Step 3), and synthesised according to Example 100, Step 4. ¹H-NMR (300 MHz, CDCl₃) δ = 1.80-2.03 (m, 4H), 2.75-2.81 (m, 2H), 3.07 (s, 3H), 3.21-3.27 (m, 1H). 3.34-3.55 (m, 4H), 3.60.3.68 (m, 1H). 3.96-4.00 (m, 1H). 4.43-4.50 (m, 1H), 6.94-0.98 (m, 1H). 7.02-7.05 (m, 1H). 7.16-7-21 (m. 1H). 7.30-7.34 (m, 1H), 7.98 (d, 2H), 8.14 (d, 2H), 8.35 (bs, 1H), 8.64 (bs, 3H).
LC/MS (Method VII) m/z 502 [M+H]⁺.

Further examples from this series are exemplified below:

| | |
|---|---|
| 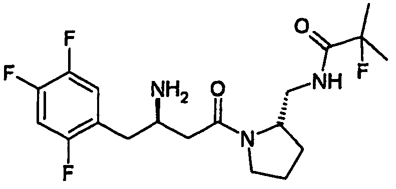 | 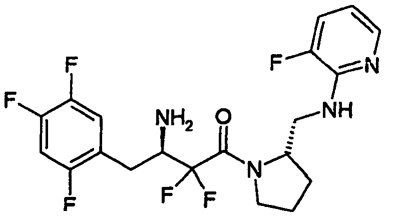 |
| 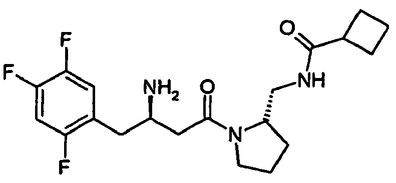 | 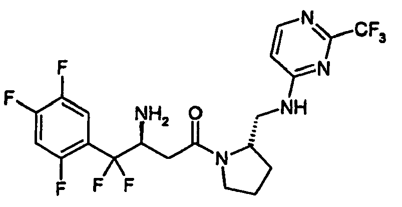 |
| 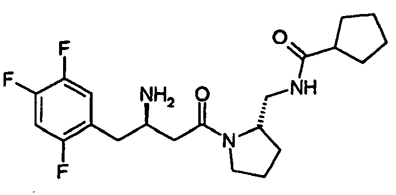 | 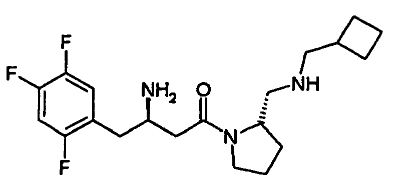 |
| 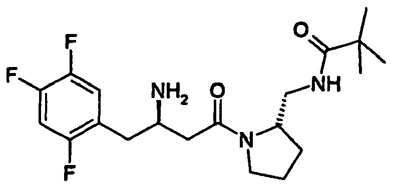 | 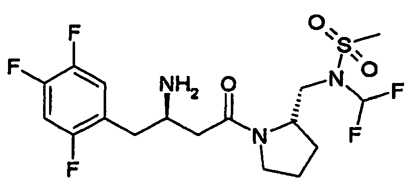 |
| 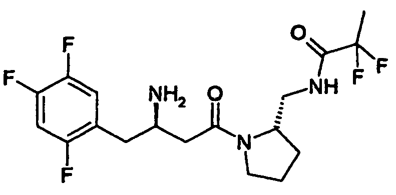 | 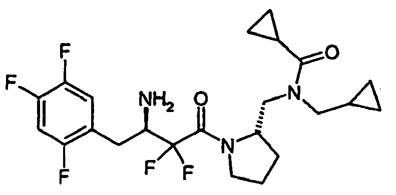 |
| 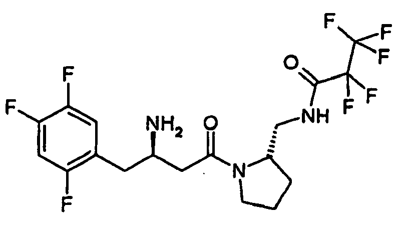 | 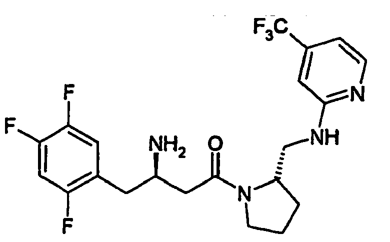 |
| 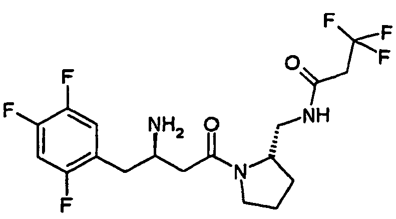 | 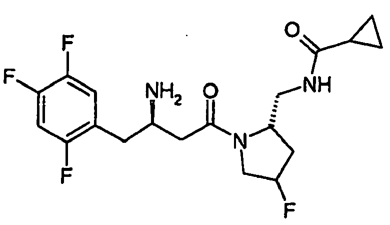 |
| 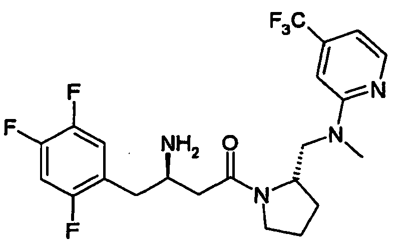 | 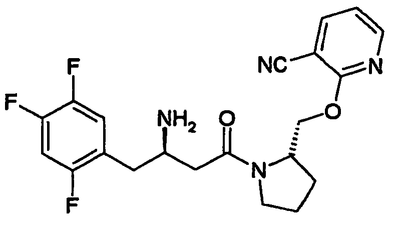 |
| 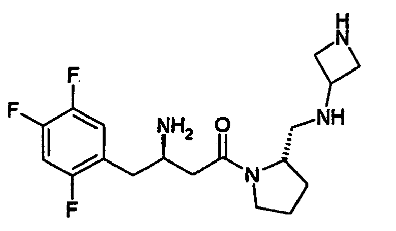 | 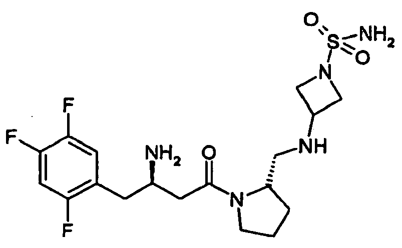 |
| 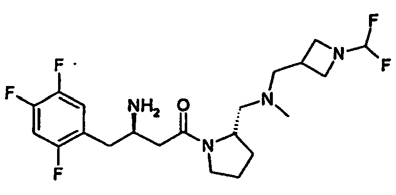 | 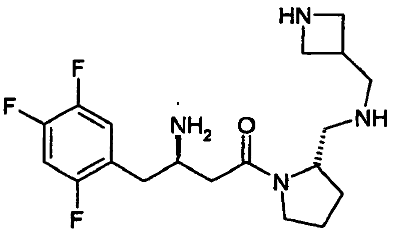 |
| 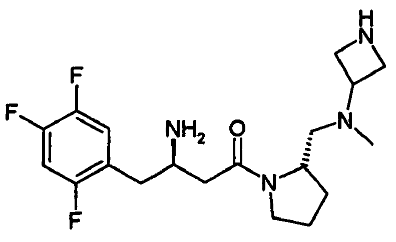 | 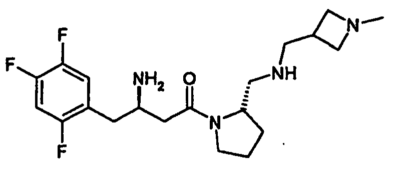 |
| 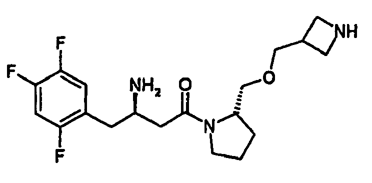 | 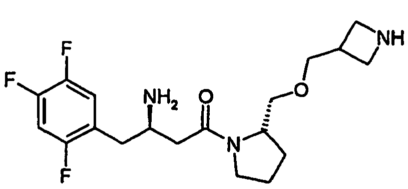 |
| 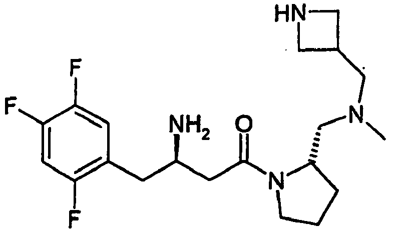 | 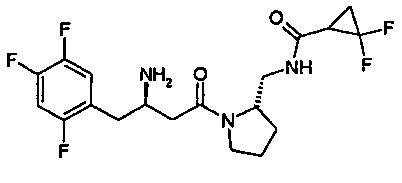 |
| 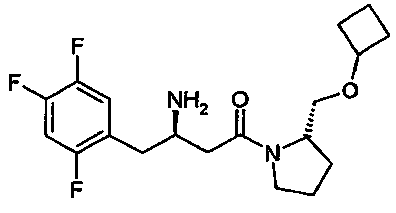 | 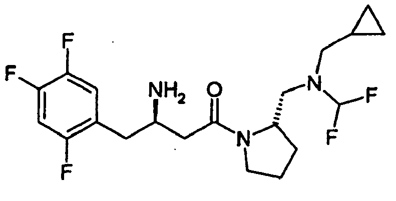 |

### ASSAYS

Inhibition of DPP-IV peptidase activity was monitored with a continuous fluorimetric assay. This assay is based on the cleavage of the substrate Gly-Pro-AMC (Bachem) by DPP-IV, releasing free AMC. The assay is carried out in 96-well microtiterplates. In a total volume of 100 µL, compounds are preincubated with 50 pM DPP-IV employing a buffer containing 10mM Hepes, 150mM NaCl, 0.005% Tween 20 (pH 7.4). The reaction is started by the addition of 16 µM substrate and the fluorescence of liberated AMC is detected for 10 minutes at 25 °C with a fluorescence reader (BMG-Fluostar; BMG-Technologies) using an excitation wavelength of 370 nm and an emission wavelength of 450 nm. The final concentration of DMSO is 1 %. The inhibitory potential of the compounds were determined. DPP-IV activity assays were carried out with human and porcine DPP-IV (see below); both enzymes showed comparable activities-include.

Soluble human DPP-IV lacking the transmembrane anchor (Gly31-Pro766) was expressed in a recombinant YEAST-strain as Pre-Pro-alpha-mating fusion. The secreted product (rhuDPP-IV-Gly31-Pro766) was purified from fermentation broth (>90% purity) and used for inhouse screening.

In the table are listed the IC₅₀ values for inhibition of DPP-IV peptidase activity determined in assays as described above. The IC₅₀ values were grouped in 3 classes: a≤ 100 nM; b ≥101 nM and ≤1001 nM; c ≥1001 nM≤ 2000 nM.

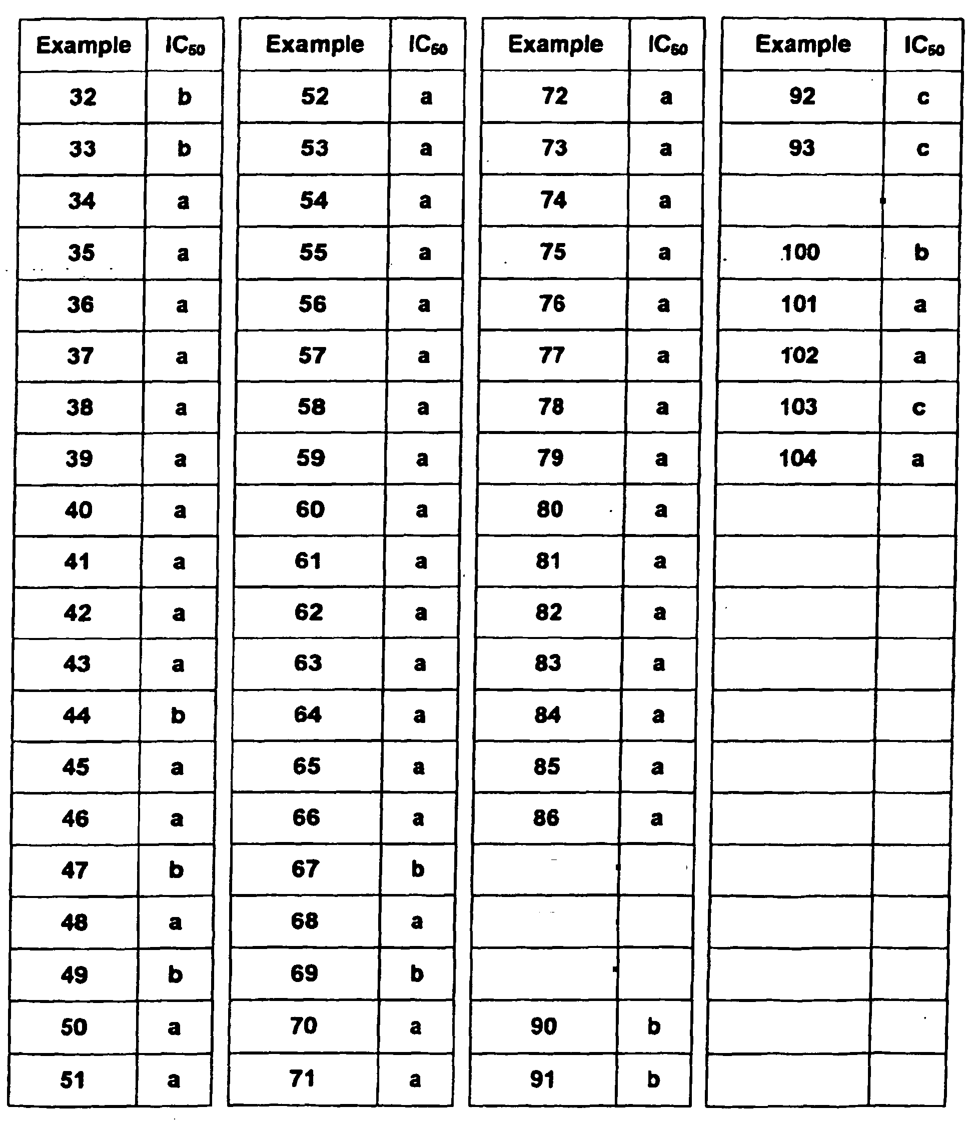

## Claims

1. A compound of formula (I) 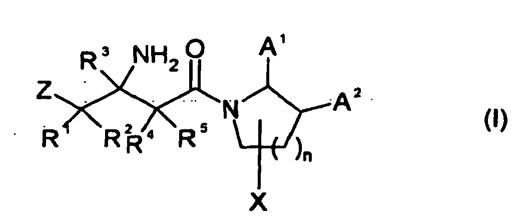 or a pharmaceutically acceptable salt thereof, wherein
Z is selected from the group consisting of
phenyl;
naphthyl;
C₃₋₇ cycloalkyl;
heterocycle; and
heterobicycle;
wherein Z is optionally substituted with one, or independently from each other, more of
halogen;
CN;
OH;
=O, where the ring is at least partially saturated;
C₁₋₆ alkyl, optionally substituted with one or more F; and
O-C₁₋₆ alkyl, optionally substituted with one or more F;
R¹, R², R⁴, R⁵ are independently from each other selected from the group consisting of
H;
F;
OH;
C₁₋₆ alkyl, optionally substituted with one or more F; and
O-C₁₋₆ alkyl, optionally substituted with one or more F;
and/or R¹ and R² optionally form together C₃₋₇ cycloalkyl, which is optionally substituted with one or more F;
and/or R² and R³ optionally form together C₃₋₇ cycloalkyl, which is optionally substituted with one or more F;
and/or R³ and R⁴ optionally form together C₃₋₇ cycloalkyl, which is optionally substituted with one or more F;
and/or R⁴ and R⁵ optionally form together C₃₋₇ cycloalkyl, which is optionally substituted with one or more F;
R³ is H or C₁₋₆ alkyl;
X is selected from the group consisting of
H;
F; and
C₁₋₆ alkyl, optionally substituted with one or more F;
n is 0 or 1;
A¹, A² are independently from each other selected from the group consisting of
H;
halogen;
C₁₋₆ alkyl, optionally substituted with one or more F; and
R⁶; provided that one of A¹ and A² is R⁶;
R⁶ is -C(R⁷R⁸)-Y-T;
R⁷, R⁸ are independently from each other selected from the group consisting of
H;
F; and
C₁₋₆ alkyl, optionally substituted with one or more F;
and/or R⁷ and R⁸ optionally form together C₃₋₇ cycloalkyl, which is optionally substituted with one or more F;
Y is selected from the group consisting of
-O-;
-C₁₋₆ alkyl-O-;
-N(R⁹)-;
-C₁₋₆ alkyl-N(R⁹)-
-S-;
-C₁₋₆ alkyl-S-;
-S(O)-;
-C₁₋₆ alkyl-S(O)-;
-S(O)₂-; and
-C₁₋₆ alkyl-S(O)₂-:
wherein each C₁₋₆ alkyl is optionally substituted with one or more F;
R⁹, T are independently from each other T¹-T² or T²;
T¹ is selected from the group consisting of
-C₁₋₆ alkyl-;
-C₁₋₆ alkyl-O-
-C₁₋₆ alkyl-N(R¹⁰)-
-C(O)-;
-C(O)-C₁₋₆ alkyl-;
-C(O)-C₁₋₆ alkyl-O-;
-C(O)-C₁₋₆ alkyl-N(R¹⁰)-;
-C(O)O-;
-C(O)O-C₁₋₆ alkyl-;
-C(O)O-C₁₋₆ alkyl-O-;
-C(O)O-C₁₋₆ alkyl-N(R¹⁰)-;
-C(O)N(R¹⁰)-;
-C(O)N(R¹⁰)-C₁₋₆ alkyl-;
-C(O)N(R¹⁰)-C₁₋₆ alkyl-O-;
-C(O)N(R¹⁰)-C₁₋₆ alkyl-N(R¹¹)-;
-S(O)₂-;
-S(O)₂-C₁₋₆ alkyl-;
-S(O)₂-C₁₋₆ alkyl-O-; and
-S(O)₂C₁₋₆ alkyl-N(R¹⁰)-;
wherein each C₁₋₆ alkyl is optionally substituted with one or more F;
R¹⁰, R¹¹ are independently from each other H or C₁₋₆ alkyl, optionally substituted with one or more F;
T² is selected from the group consisting of
H;
CF₃;
phenyl;
naphthyl;
wherein phenyl and naphthyl are optionally substituted with one, or independently from each other, more of
halogen;
CN;
R¹²;
COOH;
OH;
C(O)NH₂;
S(O)₂NH₂;
COOT³;
OT³;
C(O)NHT³;
S(O)₂NHT³; or
T³;
C₃₋₇, cycloalkyl;
heterocycle; and
heterobicycle;
wherein C₃₋₇ cycloalkyl, heterocycle and heterobicycle are optionally substituted with one, or independently from each other, more of
halogen;
CN;
R¹³;
OH;
=O, where the ring is at least partially saturated;
NH₂
COOH;
C(O)NH₂;
S(O)₂ NH₂;
COOT³;
OT³;
C(O)NHT³;
S(O)₂NNT³;
NHT³; or
T³;
whereby when R⁹ is T¹-T² and represents -C₁₋₆ alkyl and T is T¹-T² and represents -C₁₋₆ alkyl then R⁹ and T may form together a 3 to 7 membered cyclic group containing 1 N;
R¹² is selected from the group consisting of
C₁₋₆ alkyl;
O-C₁₋₆ alkyl;
COO-C₁₋₆ alkyl;
OC(O)- C₁₋₆ alkyl;
C(O)N(R¹⁵)- C₁₋₆ alkyl;
S(O)₂N(R¹⁷)-C₁₋₆ alkyl;
S(O)-C₁₋₆ alkyl;
S(O)₂-C₁₋₆ alkyl; and
N(R¹⁸)S(O)₂-C₁₋₆ alkyl;
wherein each C₁₋₆ alkyl is optionally substituted with one, or independently from each other, more of F, COOR¹⁹, C(O)N(R²⁰R²¹), S(O)₂N(R²²R²³), OR²⁴, N(R²⁵R²⁶), T³, O-T³ or N(R²⁷)-T³;
R¹³ is selected from the group consisting of
C₁₋₆ alkyl;
O-C₁₋₆ alkyl;
N(R¹⁴)-C₁₋₆ alkyl;
COO-C₁₋₈ alkyl;
OC(O)-C₁₋₆ alkyl;
C(O)N(R¹⁵)-C₁₋₆ alkyl;
N(R¹⁶)-C(O)-C₁₋₆ alkyl;
S(O)₂N(R¹⁷)-C₁₋₆ alkyl;
S(O)-C₁₋₆ alkyl;
S(O)2-C₁₋₆ alkyl; and
-N(R¹⁸)S(O)₂-C₁₋₆ alkyl;
wherein each C₁₋₆ alkyl is optionally substituted with one, or independently from each other, more of F, COOR¹⁹,C(O)N(R²⁰R²¹), S(O)₂N(R²²R²³), OR²⁴, N(R²⁵R²⁶), T³, O-T³ or N(R²⁷)-T³;
R¹⁴, R¹⁵, R¹⁶, R¹⁷, R¹⁸, R¹⁹, R²⁰ , R²¹, R²² , R²³, R²⁴, R²⁵, R²⁶, R²⁷ are independently from each other H or C₁₋₆ alkyl;
T³ is selected from the group consisting of
phenyl;
naphthyl;
wherein phenyl and naphthyl are optionally substituted with one, or independently from each other, more of
halogen;
CN;
COOH;
OH;
C(O)NH₂;
S(O)₂NH₂:
C₁₋₆ alkyl;
O-C₁₋₆ alkyl;
COO-C₁₋₆ alkyl;
OC(O)-C₁₋₆ alkyl;
C(O)N(R²⁸)-C₁₋₆ alkyl;
S(O)₂N(R²⁹)-C₁₋₆ alkyl;
S(O)₂-C₁₋₆ alkyl; or
N(R³⁰)S(O)₂-C₁₋₆ alkyl;
heterocycle;
heterobicycle; and
C₃₋₇ cycloalkyl;
wherein C₃₋₇ cycloalkyl, heterocycle and heterobicycle are optionally substituted with one, or independently from each other, more of
halogen;
CN;
OH;
=O, where the ring is at least partially saturated;
NH₂
COOH;
C(O)NH₂;
S(O)₂NH₂;
C₁₋₆ alkyl;
O-C₁₋₆ alkyl;
N(R³¹)-C₁₋₆ alkyl;
COO-C₁₋₆ alkyl;
OC(O)-C₁₋₆ alkyl;
C(O)N(R³²)-C₁₋₆ alkyl;
N(R³³)-C(O)-C₁₋₆ alkyl;
S(O)₂N(R³⁴)-C₁₋₆ alkyl;
S(O)₂-C₁₋₆ alkyl; or
-N(R³⁵)S(O)₂-C₁₋₆ alkyl.

2. A compound according to claim 1 of formula (Ia) 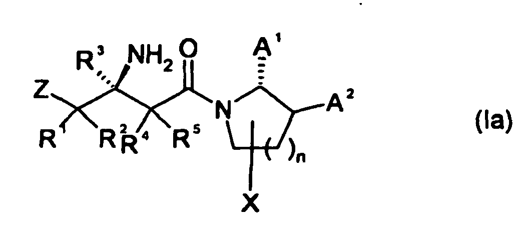 or a pharmaceutically acceptable salt thereof, wherein Z, R¹-R⁵, A¹, A², n and X have the meaning as indicated in claim 1.

3. A compound according to claim 1 or 2, wherein Z is phenyl or heterocycle and Z is optionally substituted independently from each other with up to 2 of CI, F, CN, CH₃ or OCH₃.

4. A compound according to any one of the preceding claims, wherein R¹, R², R⁴, R⁵ are independently from each other selected from the group consisting of H, F, OH CH₃, OCH₃.

5. A compound according to any one of the preceding claims, wherein R³ is H.

6. A compound according to any one of the preceding claims, wherein X is H, F or CH₃.

7. A compound according to any one of the preceding claims, wherein n is 1.

8. A compound according to any one of the preceding claims, wherein A¹ is R⁶ and A² is H, F or CH₃.

9. A compound according to any one of the preceding claims, wherein R⁶ is -CH₂-Y-T.

10. A compound according to any one of the preceding claims, wherein Y is -O-, -N(R⁹)- or -S(O)₂-.

11. A compound according to any one of the preceding claims, wherein R⁹ is selected from the group consisting of H, CH₃, COOH, COOCH₃, C(O)NH₂, C(O)N(CH₃)₂, and S(O)₂CH₃.

12. A compound according to any one of the preceding claims, wherein T is T¹-T² or T² and wherein T¹ is selected from the group consisting of
-CH₂-;
-C(O)-;
-C(O)-CH₂-;
-C(O)O-;
-C(O)O-CH₂-:
-C(O)NH-;
-C(O)NH-CH₂-;
-S(O)₂-; and
-S(O)₂-CH₂-.

13. A compound according to claim 12, wherein T is T¹-T² or T² and wherein T¹ is selected from the group consisting of -C(O)-; -CH₂-; -S(O)₂-; and -C(O)NH-.

14. A compound according to any one of the preceding claims, wherein R⁶ is -CH₂-N(R³⁶)-T, and wherein R³⁶ is H or S(O)₂CH₃.

15. A compound according to any one of the preceding claims, wherein T² is phenyl or heterocycle.

16. A compound according to claim 1 selected from the group consisting of 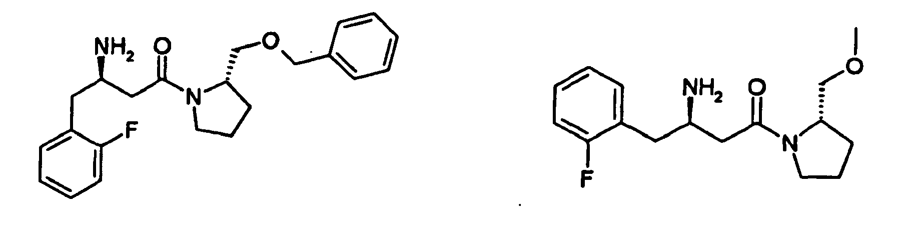 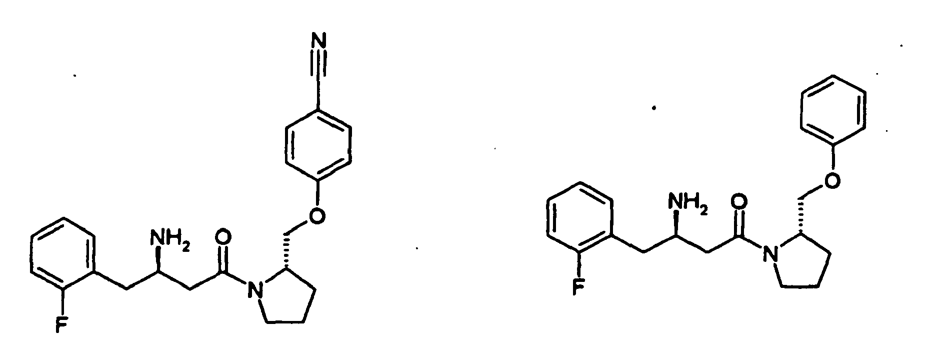 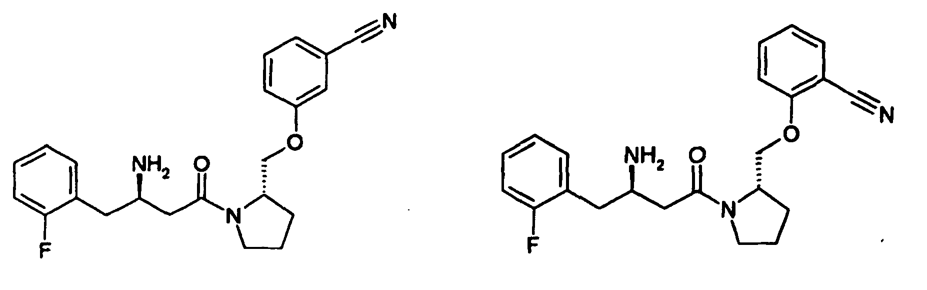 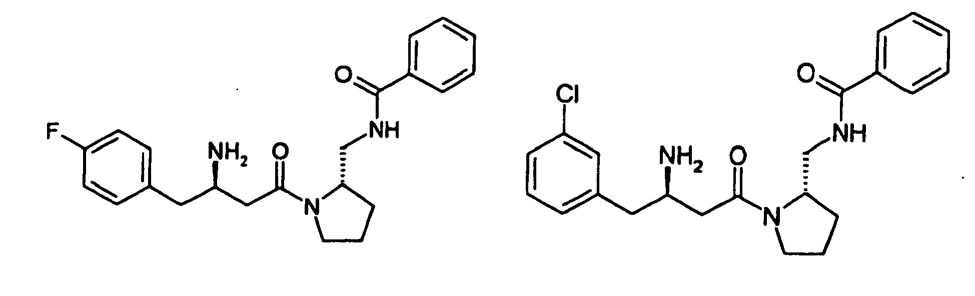 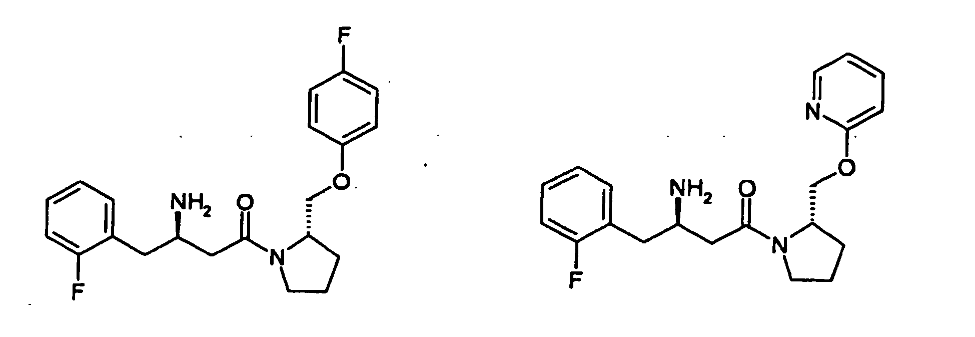 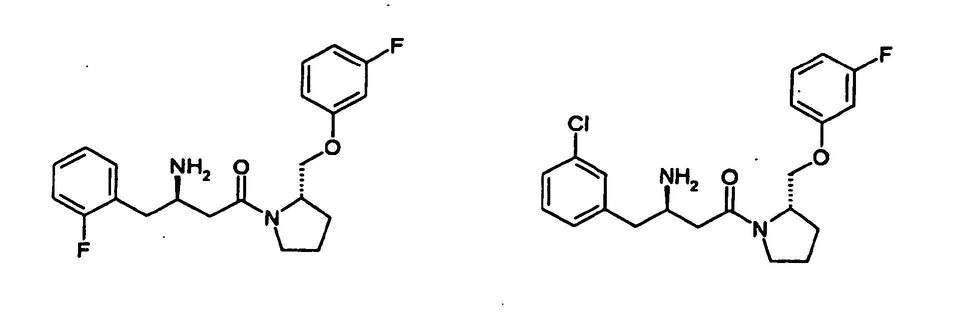 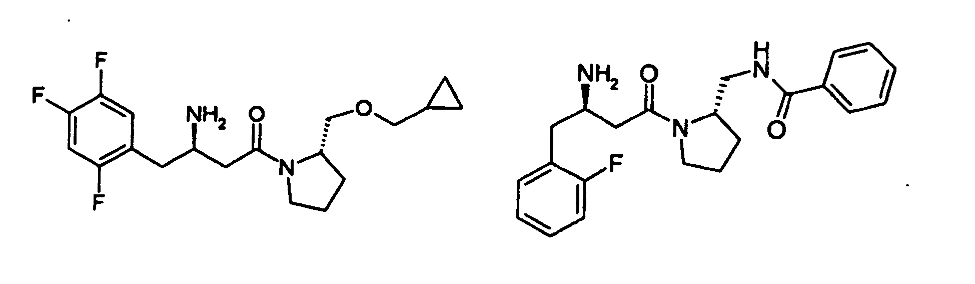 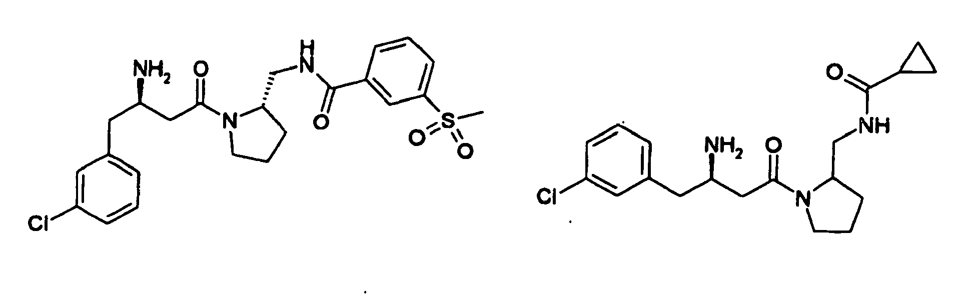 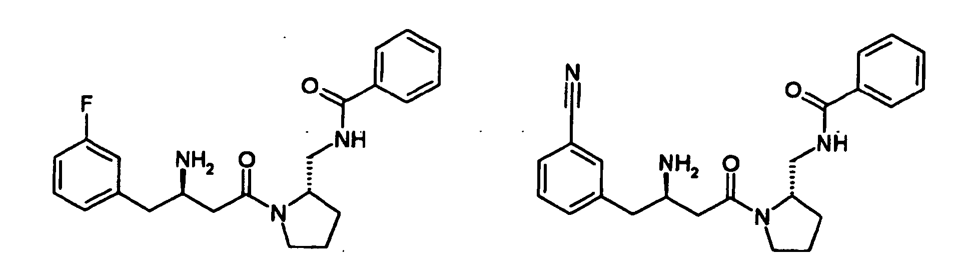 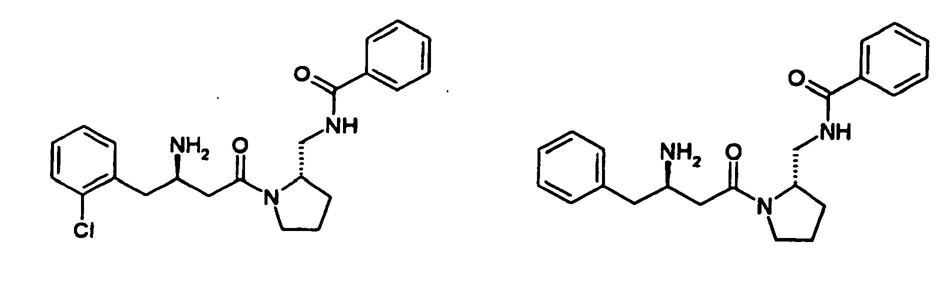 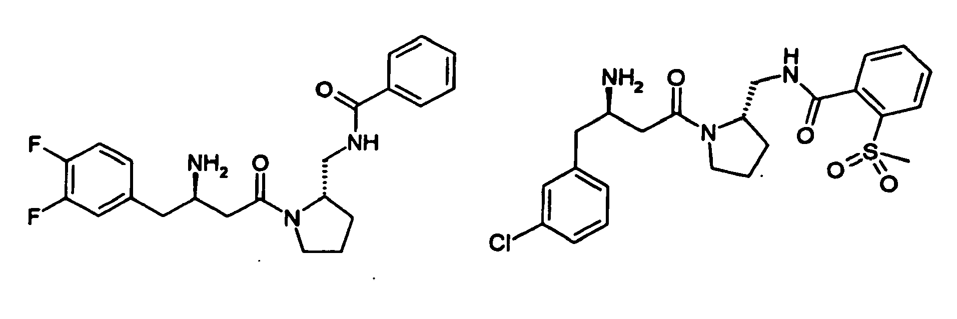 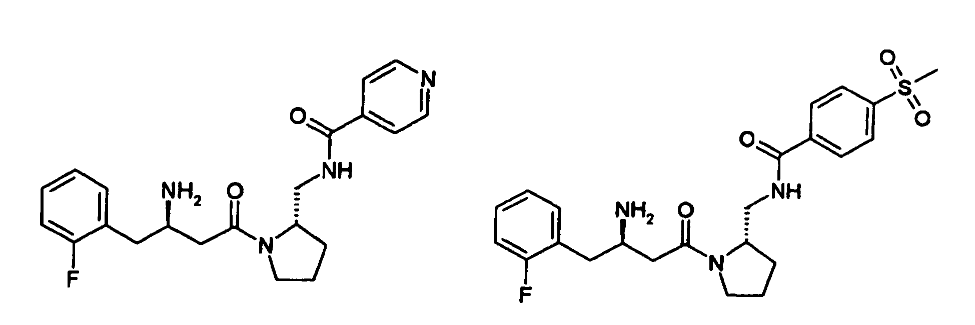 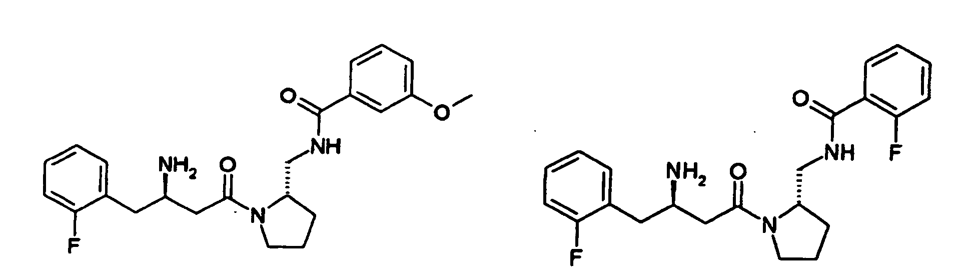 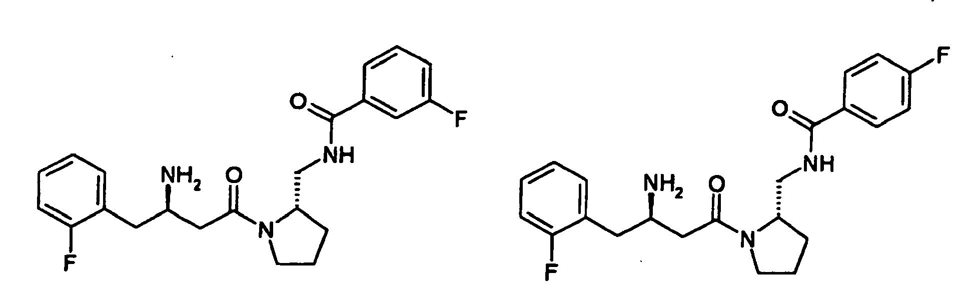 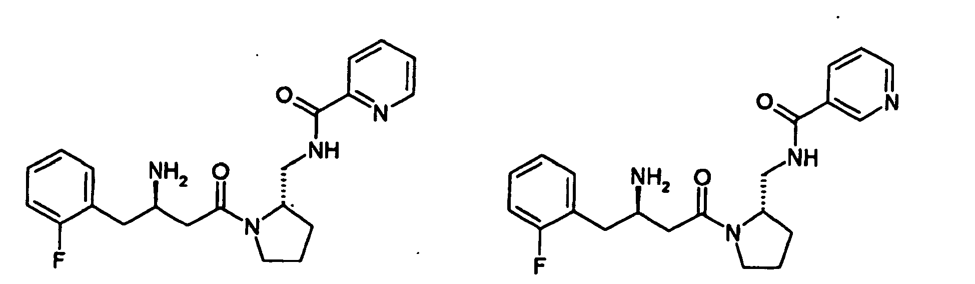 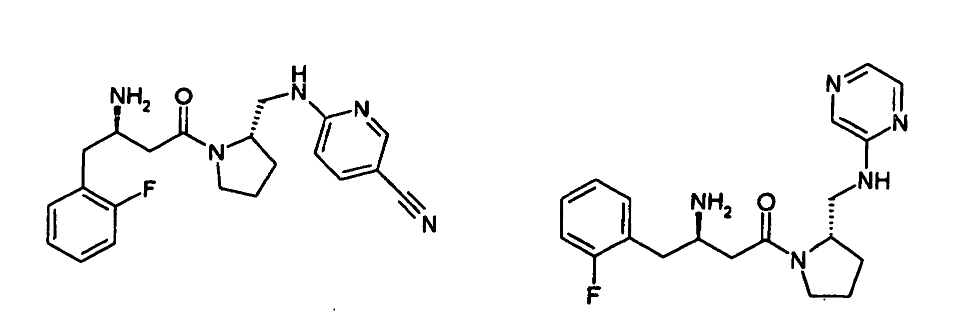 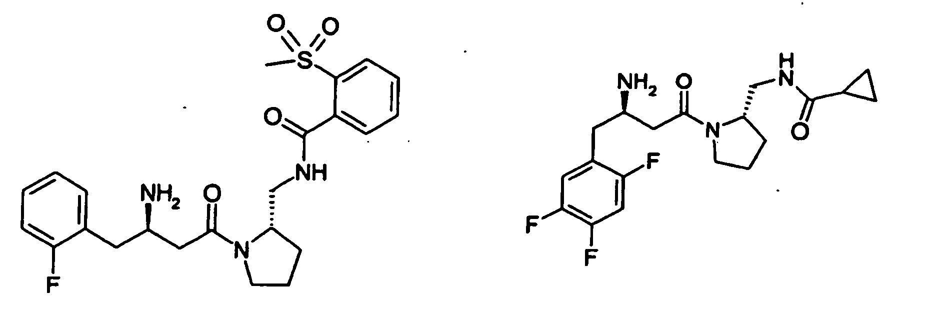 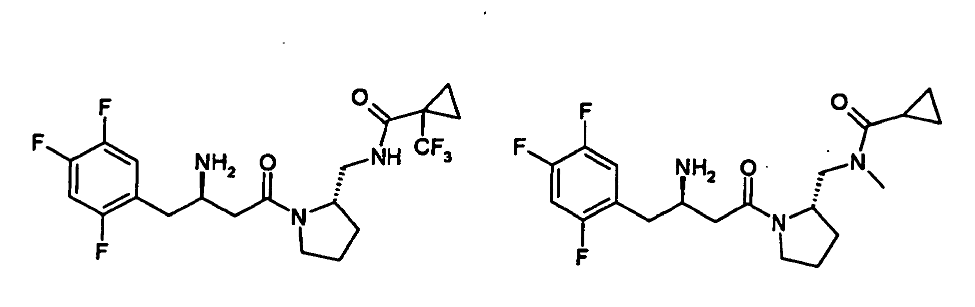 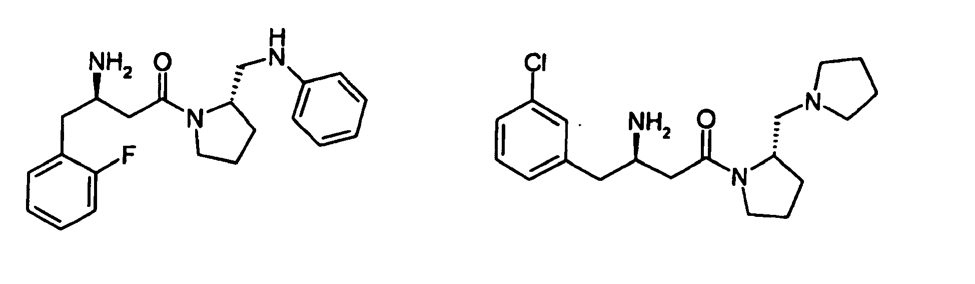 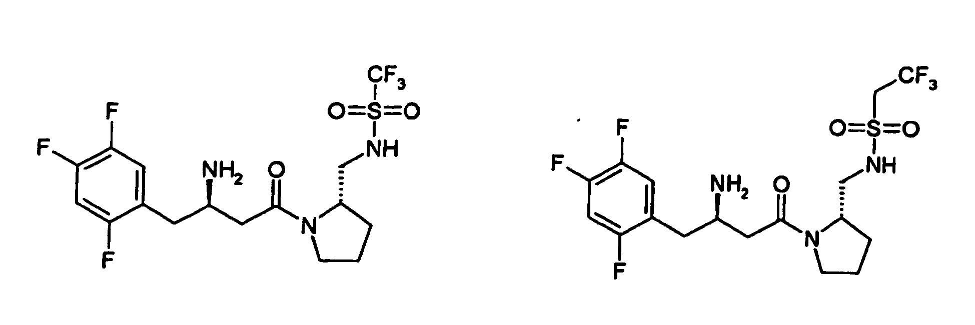 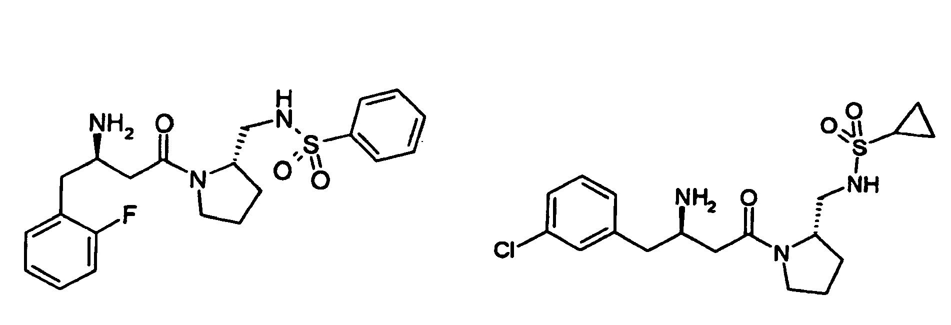 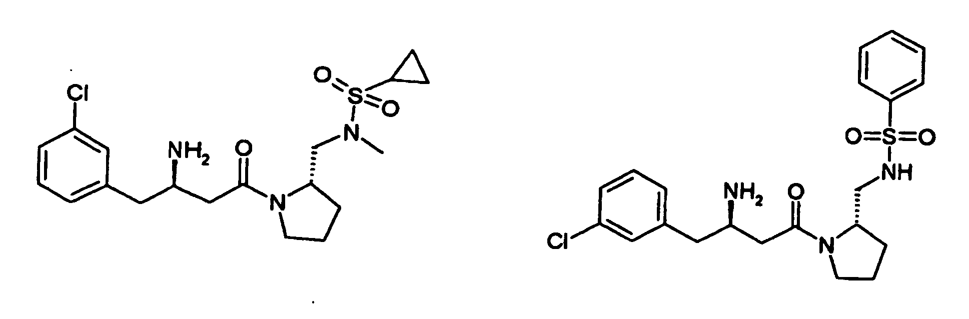 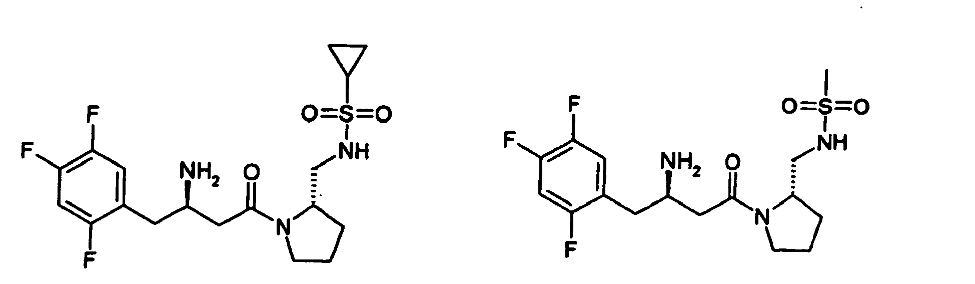 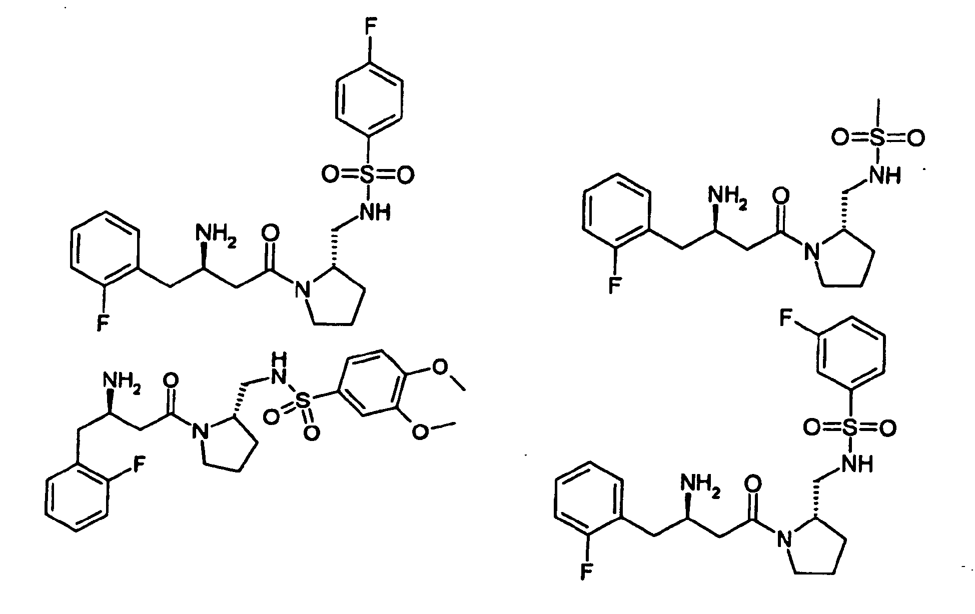 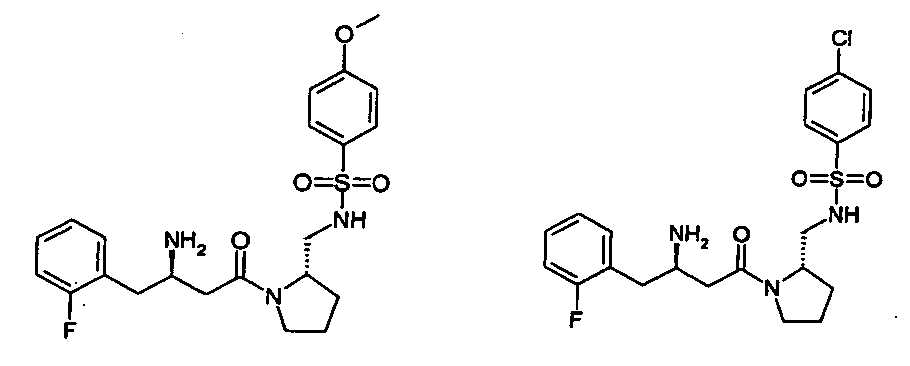 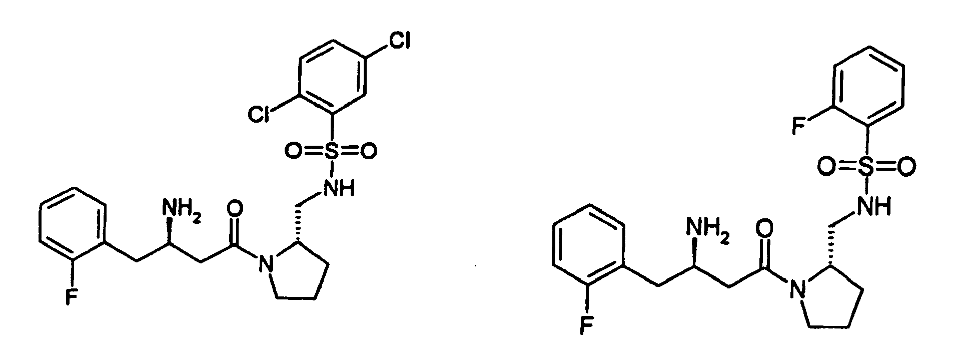 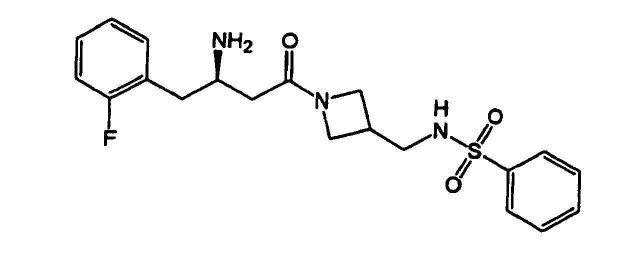 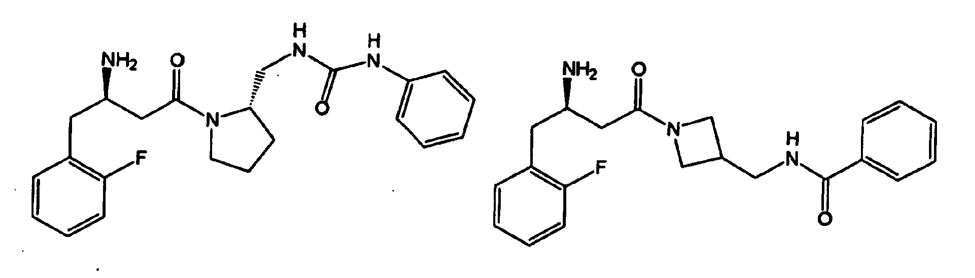 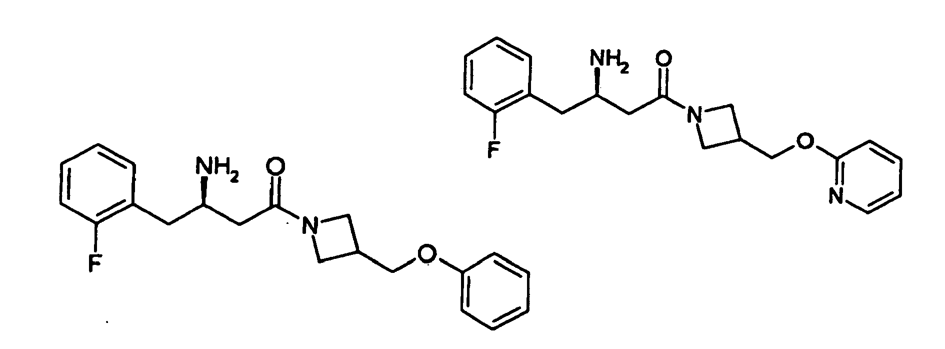 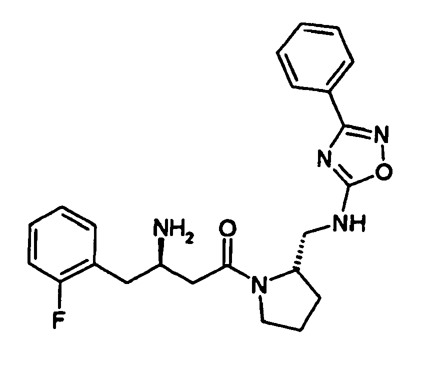 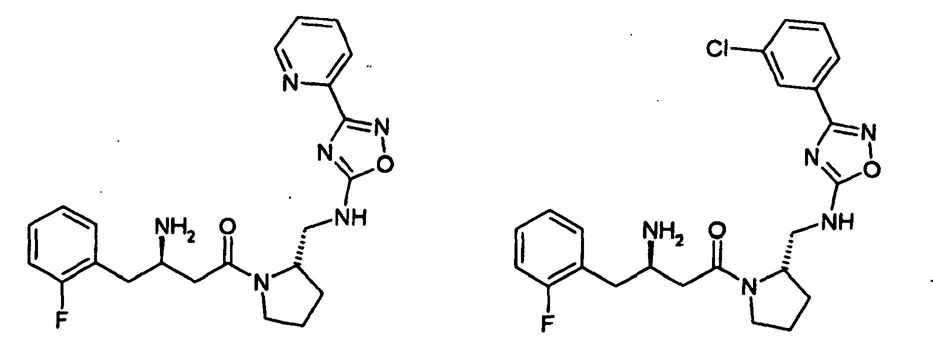 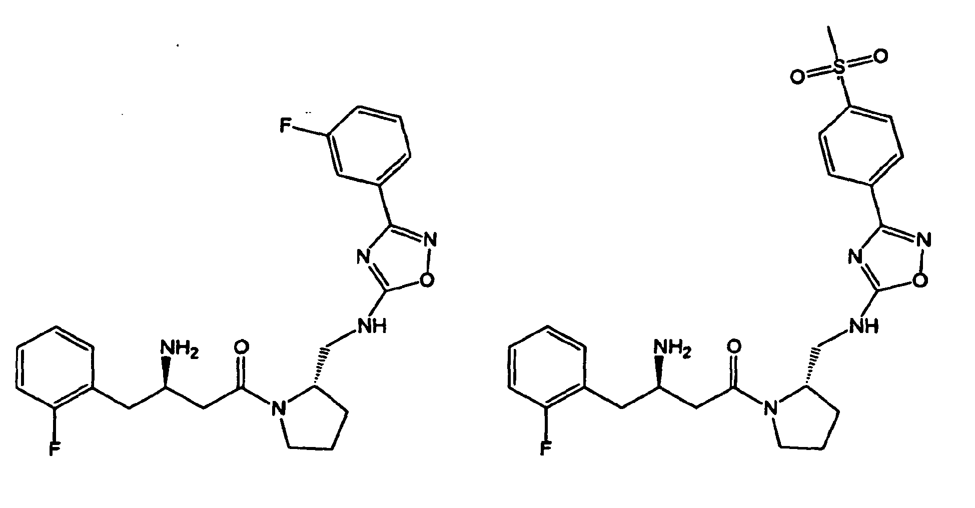 or a pharmaceutically acceptable salt thereof.

17. A prodrug compound of a compound according to any one of the claims 1 to 16 wherein the amino group of formula (I) is acylated, alkylated or phosphorylated.

18. A pharmaceutical composition comprising a compound or a pharmaceutically acceptable salt thereof according to any one of the claims 1 to 17 together with a pharmaceutically acceptable carrier.

19. A pharmaceutical composition according to claim 18, comprising one or more additional compounds or pharmaceutically acceptable salts thereof selected from the group consisting of another compound according to any one of the claims 1 to 17; another DPP-IV inhibitor; insulin sensitizers; PPAR agonists; biguanides; protein tyrosinephosphatase-IB (PTP-1 B) inhibitors; insulin and insulin mimetics; sulfonylureas and other insulin secretagogues; a-glucosidase inhibitors; glucagon receptor antagonists; GLP-1, GLP-1 mimetics, and GLP-1 receptor agonists; GIP, GIP mimetics, and GIP receptor agonists; PACAP, PACAP mimetics, and PACAP receptor 3 agonists; cholesterol lowering agents; HMG-CoA reductase inhibitors; sequestrants; nicotinyl alcohol; nicotinic acid or a salt thereof; PPARa agonists; PPARoly dual agonists; inhibitors of cholesterol absorption; acyl CoA : cholesterol acyltransferase inhibitors; anti-oxidants; PPARo agonists; antiobesity compounds; an ileal bile acid transporter inhibitor, and anti-inflammatory agents.

20. A compound or a pharmaceutically acceptable salt thereof of any one of the claims 1 to 17 for use as a medicament.

21. Use of a compound or a pharmaceutically acceptable salt thereof of any of the claims 1 to 17 for the manufacture of a medicament for the treatment or prophylaxis of non-insulin dependent (Type II) diabetes mellitus; hyperglycemia; obesity; insulin resis-tance; lipid disorders; dyslipidemia; hyperlipidemia; hypertriglyceridemia; hypercholestrerolemia; low HDL; high LDL; atherosclerosis; growth hormone deficiency; diseases related to the immune response; HIV infection; neutropenia; neuronal disorders; anxiety; depression; tumor metastasis; benign prostatic hypertrophy; gingivitis; hypertension; osteoporosis; diseases related to sperm motility; low glucose tolerance; insulin resistance; ist sequelae; vascular restenosis; irritable bowel syndrome; inflammatory bowel disease; including Crohn's disease and ulcerative colitis; other inflammatory conditions; pancreatitis; abdominal obesity; neurodegenerative disease; retinopathy; nephropathy; neuropathy; Syndrome X; ovarian hyperandrogenism (polycystic ovarian syndrome; Type n diabetes; or growth hormone deficiency.

22. Process for the preparation of a compound according to any one of the claims 1 to 17, comprising the steps of
• coupling of an amino-protected beta-amino acid of formula (IVa) 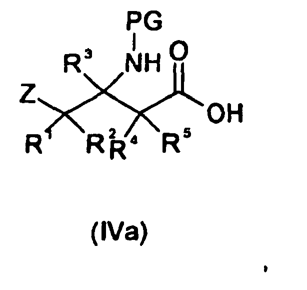 wherein PG is a protective group, with an amine of formula (III) 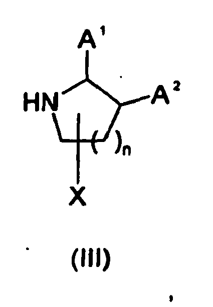 using standard peptide coupling conditions, reagents and protective groups;
• removing the protective group (PG).

23. A process according to claim 22, wherein the coupling reagents are 1-ethyl-3-(3-dimethylaminopropyl)carbodiimide hydrochloride (EDC) in combination with 1-hydroxybenzotriazole (HOBt) and a base (triethylamine or diisopropylethylamine) or 0-(7-azabenzotriazol-1-yl)-*N*,*N*,*N',N*-tetramethyluronium hexafluorophosphate (HATU) in the presence of a base and the protective group is 9-fluorenylmethoxycarbonyl or *tert-*butoxycarbonyl.

24. A process according to claim 22 or 23, wherein the protective group is removed using diethylamine in dichloromethane in the case of 9-fluorenylmethoxycarbonyl or using acidic conditions in the case of *tert*-butoxycarbonyl.

## Patentansprüche

1. Eine Verbindung der Formel (I) 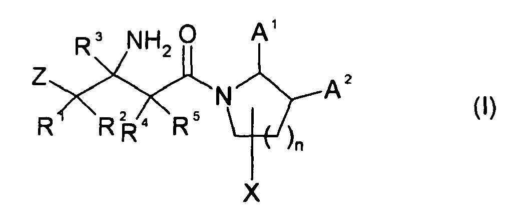 oder ein pharmazeutisch akzeptables Salz davon, worin
Z ausgewählt ist aus der Gruppe bestehend aus
Phenyl;
Naphthyl;
C₃₋₇-Cycloalkyl;
Heterocyclus; und
Heterobicyclus;
worin Z optional substituiert ist mit einem oder, unabhängig voneinander, mehreren aus
Halogen;
CN;
OH;
=O worin der Ring zumindest teilweise gesättigt ist;
C₁₋₆-Alkyl, optional mit einem oder mehreren F substituiert; und
O-C₁₋₆-Alkyl, optional mit einem oder mehreren F substituiert;
R¹, R², R⁴, R⁵ unabhängig voneinander ausgewählt sind aus der Gruppe bestehend aus
H;
F;
OH;
C₁₋₆-Alkyl, optional mit einem oder mehreren F substituiert; und
O-C₁₋₆-Alkyl, optional mit einem oder mehreren F substituiert;
und/oder R¹ und R² optional zusammen ein C₃₋₇Cycloalkyl bilden, welches optional mit einem oder mehreren F substituiert ist;
und/oder R² und R³ optional zusammen ein C₃₋₇-Cycloalkyl bilden, welches optional mit einem oder mehreren F substituiert ist;
und/oder R³ und R⁴ optional zusammen ein C₃₋₇-Cycloalkyl bilden, welches optional mit einem oder mehreren F substituiert ist;
und/oder R⁴ und R⁵ optional zusammen ein C₃₋₇Cycloalkyl bilden, welches optional mit einem oder mehreren F substituiert ist;
R³ H oder C₁₋₆-Alkyl ist;
X ausgewählt ist aus der Gruppe bestehend aus
H;
F; und
C₁₋₆-Alkyl, optional mit einem oder mehreren F substituiert;
n ist 0 oder 1;
A¹, A² unabhängig voneinander ausgewählt sind aus der Gruppe bestehend aus
H;
Halogen;
C₁₋₆-Alkyl, optional mit einem oder mehreren F substituiert; und
R⁶; mit der Maßgabe, dass eines von A¹ und A² R⁶ ist;
R⁶ -C(R⁷R⁸)-Y- T ist;
R⁷, R⁸ unabhängig voneinander ausgewählt sind aus der Gruppe bestehend aus
H;
F; und
C₁₋₆-Alkyl, optional mit einem oder mehreren F substituiert;
und/oder R⁷ und R⁸ optional zusammen ein C₃₋₇Cycloalkyl bilden, welches optional mit einem oder mehreren F substituiert ist;
Y ausgewählt ist aus der Gruppe bestehend aus
-O-;
-C₁₋₆-Alkyl-O-;
-N(R⁹)-;
-C₁₋₆-Alkyl-N(R⁹)-
-S-;
-C₁₋₆-Alkyl-S-;
-S(O)-;
-C₁₋₆-Alkyl-S(O)-;
-S(O)₂-; und
-C₁₋₆-Alkyl-S(O)₂-;
worin jedes C₁₋₆-Alkyl optional mit einem oder mehreren F substituiert ist;
R⁹, T unabhängig voneinander T¹- T² oder T² sind;
T¹ ausgewählt ist aus der Gruppe bestehend aus
-C₁₋₆-Alkyl-;
-C₁₋₆-Alkyl-O-;
-C₁₋₆-Alkyl-N(R¹⁰)-;
-C(O)-;
-C(O)-C₁₋₆-Alkyl-;
-C(O)-C₁₋₆-Alkyl-O-;
-C(O)-C₁₋₆-Alkyl-N(R¹⁰)-,
-C(O)O-;
-C(O)O-C₁₋₆-Alkyl-;
-C(O)O-C₁₋₆-Alkyl-O-;
-C(O)O-C₁₋₆-Alkyl-N(R¹⁰)-;
-C(O)N(R¹⁰)-;
-C(O)N(R¹⁰)-C₁₋₆-Alkyl-;
-C(O)N(R¹⁰)-C₁₋₆-Alkyl-O-;
-C(O)N(R¹⁰)-C₁₋₆-Alkyl-N(R¹¹)-;
-S(O)₂-;
-S(O)₂-C₁₋₆-Alkyl-;
-S(O)₂-C₁₋₆-Alkyl-O-; und
-S(O)₂-C₁₋₆-Alkyl-N(R¹⁰)-;
worin jedes C₁₋₆-Alkyl optional mit einem oder mehreren F substituiert ist;
R¹⁰, R¹¹ unabhängig voneinander H oder C₁₋₆-Alkyl, optional mit einem oder mehreren F substituiert, sind;
T² ausgewählt ist aus der Gruppe bestehend aus
H;
CF₃;
Phenyl;
Naphthyl;
worin Phenyl und Naphthyl optional substituiert sind mit einem oder, unabhängig voneinander, mehreren aus
Halogen;
CN;
R¹²;
COOH;
OH;
C(O)NH₂;
S(0)₂NH₂;
COOT³;
OT³;
C(O)NHT³;
S(O)₂NHT³; oder
T³;
C₃₋₇-Cycloakyl;
Heterocyclus; und
Heterobicyclus;
worin C₃₋₇-Cycloalkyl, Heterocyclus und Heterobicyclus optional mit einem oder, unabhängig voneinander, mehreren aus
Halogen;
CN;
R¹³;
OH;
=O, worin der Ring zumindest teilweise gesättigt ist;
NH₂
COOH;
C(O)NH₂;
S(O)₂NH₂;
COOT³;
OT³;
C(O)NHT³;
S(O)₂NHT³;
NHT³; oder
T³;
worin, wenn R⁹ T¹- T² ist und C₁₋₆-Alkyl darstellt und T T¹ - T² ist und C₁₋₆-Alkyl darstellt, dann können R⁹ und T zusammen eine 3- bis 7-gliedrige zyklische Gruppe enthaltend 1 N bilden;
R¹² ausgewählt ist aus der Gruppe bestehend aus
C₁₋₆-Alkyl;
O-C₁₋₆-Alkyl;
COO-C₁₋₆-Alkyl;
OC(O)-C₁₋₆-Alkyl;
C(O)N(R¹⁵)-C₁₋₆-Alkyl;
S(O)₂N(R¹⁷)-C₁₋₆-Alkyl;
S(O)-C₁₋₆-Alkyl;
S(O)₂-C₁₋₆-Alkyl; und
N(R¹⁸)S(O)₂-C₁₋₆-Alkyl;
worin jedes C₁₋₆-Alkyl optional mit einem oder, unabhängig voneinander, mehreren aus F, COOR¹⁹, C(O)N(R²⁰R²¹), S(O)₂N(R²²R²³), OR²⁴,
N(R²⁵R²⁶), T³, O-T³ oder N(R²⁷)-T³ substituiert ist;
R¹³ ausgewählt ist aus der Gruppe bestehend aus
C₁₋₆-Alkyl;
O-C₁₋₆-Alkyl;
N(R¹⁴)-C₁₋₆-Alkyl;
COO-C₁₋₆-Alkyl;
OC(O)-C₁₋₆-Alkyl;
C(O)N(R¹⁵)-C₁₋₆-Alkyl;
N(R¹⁶)-C(O)-C₁₋₆-Alkyl;
S(O)₂N(R¹⁷)-C₁₋₆-Alkyl;
S(O)-C₁₋₆-Alkyl;
S(O)₂-C₁₋₆-Alkyl; und
-N(R¹⁸)S(O)₂-C₁₋₆-Alkyl;
worin jedes C₁₋₆-Alkyl optional mit einem oder, unabhängig voneinander, mehreren aus F, COOR¹⁹, C(O)N(R²⁰R²¹), S(O)₂N(R²²R²³), OR²⁴, N(R²⁵R²⁶), T³, O-T³ oder N(R²⁷)-T³ substituiert ist;
R¹⁴, R¹⁵, R¹⁶, R¹⁷, R¹⁸, R¹⁹, R²⁰, R²¹, R²², R²³, R²⁴, R²⁵, R²⁶, R²⁷ unabhängig voneinander H oder C₁₋₆-Alkyl sind;
T³ ausgewählt ist aus der Gruppe bestehend aus
Phenyl;
Naphthyl;
worin Phenyl und Naphthyl optional mit einem oder, unabhängig voneinander, mehreren substituiert sind aus
Halogen;
CN;
COOH;
OH;
C(0)NH₂;
S(0)₂NH₂;
C₁₋₆-Alkyl;
O-C₁-₆-Alkyl;
COO-C₁₋₆-Alkyl;
OC(O)-C₁₋₆-Alkyl;
C(O)N(R²⁸)-C₁₋₆-Alkyl;
S(O)₂N(R²⁹)-C₁₋₆-Alkyl;
S(O)₂-C₁₋₆-Alkyl; oder
N(R³⁰)S(O)₂-C₁₋₆-Alkyl;
Heterocyclus;
Heterobicyclus; und
C₃₋₇Cycloalkyl;
worin C₃₋₇-Cycloalkyl, Heterocylus und Heterobicyclus optional substituiert sind mit einem oder mehreren aus
Halogen,
CN;
OH;
=O, worin der Ring zumindest teilweise gesättigt ist;
NH₂
COOH;
C(O)NH₂;
S(O)₂NH₂;
C₁₋₆-Alkyl;
O-C₁₋₆-Alkyl;
N(R³¹)-C₁₋₆-Alkyl;
COO-C₁₋₆-Alkyl;
OC(O)-C₁₋₆-Alkyl;
C(O)N(R³²)-C₁₋₆-Alkyl;
N(R³³)-C(O)-C₁₋₆-Alkyl;
S(O)₂N(R³⁴)-C₁₋₆-Alkyl;
S(O)₂-C₁₋₆-Alkyl; oder
-N(R³⁵)S(O)₂-C₁₋₆-Alkyl.

2. Verbindung gemäß Anspruch 1 nach Formel (la) 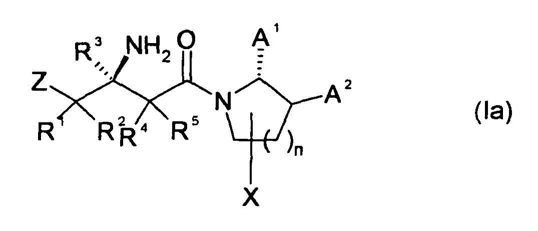 oder ein pharmazeutisch akzeptables Salz davon, worin Z, R¹-R⁵, A¹, A², n und X die in Anspruch 1 beschriebene Bedeutung haben.

3. Verbindung gemäß Anspruch 1 oder 2, worin Z Phenyl oder Heterocyclus ist und Z optional unabhängig voneinander mit bis zu 2 aus Cl, F, CN, CH₃ oder OCH₃ substituiert ist.

4. Verbindung gemäß einem der vorangehenden Ansprüche, worin R¹, R², R⁴, R⁵ unabhängig voneinander ausgewählt sind aus der Gruppe bestehend aus H, F, OH, CH₃, OCH₃.

5. Verbindung gemäß einem der vorangehenden Ansprüche, worin R³ H ist.

6. Verbindung gemäß einem der vorangehenden Ansprüche, worin X H, F oder CH₃ ist.

7. Verbindung gemäß einem der vorangehenden Ansprüche, worin n 1 ist.

8. Verbindung gemäß einem der vorangehenden Ansprüche, worin A¹ R⁶ ist und A² H, F oder CH₃ ist.

9. Verbindung gemäß einem der vorangehenden Ansprüche, worin R⁶ -CH₂-X-T ist.

10. Verbindung gemäß einem der vorangehenden Ansprüche, worin Y -O-, -N(R⁹)- oder -S(O)₂- ist.

11. Verbindung gemäß einem der vorangehenden Ansprüche, worin R⁹ ausgewählt ist aus der Gruppe bestehend aus H, CH₃, COOH, COOCH₃, C(O)NH₂, C(O)N(CH₃)₂ und S(O)₂CH₃ .

12. Verbindung gemäß einem der vorangehenden Ansprüche, worin T T¹-T² oder T² ist und worin T¹ ausgewählt ist aus der Gruppe bestehend aus
-CH₂-;
-C(O)-;
-C(O)-CH₂-;
-C(O)O-;
-C(O)O-CH₂-;
-C(O)NH-;
-C(O)NH-CH₂-;
-S(O)₂-; und
-S(O)₂-CH₂-.

13. Verbindung gemäß Anspruch 12, worin T T¹-T² oder T² ist und worin T¹ ausgewählt ist aus der Gruppe bestehend aus -C(O)-; -CH₂-; -S(O)₂-; and -C(O)NH-.

14. Verbindung gemäß einem der vorangehenden Ansprüche, worin R⁶-CH₂-N(R³⁶)-T ist und worin R³⁶ H oder S(O)₂CH₃ ist.

15. Verbindung gemäß einem der vorangehenden Ansprüche, worin T² Phenyl oder Heterocyclus ist.

16. Verbindung gemäß Anspruch 1 ausgewählt aus der Gruppe bestehend aus 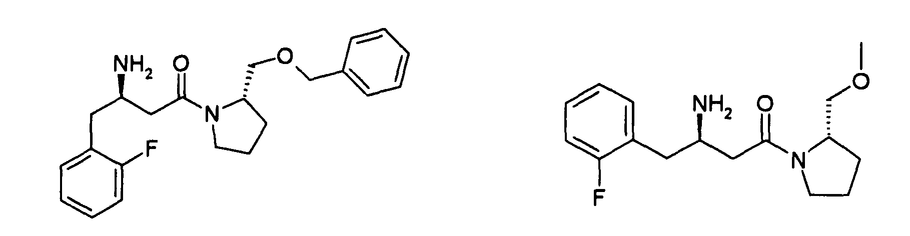 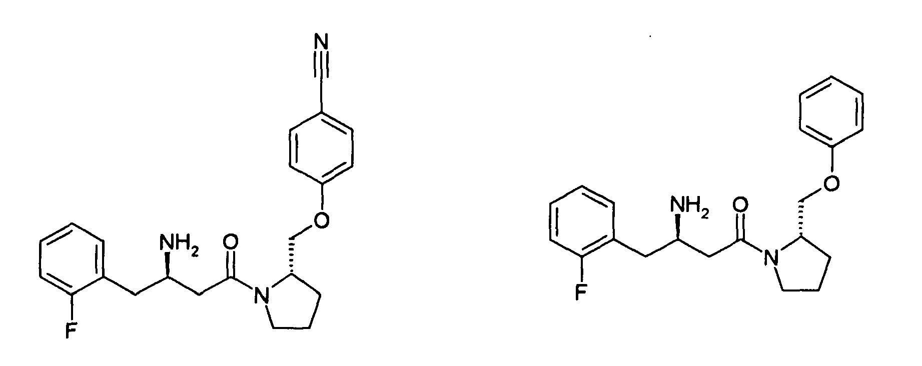 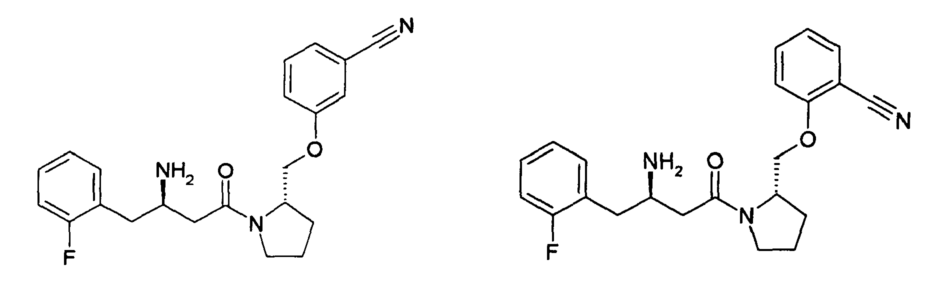 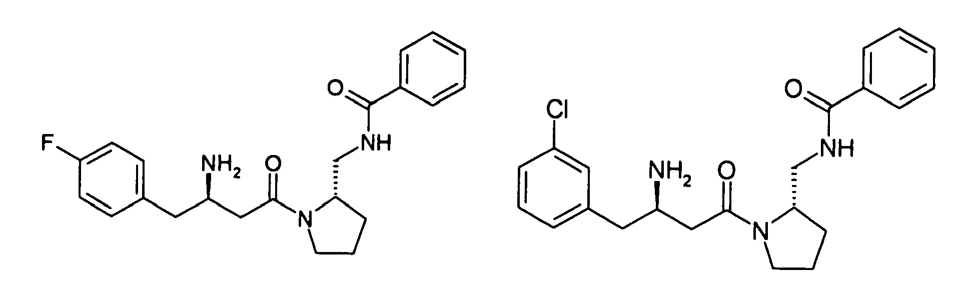 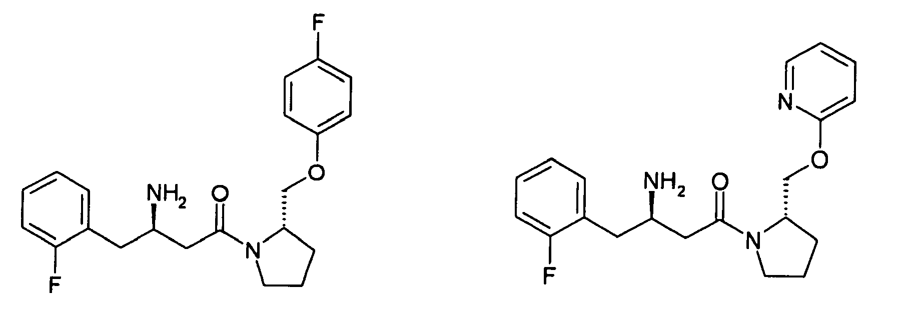 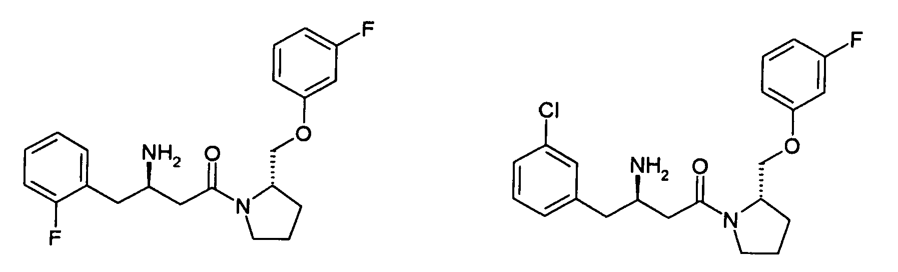 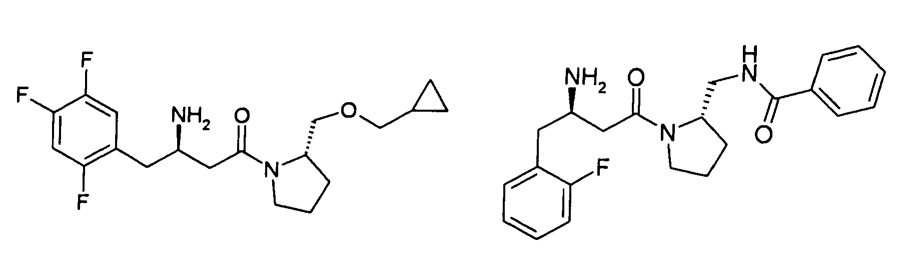 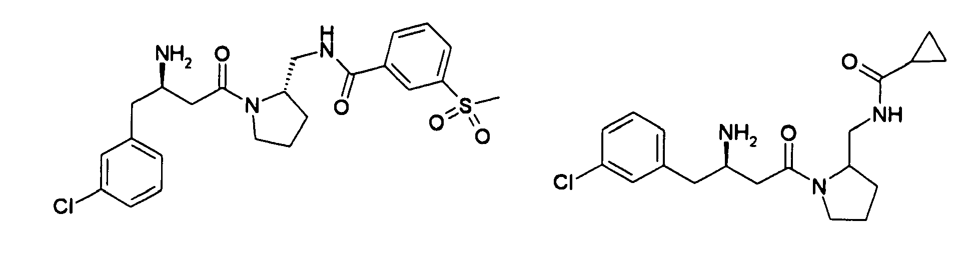 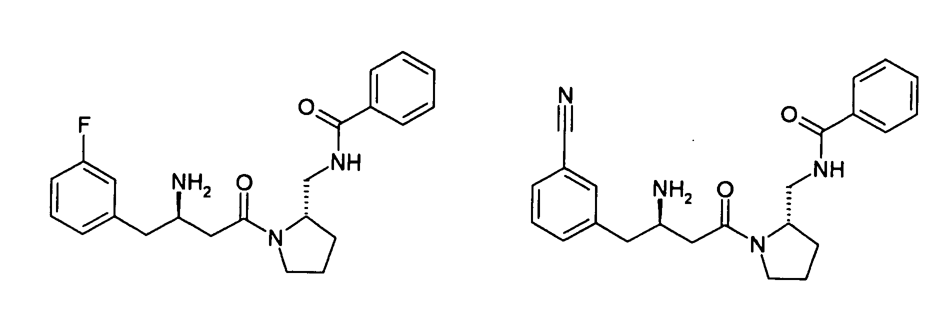 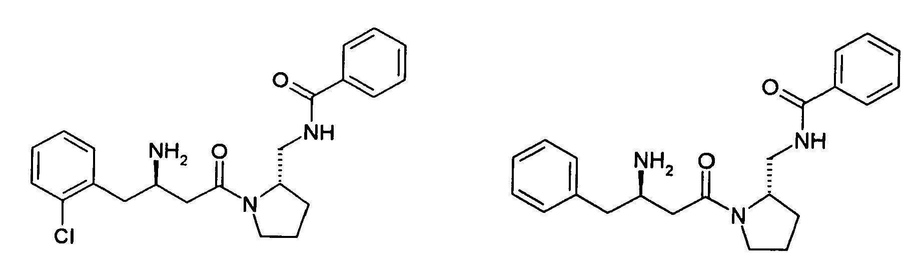 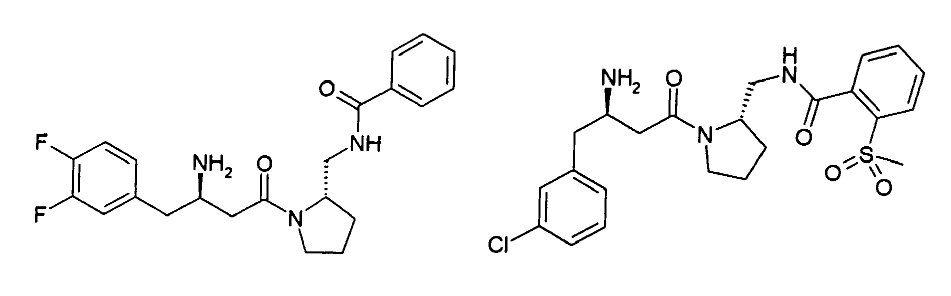 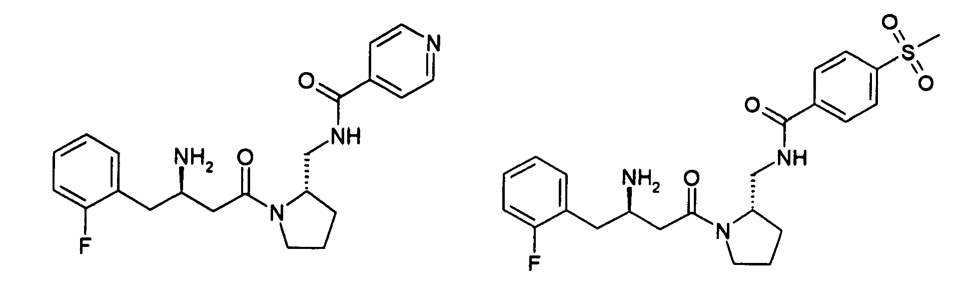 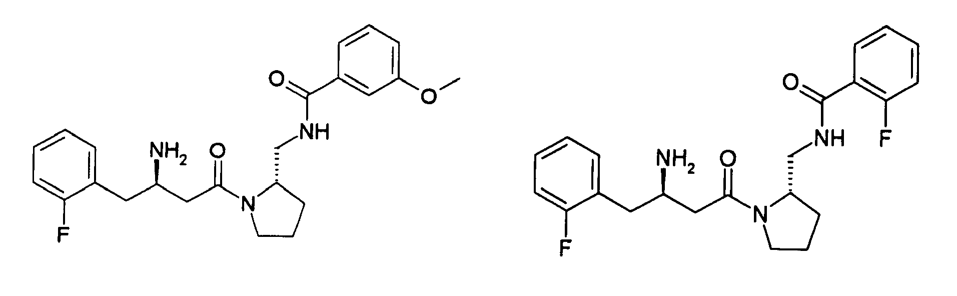 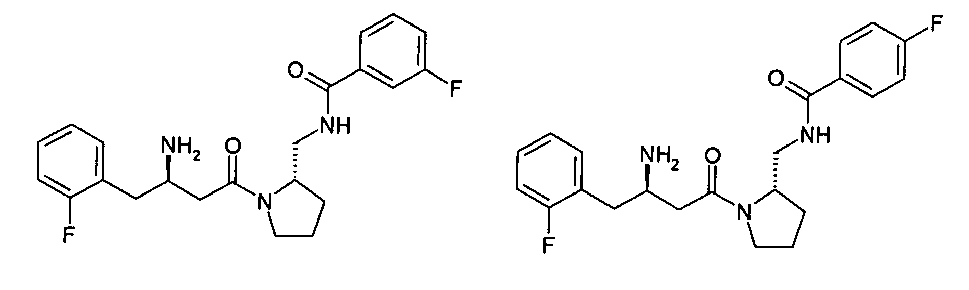 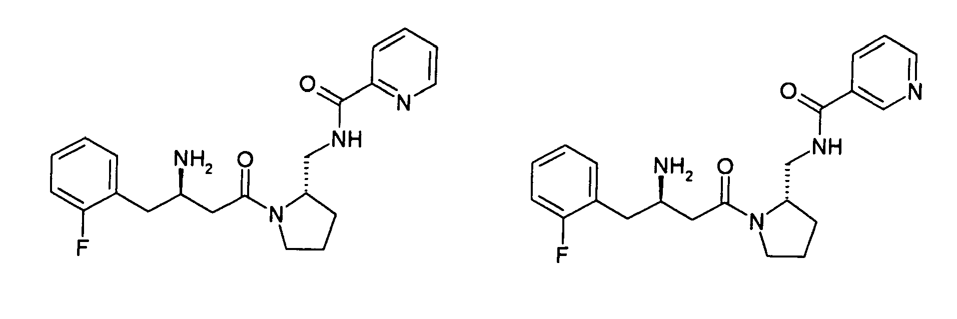 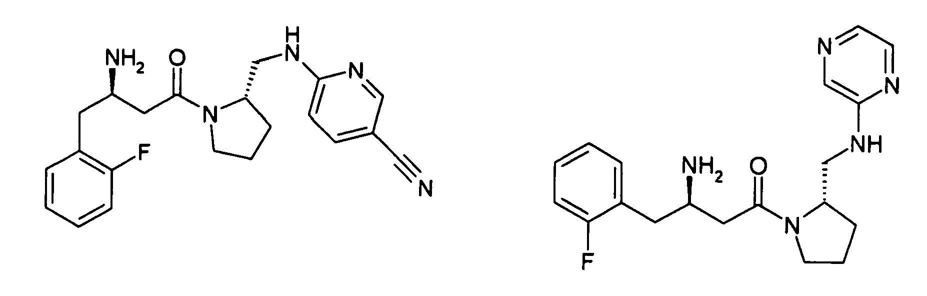 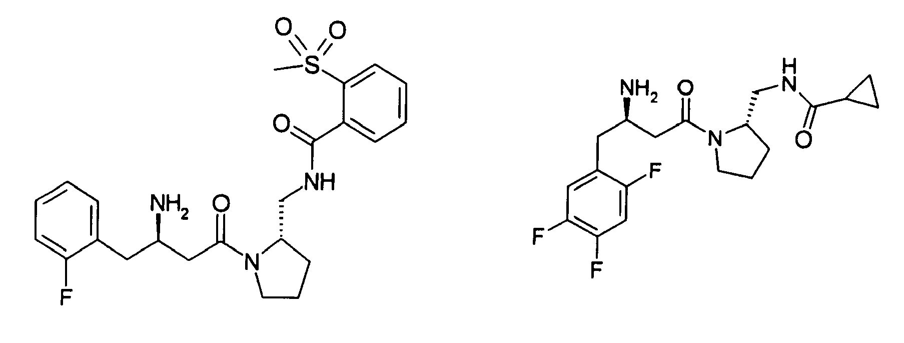 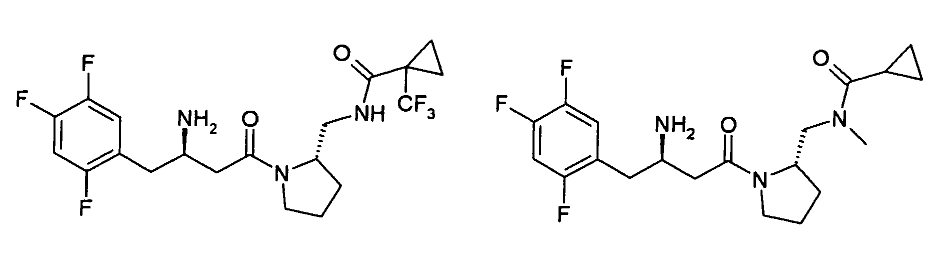 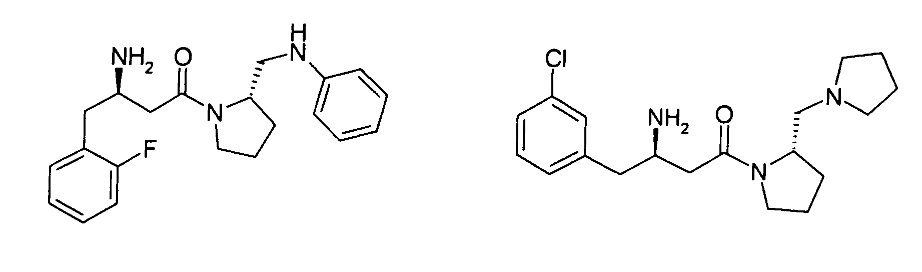 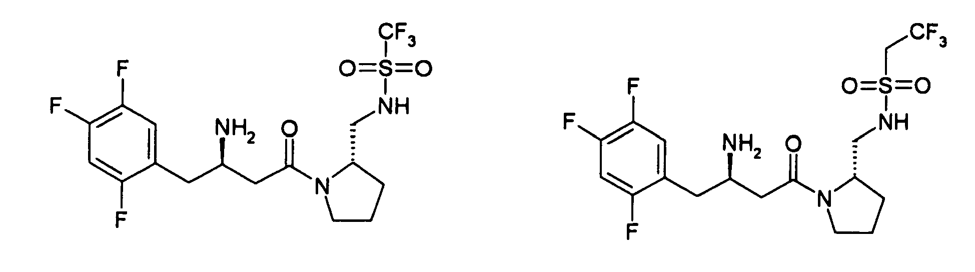 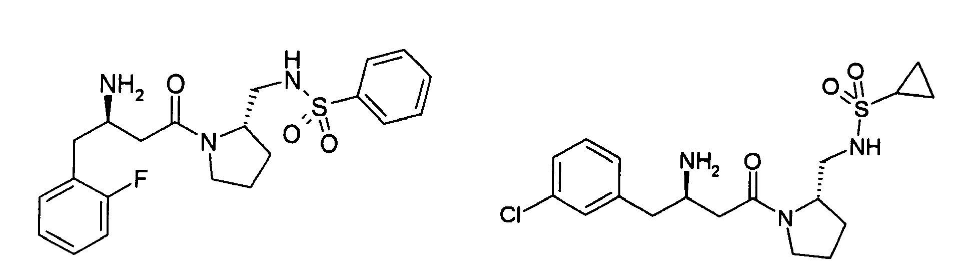 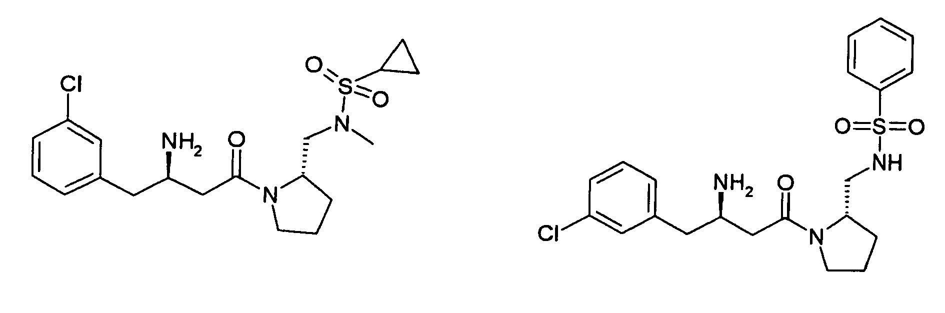 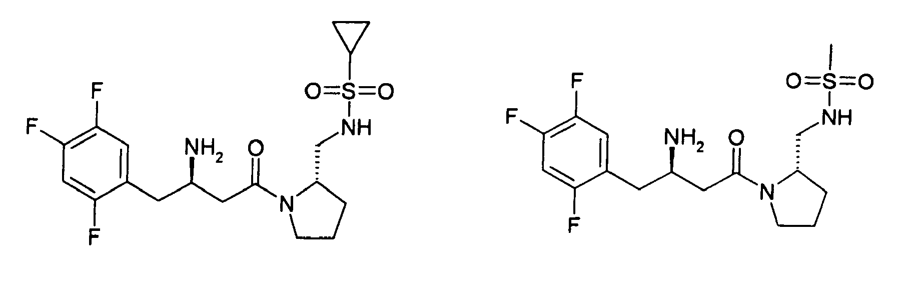 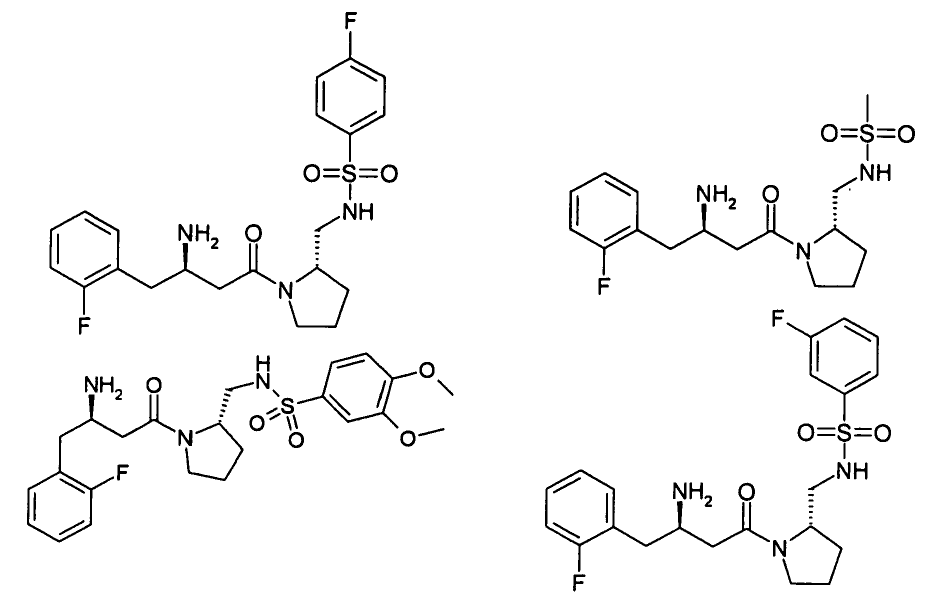 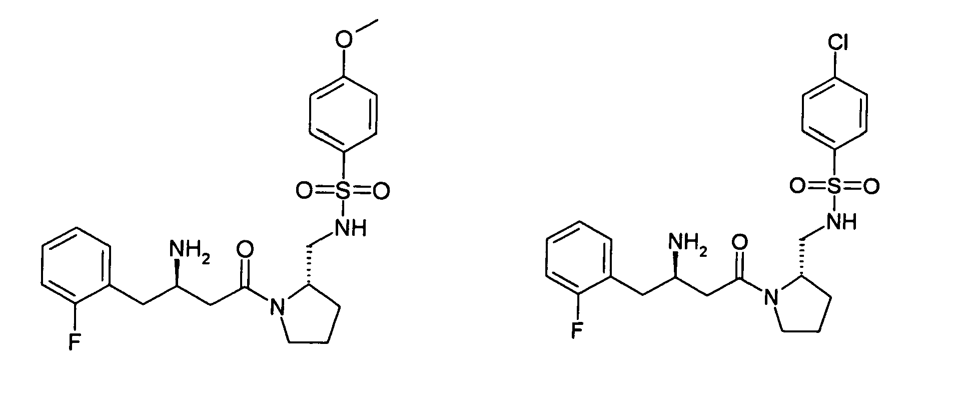 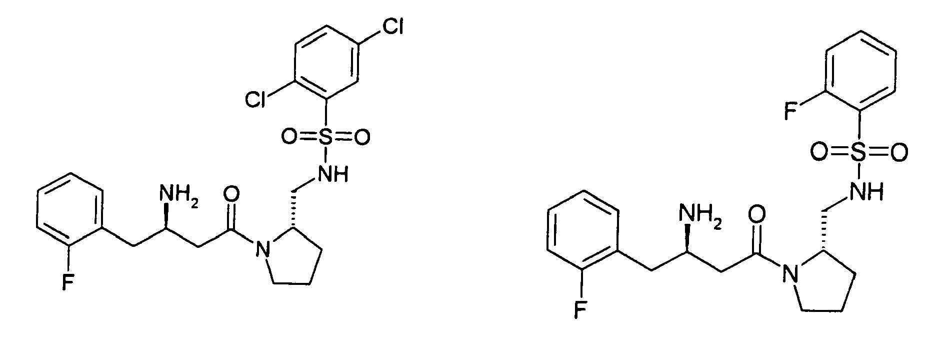 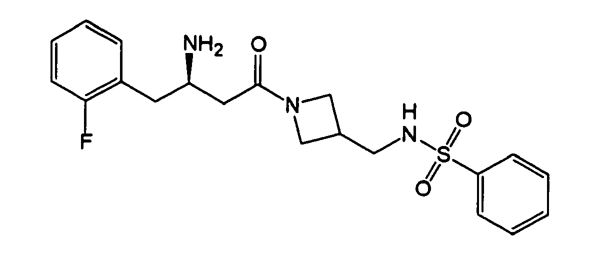 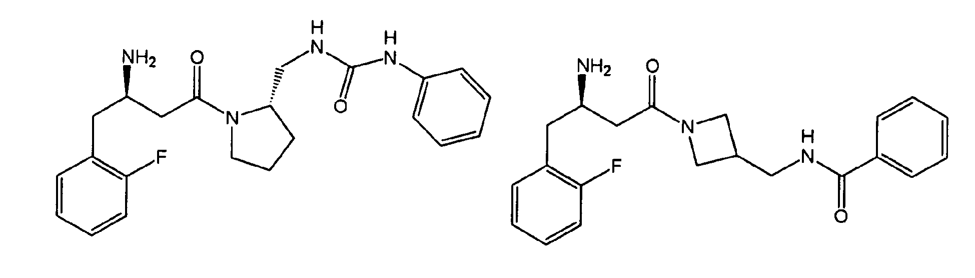 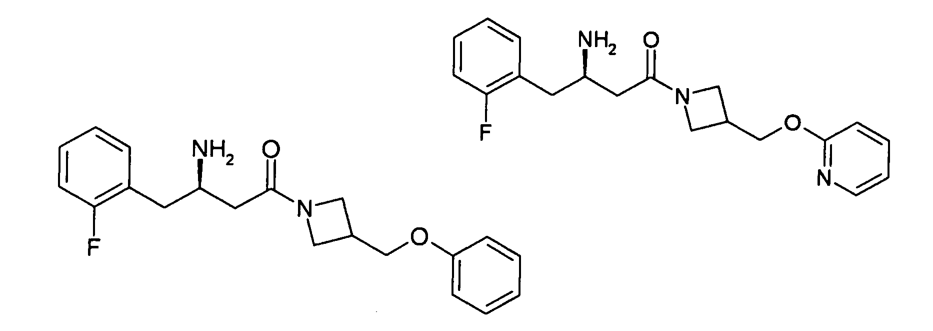 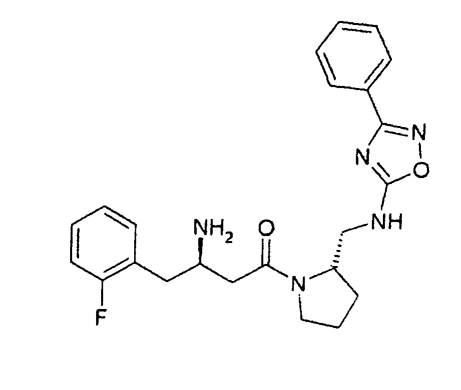 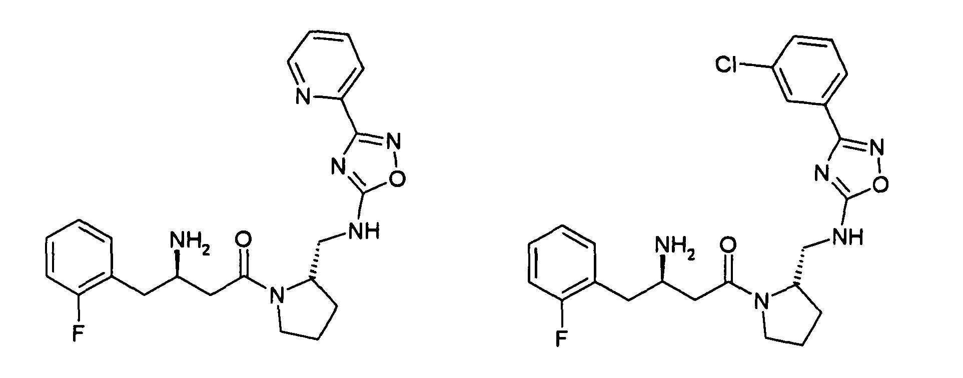 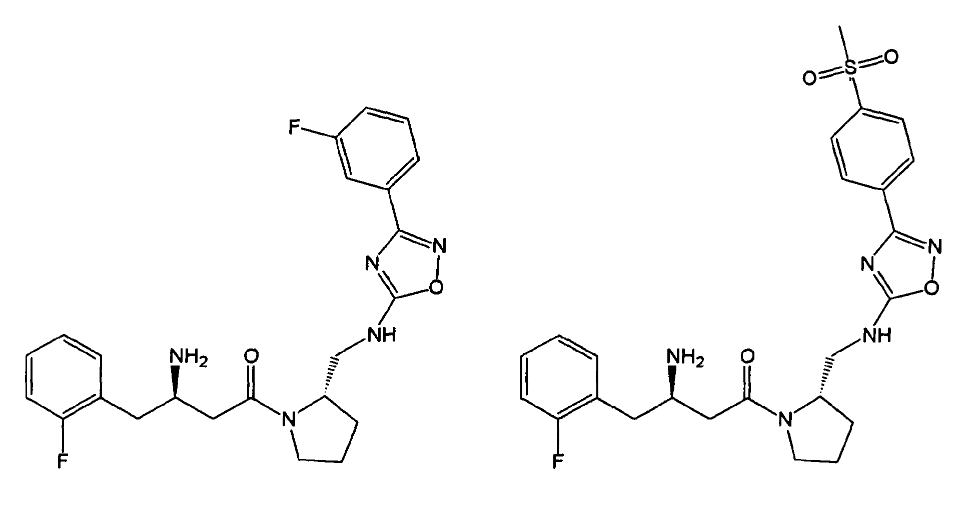 oder ein pharmazeutisch akzeptables Salz davon.

17. Eine Prodrug-Verbindung einer Verbindung gemäß einem der Ansprüche 1 bis 16, worin die Aminogruppe der Formel (I) acyliert, alkyliert oder phosphoryliert ist.

18. Eine pharmazeutische Zusammensetzung umfassend eine Verbindung oder ein pharmazeutisch akzeptables Salz davon gemäß einem der Ansprüche 1 bis 17 zusammen mit einem pharmazeutisch akzeptablen Träger.

19. Pharmazeutische Zusammensetzung gemäß Anspruch 18, umfassend eine oder mehrere zusätzliche Verbindungen oder pharmazeutisch akzeptable Salze davon, ausgewählt aus der Gruppe bestehend aus einer weiteren Verbindung gemäß einem der Ansprüche 1 bis 17; einem weiteren DPP-IV-Inhibitor; Insulin-Sensibilisatoren; PPAR-Agonisten; Biguanide; Protein-Tyrosinphosphatase-IB (PTP-1 B)-Inhibitoren; Insulin und Insulin-Mimetika; Sulfonylharnstoffe und weitere Insulin-Sekretationsstimulanzien; a-Glucosidase-Inhibitoren; Glukagon-Rezeptor-Antagonisten; GLP-1, GLP-1-Mimetika und GLP-1-Rezeptor-Agonisten; GIP, GIP-Mimetika und GIP-Rezeptor-Agonisten; PACAP, PACAP-Mimetika und PACAP-Rezeptor-3-Agonisten; Cholesterin-senkende Mittel; HMG-CoA-Reduktase-Inhibitoren; Komplexbildner; Nikotinalkohol; Nikotinsäure oder ein Salz davon; PPARa-Agonisten; PPARoly-Dualagonisten; Inhibitoren der Cholesterinabsorption; Acyl-CoA : Cholesterinacyltransferaseinhibitoren; Antioxidantien; PPARo-Agonisten; Verbindungen gegen Fettleibigkeit; ein Inhibitor des Ileal-Gallensäuretransporters; und antientzündliche Mittel.

20. Verbindung oder ein pharmazeutisch akzeptables Salz davon gemäß einem der Ansprüche 1 bis 17 zur Verwendung als ein Arzneimittel.

21. Verwendung einer Verbindung oder eines pharmazeutisch akzeptablen Salzes davon nach einem der Ansprüche 1 bis 17 für die Herstellung eines Arzneimittels zur Behandlung oder Prophylaxe von nicht-Insulin-abhängigen (Typ II) Diabetes mellitus; Hyperglykämie; Fettleibigkeit; Insulinresistenz; Lipid-Störungen; Dyslipidämie; Hyperlipidämie, Hypertriglyceridämie; Hypercholesterinämie; niedriges HDL; hohes LDL; Artherosklerose; Wachstumshormondefizienz; Krankheiten verbunden mit einer Immunantwort; HIV-Infektion; Neutropenie; neuronale Krankheiten; Angstzustände; Depression; Tumormetastasen; gutartige Prostata-Hypertrophy; Gingivitis; Bluthochdruck; Osteoporose; Krankheiten verbunden mit Spermabeweglichkeit; niedrige Glukose-Toleranz; Insulinresistenz; ist Sequelae; vaskuläre Restinose; Reizdarm-Syndrom; entzündliche Darmkrankheit; beinhaltend Crohn's Krankheit und Dickdarmentzündung; weitere entzündliche Bedingungen; Pankreatitis; Bierbauch; neurodegenerative Krankheiten; Retinopathy; Nephropathy; Neuropathy; Syndrom X; polycystisches Ovarien-Syndrom; Typ n Diabetes; oder Wachstumshormon-Defizienz.

22. Verfahren zur Herstellung einer Verbindung gemäß einem der Ansprüche 1 bis 17, umfassend die Schritte von
• Kuppeln einer aminogeschützten Beta-Aminosäure der Formel (IVa) 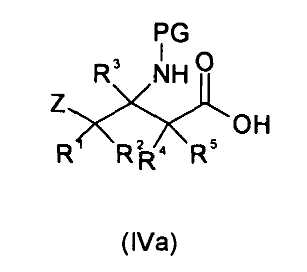 worin PG eine Schutzgruppe ist, mit einem Amin der Formel (III) 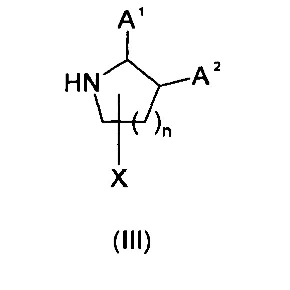 unter Verwendung von Standard-Peptid-Kupplungsbedingungen, Reagenzien und Schutzgruppen;
• Entfernen der Schutzgruppe (PG).

23. Verfahren gemäß Anspruch 22, worin die Kupplungsreagenzien 1-Ethyl-3-(3-dimethylaminopropyl)carbodiimidhydrochlorid (EDC) in Kombination mit 1-Hydroxybenzotriazol (HOBt) und einer Base (Triethylamin oder Diisopropylethylamin) oder O-(7-azabenzotriazol-1-yl)-*N*,*N*,*N',N'*-tetramethyluroniumhexafluorphosphat (HATU) sind in der Gegenwart einer Base und in die Schutzgruppe 9-Fluorenylmethoxycarbonyl oder *tert*-Butoxycarbonyl ist.

24. Verfahren gemäß Anspruch 22 oder 23, worin die Schutzgruppe unter Verwendung von Diethylamin in Dichlormethan im Falle von 9-Fluorenylmethoxycarbonyl entfernt wird oder unter Verwendung von sauren Bedingungen im Falle von *tert-*Butoxycarbonyl.

## Revendications

1. Composé de formule (I) 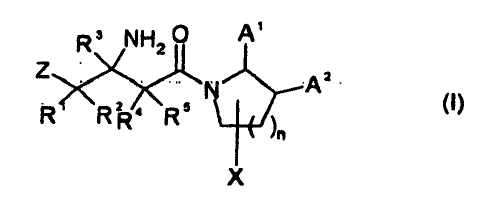 ou sel pharmaceutiquement acceptable de celui-ci, dans lequel
Z est choisi dans le groupe constitué par
phényle ;
naphtyle ;
cycloalkyle en C₃₋₇ ;
hétérocycle ; et
hétérobicycle ;
où Z est facultativement substitué par un ou, indépendamment les uns des autres, plusieurs parmi
halogène;
CN ;
OH ;
=O, où le cycle est au moins partiellement saturé ;
alkyle en C₁₋₆, facultativement substitué par un ou plusieurs F ; et
O-alkyle en C₁₋₆, facultativement substitué par un ou plusieurs F ;
R¹, R², R⁴, R⁵ sont, indépendamment les uns des autres, choisis dans le groupe constitué par
H ;
F ;
OH ;
alkyle en C₁₋₆, facultativement substitué par un ou plusieurs F ; et
O-alkyle en C₁₋₆, facultativement substitué par un ou plusieurs F ;
et/ou R¹ et R² forment facultativement ensemble un cycloalkyle en C₃₋₇, qui est facultativement substitué par un ou plusieurs F ;
et/ou R² et R³ forment facultativement ensemble un cycloalkyle en C₃₋₇, qui est facultativement substitué par un ou plusieurs F ;
et/ou R³ et R⁴ forment facultativement ensemble un cycloalkyle en C₃₋₇, qui est facultativement substitué par un ou plusieurs F ;
et/ou R⁴ et R⁵ forment facultativement ensemble un cycloalkyle en C₃₋₇, qui est facultativement substitué par un ou plusieurs F ;
R³ est H ou alkyle en C₁₋₆ ;
X est choisi dans le groupe constitué par
H ;
F ; et
alkyle en C₁₋₆, facultativement substitué par un ou plusieurs F ;
n est 0 ou 1 ;
A¹, A² sont, indépendamment l'un de l'autre, choisis dans le groupe constitué par
H ;
halogène ;
alkyle en C₁₋₆, facultativement substitué par un ou plusieurs F ; et
R⁶ ; étant entendu que l'un de A¹ et A² est R⁶ ;
R⁶ est -C(R⁷R⁸)-Y-T ;
R⁷, R⁸ sont, indépendamment l'un de l'autre, choisis
dans le groupe constitué par
H ;
F ; et
alkyle en C₁₋₆, facultativement substitué par un ou plusieurs F ;
et/ou R⁷ et R⁸ forment facultativement ensemble un cycloalkyle en C₃₋₇, qui est facultativement substitué par un ou plusieurs F ;
Y est choisi dans le groupe constitué par
-O- ;
-alkyl en C₁₋₆-O- ;
-N(R⁹)- ;
-alkyl en C₁₋₆-N (R⁹)-
-S- ;
-alkyl en C₁₋₆-S- ;
-S (O) - ;
-alkyl en C₁₋₆-S(O)- ;
-S(O)₂- ; et
-alkyl en C₁₋₆-S(O)₂- ;
où chaque alkyle en C₁₋₆ est facultativement substitué par un ou plusieurs F ;
R⁹, T sont, indépendamment l'un de l'autre, T¹-T² ou
T² ;
T¹ est choisi dans le groupe constitué par
-alkyl en C₁₋₆- ;
-alkyl en C₁₋₆-O-
-alkyl en C₁₋₆-N(R¹⁰)-
-C (O) - ;
-C (O) -alkyl en C₁₋₆- ;
-C (O) -alkyl en C₁₋₆-O- ;
-C (O) -alkyl en C₁₋₆-N(R¹⁰)- ;
-C(O)O- ;
-C (O) O-alkyl en C₁₋₆- ;
-C (O) O-alkyl en C₁₋₆-O- ;
-C (O) O-alkyl en C₁₋₆-N(R¹⁰)- ;
-C(O)N(R¹⁰)- ;
-C(O)N(R¹⁰)-alkyl en C₁₋₆- ;
-C (O) N (R¹⁰) -alkyl en C₁₋₆-O- ;
-C (O) N (R¹⁰) -alkyl en C₁₋₆-N(R¹¹)- ;
-S (O) ₂- ;
-S(O)₂-alkyl en C₁₋₆- ;
-S(O)₂-alkyl en C₁₋₆-O- ; et
-S(O)₂-alkyl en C₁₋₆N(R¹⁰)- ;
où chaque alkyle en C₁₋₆ est facultativement substitué par un ou plusieurs F ;
R¹⁰, R¹¹ sont, indépendamment l'un de l'autre, H ou alkyle en C₁₋₆, facultativement substitué par un ou plusieurs F ;
T² est choisi dans le groupe constitué par
H ;
CF₃ ;
phényle ;
naphtyle ;
où phényle et naphtyle sont facultativement substitués par un ou, indépendamment les uns des autres, plusieurs parmi
halogène ;
CN ;
R¹² ;
COOH ;
OH ;
C(O)NH₂ ;
S(O)₂NH₂ ;
COOT³ ;
OT³ ;
C(O)NHT³ ;
S(O)₂NHT³ ; ou
T³ ;
cycloalkyle en C₃₋₇ ;
hétérocycle ; et
hétérobicycle ;
où cycloalkyle en C₃₋₇, hétérocycle et hétérobicycle sont facultativement substitués par un ou, indépendamment les uns des autres, plusieurs parmi
halogène ;
CN ;
R¹³ ;
OH ;
=O, où le cycle est au moins partiellement saturé ;
NH₂
COOH ;
C(O)NH₂ ;
S(O)₂NH₂ ;
COOT³ ;
OT³ ;
C(O)NHT³;
S(O)₂NHT³ ;
NHT³ ; ou
T³ ;
de sorte que lorsque R⁹ est T¹-T² et représente -alkyle en C₁₋₆ et T est T¹-T² et représente -alkyle en C₁₋₆, alors R⁹ et T peuvent former ensemble un groupe cyclique à 3 à 7 chaînons contenant 1 N ;
R¹² est choisi dans le groupe constitué par
alkyle en C₁₋₆ ;
O-alkyle en C₁₋₆ ;
COO-alkyle en C₁₋₆ ;
OC (O) -alkyle en C₁₋₆ ;
C(O)N(R¹⁵)-alkyle en C₁₋₆ ;
S (O)₂N(R¹⁷)-alkyle en C₁₋₆ ;
S(O)-alkyle en C₁₋₆ ;
S(O)₂-alkyle en C₁₋₆ ; et
N (R¹⁸) S (O) ₂-alkyle en C₁₋₆ ;
où chaque alkyle en C₁₋₆ est facultativement substitué par un ou, indépendamment les uns des autres, plusieurs parmi F, COOR¹⁹, C(O)N(R²⁰R²¹), S (O)₂N(R²²R²³), OR²⁴, N(R²⁵R²⁶), T³, O-T³ ou N (R²⁷) -T³ ;
R¹³ est choisi dans le groupe constitué par
alkyle en C₁₋₆ ;
O-alkyle en C₁₋₆ ;
N(R¹⁴)-alkyle en C₁₋₆ ;
COO-alkyle en C₁₋₆ ;
OC(O)-alkyle en C₁₋₆ ;
C(O)N(R¹⁵)-alkyle en C₁₋₆ ;
N(R¹⁶)-C(O)-alkyle en C₁₋₆ ;
S(O)₂N(R¹⁷)-alkyle en C₁₋₆ ;
S (0) -alkyle en C₁₋₆ ;
S(0)₂-alkyle en C₁₋₆ ; et
-N (R¹⁸) S (O) ₂-alkyle en C₁₋₆ ;
où chaque alkyle en C₁₋₆ est facultativement substitué par un ou, indépendamment les uns des autres, plusieurs parmi F, COOR¹⁹, C (O) N (R²⁰R²¹), S (O) ₂N (R²²R²³), OR²⁴, N (R²⁵R²⁶), T³, O-T³ ou N (R²⁷) -T³ ;
R¹⁴, R¹⁵, R¹⁶, R¹⁷, R¹⁸, R¹⁹, R²⁰, R²¹, R²², R²³, R²⁴, R²⁵,
R²⁶, R²⁷ sont, indépendamment les uns des autres, H ou alkyle en C₁₋₆ ;
T³ est choisi dans le groupe constitué par
phényle ;
naphtyle ;
où phényle et naphtyle sont facultativement substitués par un ou, indépendamment les uns des autres, plusieurs parmi
halogène ;
CN ;
COOH ;
OH ;
C(O)NH₂ ;
S(O)₂NH₂ ;
alkyle en C₁₋₆ ;
O-alkyle en C₁₋₆ ;
COO-alkyle en C₁₋₆ ;
OC(O)-alkyle en C₁₋₆ ;
C(O)N(R²⁸)-alkyle en C₁₋₆ ;
S(O)₂N(R²⁹)-alkyle en C₁₋₆ ;
S(O)₂-alkyle en C₁₋₆ ; ou
N(R³⁰)S(O)₂-alkyle en C₁₋₆ ;
hétérocycle ;
hétérobicycle ; et
cycloalkyle en C₃₋₇ ;
où cycloalkyle en C₃₋₇, hétérocycle et hétérobicycle sont facultativement substitués par un ou, indépendamment les uns des autres, plusieurs parmi
halogène ;
CN ;
OH ;
=O, où le cycle est au moins partiellement saturé ;
NH₂ ;
COOH ;
C(O)NH₂ ;
S(O)₂NH₂ ;
alkyle en C₁₋₆ ;
O-alkyle en C₁₋₆ ;
N (R³¹) -alkyle en C₁₋₆ ;
COO-alkyle en C₁₋₆ ;
OC (O) -alkyle en C₁₋₆ ;
C (O) N (R³²) -alkyle en C₁₋₆ ;
N (R³³) -C (O) -alkyle en C₁₋₆ ;
S(O)₂N(R³⁴)-alkyle en C₁₋₆ ;
S (O)₂-alkyle en C₁₋₆ ; ou
-N (R³⁵) S (O)₂-alkyle en C₁₋₆.

2. Composé selon la revendication 1, de formule (Ia) 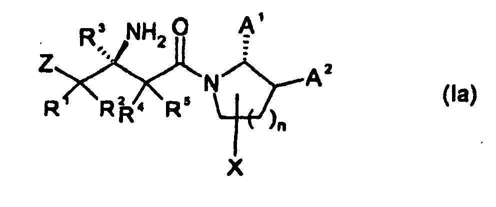 ou sel pharmaceutiquement acceptable de celui-ci, où Z, R¹ à R⁵, A¹, A², n et X ont la signification telle qu'indiquée dans la revendication 1.

3. Composé selon la revendication 1 ou 2, dans lequel Z est phényle ou hétérocycle et Z est facultativement substitué, indépendamment les uns des autres, par jusqu'à 2 parmi Cl, F, CN, CH₃ ou OCH₃.

4. Composé selon l'une quelconque des revendications précédentes, dans lequel R¹, R², R⁴, R⁵ sont, indépendamment les uns des autres, choisis dans le groupe constitué par H, F, OH, CH₃, OCH₃.

5. Composé selon l'une quelconque des revendications précédentes, dans lequel R³ est H.

6. Composé selon l'une quelconque des revendications précédentes, dans lequel X est H, F ou CH₃.

7. Composé selon l'une quelconque des revendications précédentes, dans lequel n est 1.

8. Composé selon l'une quelconque des revendications précédentes, dans lequel A¹ est R⁶ et A² est H, F ou CH₃.

9. Composé selon l'une quelconque des revendications précédentes, dans lequel R⁶ est -CH₂-Y-T .

10. Composé selon l'une quelconque des revendications précédentes, dans lequel Y est -O-, -N (R⁹) - ou -S (O) ₂-.

11. Composé selon l'une quelconque des revendications précédentes, dans lequel R⁹ est choisi dans le groupe constitué par H, CH₃, COOH, COOCH₃, C(0)NH₂, C(0)N(CH₃)₂ et S (O) ₂CH₃.

12. Composé selon l'une quelconque des revendications précédentes, dans lequel T est T¹-T² ou T² et dans lequel T¹ est choisi dans le groupe constitué par
-CH₂- ;
-C (O) - ;
-C (O) -CH₂- ;
-C (O) O- ;
-C (O)O-CH₂- ;
-C(O)NH- ;
-C(O)NH-CH₂- ;
-S(O)₂- ; et
-S(O)₂-CH₂-.

13. Composé selon la revendication 12, dans lequel T est T¹-T² ou T² et dans lequel T¹ est choisi dans le groupe constitué par -C (O) - ; -CH₂- ; -S (O)₂- ; et
-C(O)NH-.

14. Composé selon l'une quelconque des revendications précédentes, dans lequel R⁶ est -CH₂-N(R³⁶)-T, et dans lequel R³⁶ est H ou S(O)₂CH₃.

15. Composé selon l'une quelconque des revendications précédentes, dans lequel T² est phényle ou hétérocycle.

16. Composé selon la revendication 1, choisi dans le groupe constitué par 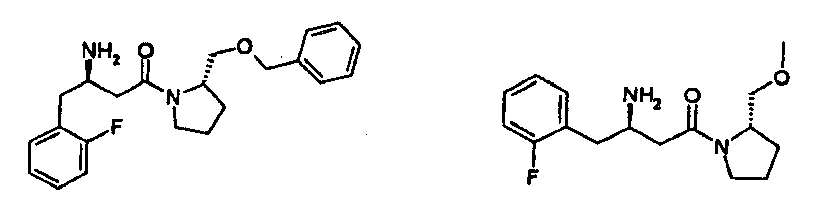 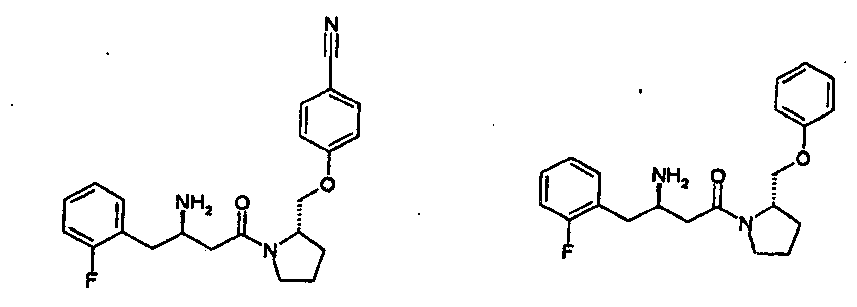 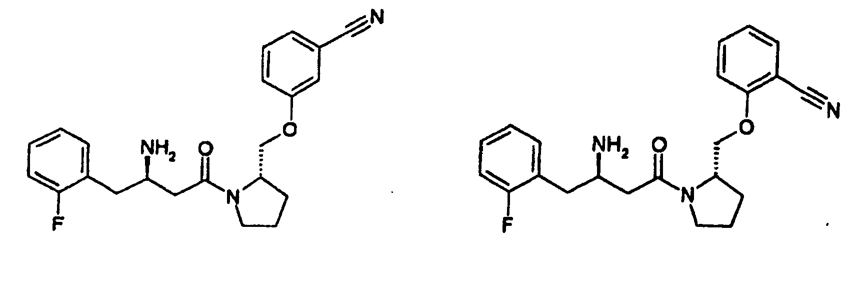 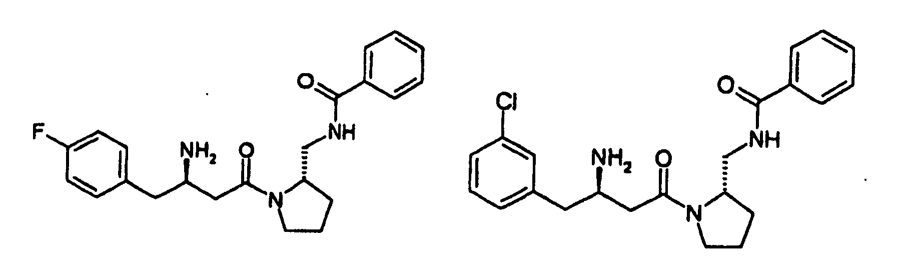 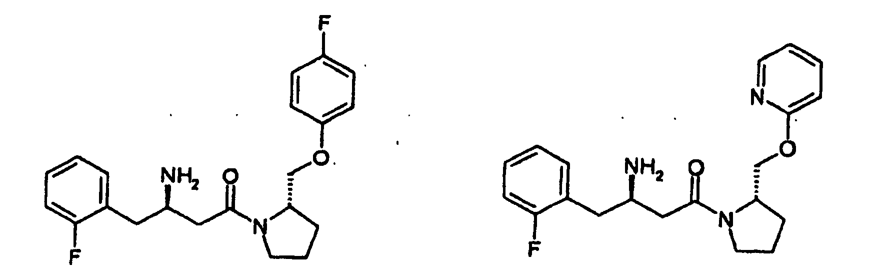 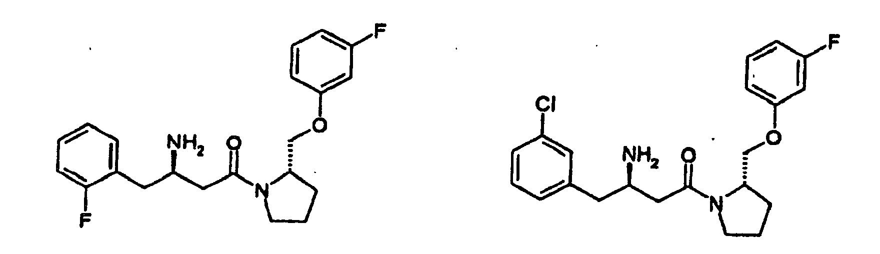 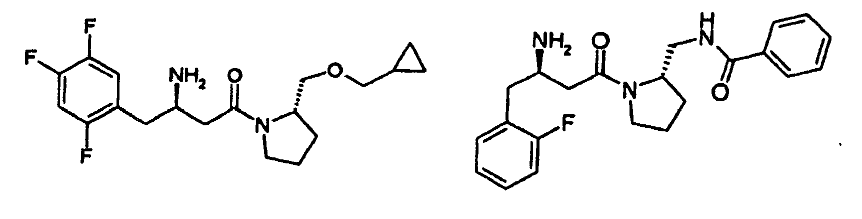 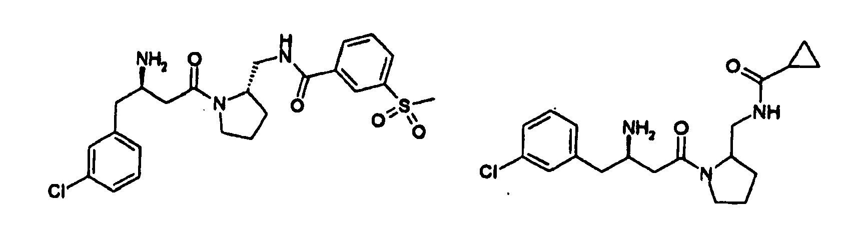 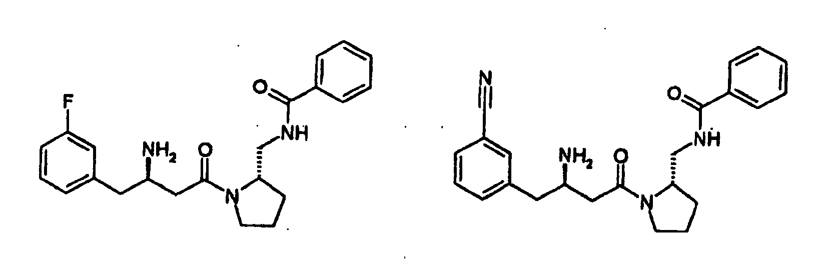 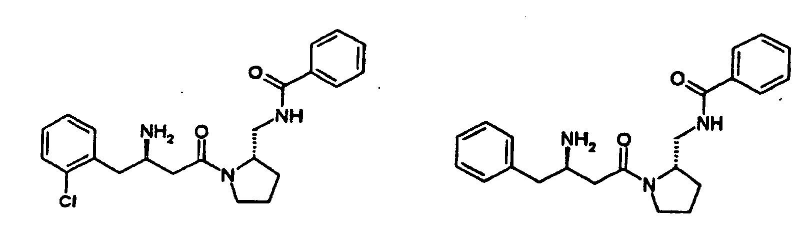 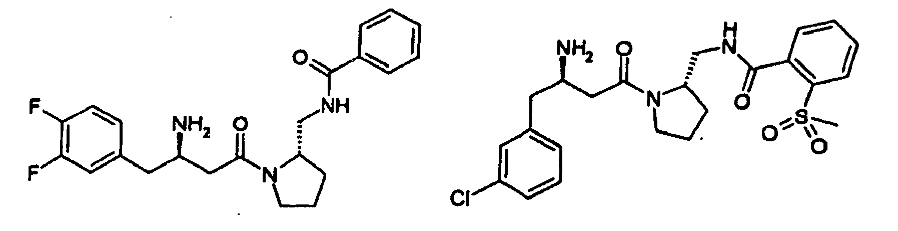 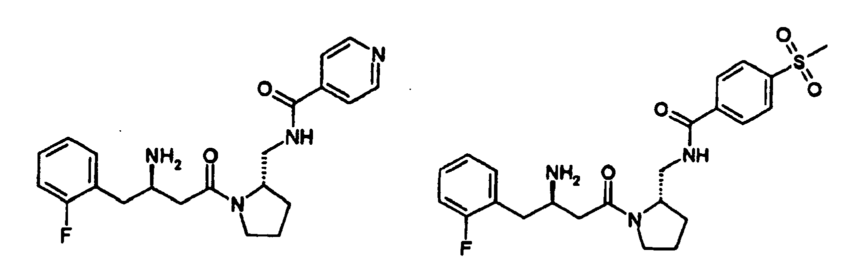 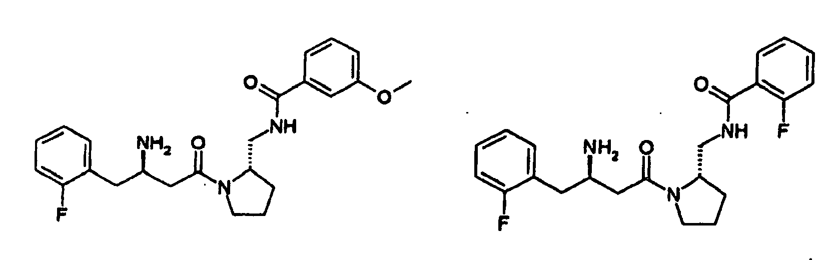 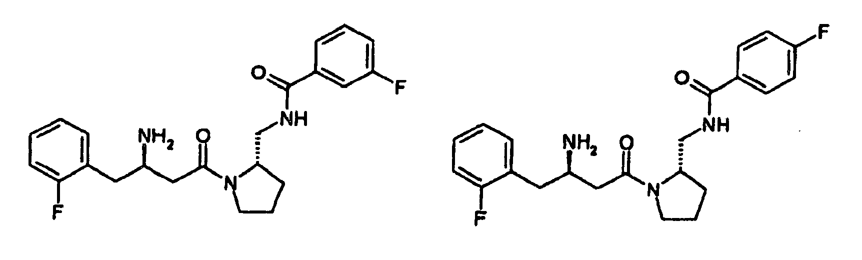 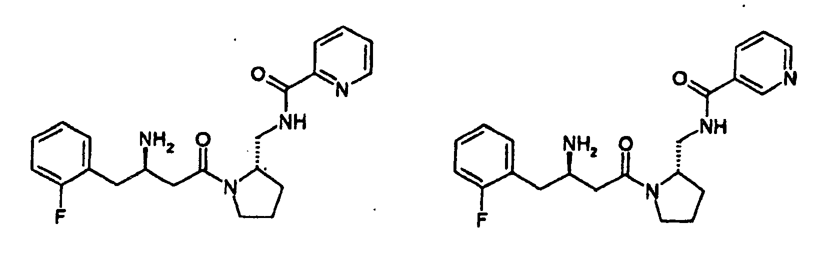 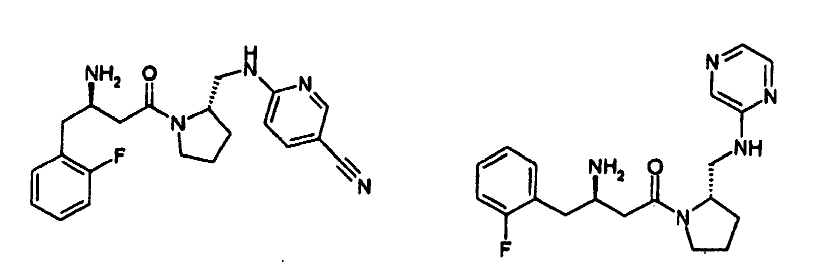 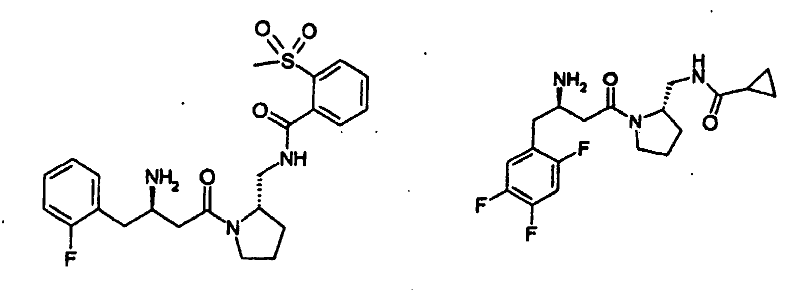 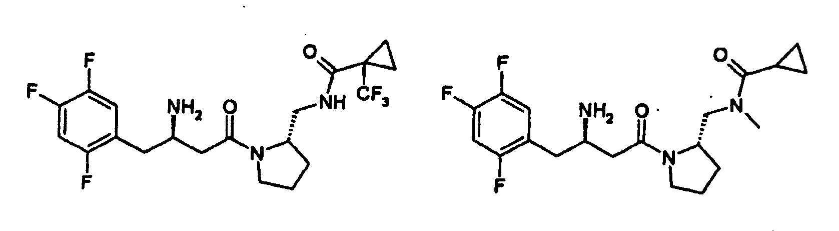 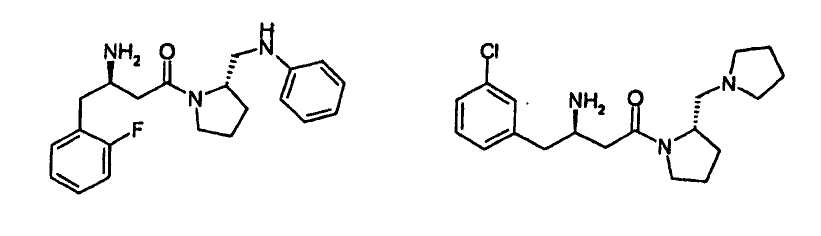 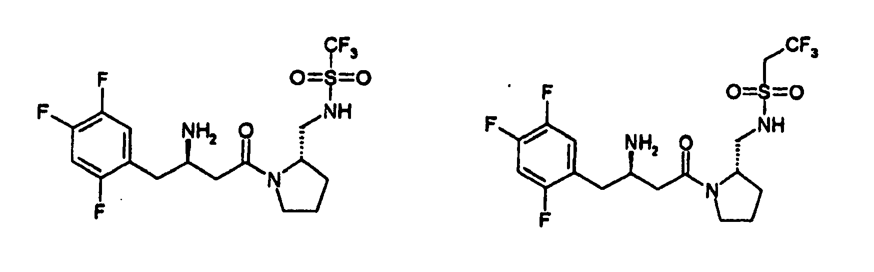 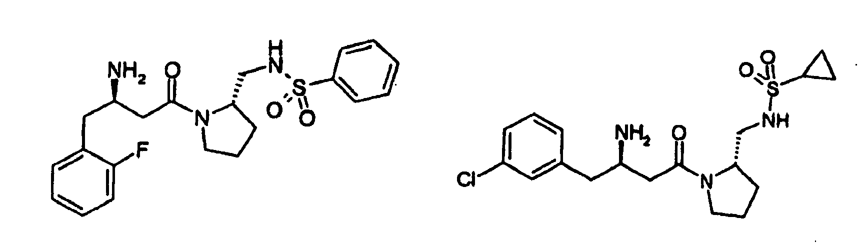 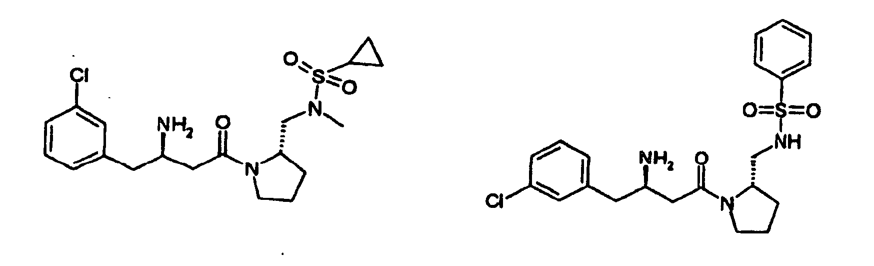 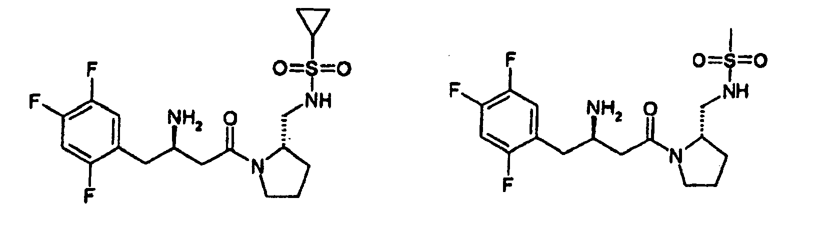 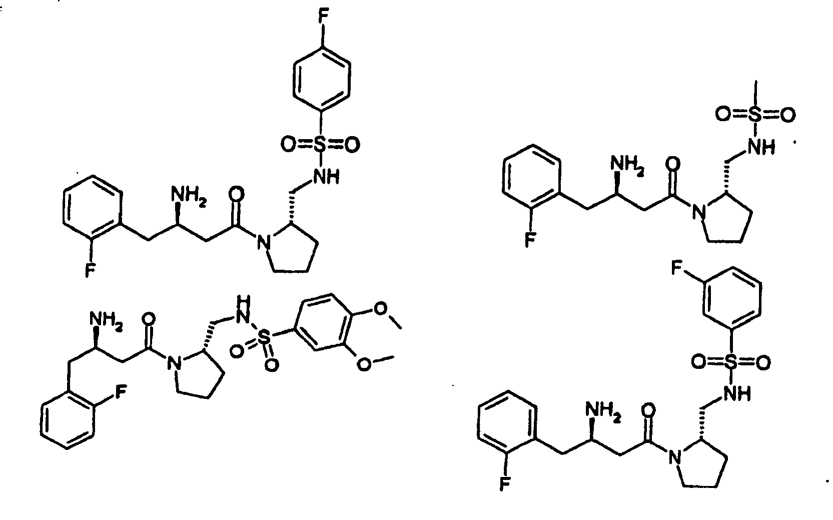 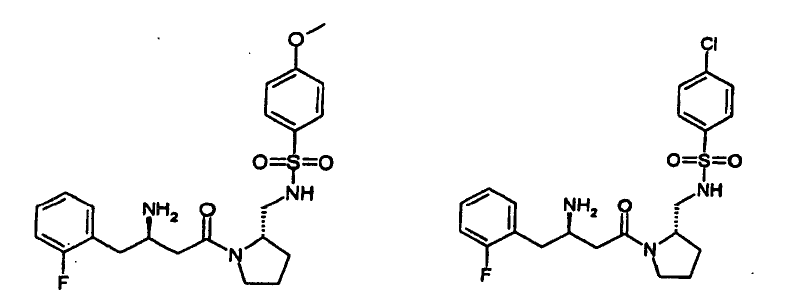 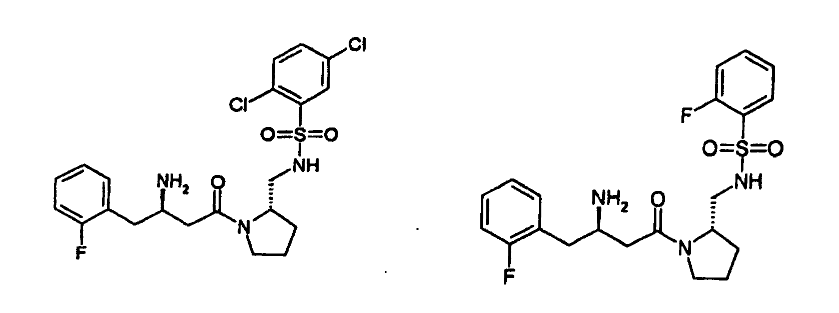 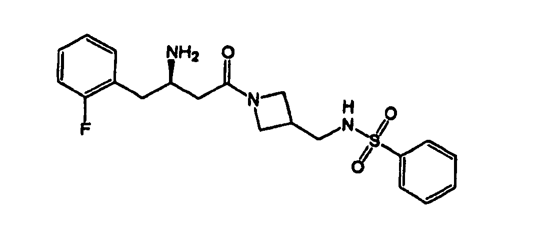 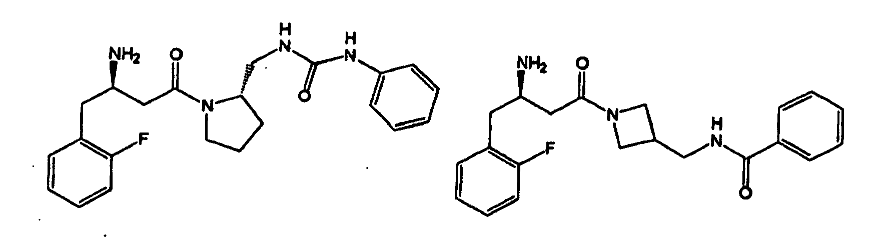 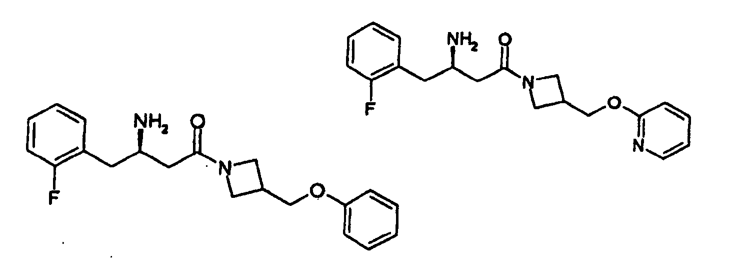 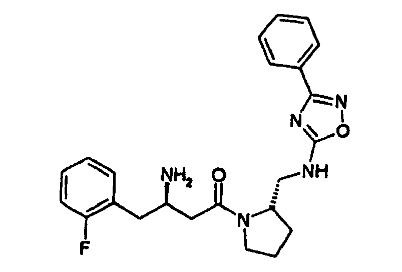 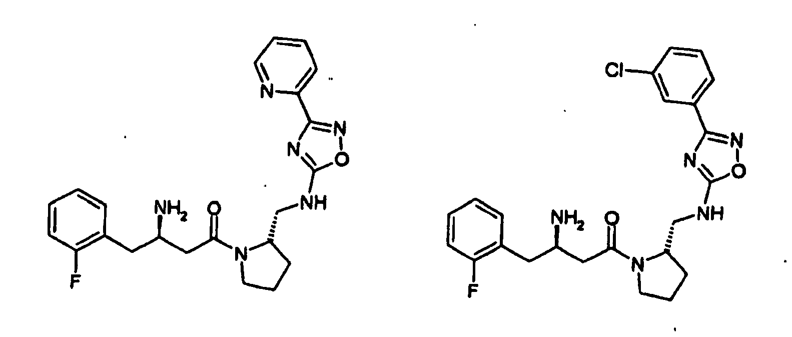 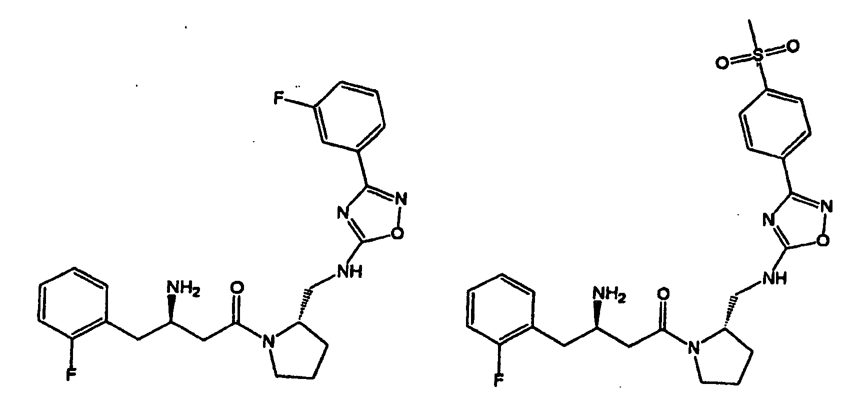 ou un sel pharmaceutiquement acceptable de celui-ci.

17. Composé prodrogue d'un composé selon l'une quelconque des revendications 1 à 16, dans lequel le groupe amino de formule (I) est acylé, alkylé ou phosphorylé.

18. Composition pharmaceutique comprenant un composé ou un sel pharmaceutiquement acceptable de celui-ci selon l'une quelconque des revendications 1 à 17, conjointement avec un véhicule pharmaceutiquement acceptable.

19. Composition pharmaceutique selon la revendication 18, comprenant un ou plusieurs composés supplémentaires ou sels pharmaceutiquement acceptables de ceux-ci, choisis dans le groupe constitué par un autre composé selon l'une quelconque des revendications 1 à 17 ; un autre inhibiteur de la DPP-IV ; les sensibilisateurs à l'insuline ; les agonistes du PPAR ; les biguanides ; les inhibiteurs de la protéine tyrosinephosphatase-IB (PTP-1B); l'insuline et les mimétiques d'insuline ; les sulfonylurées et autres sécrétagogues de l'insuline ; les inhibiteurs de l'a-glucosidase ; les antagonistes des récepteurs du glucagon ; GLP-1, les mimétiques du GLP-1 et les agonistes des récepteurs du GLP-1 ; GIP, les mimétiques du GIP et les agonistes des récepteurs du GIP ; le PACAP, les mimétiques du PACAP et les agonistes du récepteur 3 du PACAP ; les agents hypocholestérolémiants ; les inhibiteurs de la HMG-CoA réductase ; les séquestrants ; l'alcool nicotinylique ; l'acide nicotinique ou un sel de celui-ci ; les agonistes du PPARa ; les agonistes doubles du PPARoly ; les inhibiteurs de l'absorption du cholestérol ; l'acyl CoA : les inhibiteurs de la cholestérol acyltransférase ; les antioxydants ; les agonistes du PPARo ; les composés antiobésité ; un inhibiteur du transporteur de l'acide biliaire iléal ; et les agents anti-inflammatoires.

20. Composé ou sel pharmaceutiquement acceptable de celui-ci selon l'une quelconque des revendications 1 à 17 pour l'utilisation en tant que médicament.

21. Utilisation d'un composé ou d'un sel pharmaceutiquement acceptable de celui-ci selon l'une quelconque des revendications 1 à 17, pour la fabrication d'un médicament pour le traitement ou la prophylaxie d'un diabète non insulino-dépendant (type II) ; d'une hyperglycémie ; d'une obésité ; d'une insulinorésistance ; de troubles lipidiques ; d'une dyslipidémie ; d'une hyperlipidémie ; d'une hypertriglycéridémie ; d'une hypercholestérolémie ; de HDL bas ; de LDL élevés ; d'une athérosclérose ; d'un déficit en hormone de croissance ; de maladies liées à la réponse immunitaire ; d'une infection par VIH ; d'une neutropénie ; de troubles neuronaux ; d'une anxiété ; d'une dépression ; d'une métastase tumorale ; d'une hypertrophie bénigne de la prostate ; d'une gingivite ; d'une hypertension ; d'une ostéoporose ; de maladies liées à la motilité du sperme ; d'une faible tolérance au glucose ; d'une insulinorésistance ; de sequelles d'ist ("ist sequelae") ; d'une resténose vasculaire ; d'un syndrome du côlon irritable ; d'une maladie intestinale inflammatoire ; incluant la maladie de Crohn et la recto-colite hémorragique ; d'autres affections inflammatoires ; d'une pancréatite ; d'une obésité abdominale ; d'une maladie neurodégénérative ; d'une rétinopathie ; d'une néphropathie ; d'une neuropathie ; d'un syndrome X ; d'un hyperandrogénisme ovarien (syndrome ovarien polycystique) ; d'un diabète de type n ; ou d'un déficit en hormone de croissance.

22. Procédé pour la préparation d'un composé selon l'une quelconque des revendications 1 à 17, comprenant les étapes consistant
• à coupler un acide bêta-amino à protection amino, de formule (IVa) 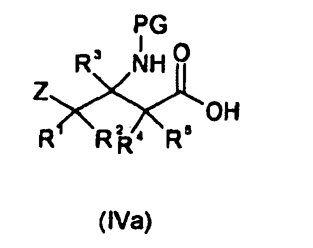 dans laquelle PG est un groupe protecteur, avec une amine de formule (III) 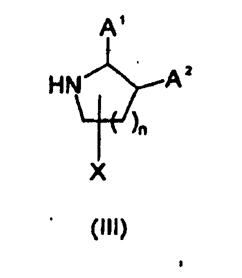 en utilisant des conditions de couplage peptidique, des réactifs et des groupes protecteurs standards ;
• à enlever le groupe protecteur (PG).

23. Procédé selon la revendication 22, dans lequel les réactifs de couplage sont le chlorhydrate de 1-éthyl-3-(3-diméthylaminopropyl)carbodiimide (EDC) en combinaison avec le 1-hydroxybenzotriazole (HOBt) et une base (triéthylamine ou diisopropyléthylamine) ou l'hexafluorophosphate de O-(7-azabenzotriazol-1-yl)-*N,N,N',N'-*tétraméthyluronium (HATU) en présence d'une base, et le groupe protecteur est le 9-fluorénylméthoxycarbonyle ou le tert-butoxycarbonyle.

24. Procédé selon la revendication 22 ou 23, dans lequel le groupe protecteur est enlevé en utilisant de la diéthylamine dans du dichlorométhane dans le cas du 9-fluorénylméthoxycarbonyle, ou en utilisant des conditions acides dans le cas du *tert*-butoxycarbonyle.
